# EUROPEAN PATENT SPECIFICATION

(11) **EP 2 488 010 B1**
(45) Date of publication and mention of the grant of the patent: **06.12.2017**
(21) Application number: 10822907.1
(22) Date of filing: 15.10.2010
(51) Int. Cl.: A01H 5/00, C12N 15/29, C12N 15/82, C12Q 1/68

(54) **MANIPULATION OF FLAVONOID BIOSYNTHETIC PATHWAY**
MANIPULATION DES BIOSYNTHESE-SIGNALWEGES VON FLAVONOIDEN
MANIPULATION D'UNE VOIE DE BIOSYNTHÈSE DE FLAVONOÏDES

(30) Priority: 16.10.2009 AU 2009905057; 16.10.2009 AU 2009905058; 16.10.2009 AU 2009905063
(43) Date of publication of application: 22.08.2012
(73) Proprietor: Agriculture Victoria Services Pty Ltd, Attwood Victoria 3049 (AU)
(72) Inventor: MOURADOV, Aidyn, Mill Park, Victoria 3802 (AU); SPANGENBERG, German, Bundoora, Victoria 3083 (AU)
(74) Representative: Grund, Martin
(86) International application number: PCT/AU2010/001362
(87) International publication number: WO 2011/044632

(56) References cited:
- WO-A1-2009/003216
- WO-A1-2010/025247
- WO-A2-02/10412
- WO-A2-02/39809
- WO-A2-2008/134372
- WO-A2-2008/134372
- AIDYN MOURADOV ET AL: "Molecular dissection of proanthocyanidin and anthocyanidin biosynthesis in white clover (trifolium repens)", 1 January 2009 (2009-01-01), MOLECULAR BREEDING OF FORAGE AND TURF, SPRINGER, PAGE(S) 133 - 139, XP009174672, ISBN: 978-0-387-79143-2 * page 134, paragraph 2 - page 135, paragraph 1 *
- XIE D-Y ET AL: "Role of anthocyanidin reductase, encoded by BANYULS in plant flavonoid biosynthesis", SCIENCE, AMERICAN ASSOCIATION FOR THE ADVANCEMENT OF SCIENCE, WASHINGTON, DC; US, vol. 299, 17 January 2003 (2003-01-17), pages 396-399, XP002982939, ISSN: 0036-8075, DOI: 10.1126/SCIENCE.1078540
- Abeynayke, shamila Weerakoon: "Analysis of genes involved in condensed tannin biosynthesis in white clover (trifolium repens. L).", , 2009, XP002717356, Retrieved from the Internet: URL:http://library.latrobe.edu.au/record=b 2543251 [retrieved on 2013-11-27]
- JAAKOLA LAURA ET AL: "Expression of genes involved in anthocyanin biosynthesis in relation to anthocyanin, proanthocyanidin, and flavonol levels during bilberry fruit development", PLANT PHYSIOLOGY (ROCKVILLE), vol. 130, no. 2, October 2002 (2002-10), pages 729-739, XP002717357, ISSN: 0032-0889
- S. W. ABEYNAYAKE ET AL: "Biosynthesis of Proanthocyanidins in White Clover Flowers: Cross Talk within the Flavonoid Pathway", PLANT PHYSIOLOGY, vol. 158, no. 2, December 2011 (2011-12), pages 666-678, XP055090636, ISSN: 0032-0889, DOI: 10.1104/pp.111.189258
- RAY, H. ET AL.: 'Expression of Anthocyanins and Proanthocyanidins after Transformation of Alfalfa with Maize Lc' PLANT PHYSIOLOGY vol. 132, 2003, pages 1448 - 1463, XP002398098
- MATOUSEK, J. ET AL.: '''Sequence Analysis of a ''True'' Chalcone Synthase (chs-H1) Oligofamily from hop (Humulus lupulus L.) and PAP Activation of chs_H1 in Heterologous Systems''' JOURNAL OF AGRICULTURAL AND FOOD CHEMISTRY vol. 54, 2006, pages 7606 - 7615, XP008161403
- NAKATSUKA, T. ET AL.: 'Two different mutations are involved in the formation of white-flowered gentian plants' PLANT SCIENCE vol. 169, 2005, pages 949 - 958, XP027783709
- WANG, C.-K. ET AL.: 'Cosuppression of tobacco chalcone synthase using Petunia chalcone synthase construct results in white flowers' BOTANICAL STUDIES vol. 47, 2006, pages 71 - 82, XP008161426
- PARK, J.-S. ET AL.: 'Genes up-regulated during red coloration in UV-B irradiated lettuce leaves' PLANT CELL REPORTS vol. 26, 2007, pages 507 - 516, XP019490261
- Shamila Weerakoon Abeynayake: "Analysis of genes involved in condensed tannin biosynthesis in white clover (Trifolium repens L.)" In: "Thesis submitted for the degree of Doctor of Philosophy to the Department of Botany School of Life Sciences Faculty of Science, Technology and Engineering La Trobe University Bundoora, Victoria 3086 Australia", 1 April 2009 (2009-04-01), XP055100605, page 214 pp.,

## Description

### Field of the invention

The present invention relates to methods for manipulating or identifying genes involved in the flavonoid biosynthetic pathway in plants.

### Background of the invention

Flavonoids constitute a relatively diverse family of aromatic molecules that are derived from phenyalanine and malonyl-coenzyme A (CoA, via the fatty acid pathway). These compounds include six major subgroups that are found in most higher plants: the chalcones, flavones, flavonols, flavandiols, anthocyanins and proanthocyanidins (or condensed tannins). A seventh group, the aurones, is widespread, but not ubiquitous.

Some plant species also synthesize specialised forms of flavonoids, such as the isoflavonoids that are found in legumes and a small number of non-legume plants. Similarly, sorghum, maize and gloxinia are among the few species known to synthesize 3-deoxyanthocyanins (or phlobaphenes in the polymerised form). The stilbenes, which are closely related to flavonoids, are synthesised by another group of unrelated species that includes grape, peanut and pine.

Besides providing pigmentation to flowers, fruits, seeds, and leaves, flavonoids also have key roles in signalling between plants and microbes, in male fertility of some plant species, in defense as antimicrobial agents and feeding deterrants, and in UV protection.

Flavonoids also have significant activities when ingested by animals, and there is great interest in their potential health benefits, particularly for compounds such as isoflavonoids, which have been linked to anticancer benefits, and stilbenes that are believed to contribute to reduced heart disease.

Flavonoid biosynthesis is one of the most intensively studied secondary metabolism pathways in plants. It is regulated by a complex network of signals triggered by internal metabolic cues and external signals, including visible light, ultraviolet (UV) radiation, pathogen attack, nitrogen, phosphorus and iron deficiencies, low temperature and wounding. Regulation of the flavonoid branch pathway producing the flavan-3-ols, the building blocks of proanthocyanidins (PAs) has been studied in species including *Arabidopsis thaliana*, legumes (*Medicago sativa, M. truncatula, Desmodium uncinatum, Lotus corniculatus*), grape, apple and tobacco.

Much of our recent understanding of flavan-3-ol and PA biosynthesis has arisen from genetic and biochemical analyses of mutants in the model plant *A. thaliana.* These mutant lines have a 'transparent testa' phenotype because they fail to accumulate or oxidize PAs, which normally give seed coats their brown pigmentation. Sixteen out of nineteen *TRANSPARENT TESTA* (*TT*) genes have been identified and characterized at the molecular level. Eight are structural genes, encoding the biosynthetic enzymes chalcone synthase (CHS), chalcone isomerase (CHI), flavonoid 3 - hydroxylase (F3H), flavonoid 3'- hydroxylase (F3'H), flavonoid 3'-5'- hydroxylase (F3'5'H), dihydroflavonol-4-reductase (DFR), anthocyanidin synthase (ANS) and anthocyanidin reductase (ANR, *BANYULS* gene). Four TT genes (*TT1, TT2, TT8, TT16)*, two *TRANSPARENT TESTA GLABRA* genes (*TTG1, TTG2*) and *PURPLE ANTHOCYANIN PIGMENTATION 1 (PAP1*) encode regulatory proteins.

Mutations of *TT1,* encoding a zinc finger protein, and *TT16*/*ABS*, encoding a MADS-box factor, affect the spatial pattern of *BANYULS* expression. *TT16*/*ABS* mediates the expression of *BANYULS* and PA accumulation in the endothelium of seed coats except for the chalazal-micropylar area. *TTG2*, a WRKY-box transcription factor, is involved in regulating late steps of PA biosynthesis after the leucoanthocyanidin branch point. Cooperative action of two transcription factors, TT2, an R2R3-MYB factor, and TT8, an R/B-like bHLH factor, directly regulate expression of the late biosynthesis genes (LBG) involved in PA production.

Two *TT* genes (*TT12, TT19*) and *Arabidopsis* H+-ATPase 10 are involved in the compartmentalization of flavonoids. The *TT10* gene, encoding a laccase-like enzyme thought to be involved in oxidation and condensation of PA subunits, has also been characterized. Recent studies have identified key genes and enzymes of the PA branch of the flavonoid pathway controlling the biosynthesis of the 2,3-*trans*-flavan-3-ols (afzelechin, catechin, and gallocatechin) and 2,3-*cis*-flavan-3-ols (epiafzelechin, epicatechin, and epigallocatechin) from flavan-3,4-diols (leucoanthocyanidins). The first pathway involves direct reduction of 2,3-flavan-3,4-diols to 2,3-*trans*-flavan-3-ols by leucoanthocyanidin reductase (LAR, EC 1.17.1.3). The corresponding gene was initially isolated from *D. uncinatum* and was later characterized in other legumes, camellia, grape and apple.

The second pathway involves the sequential conversion of 2,3-flavan-3,4-diols to anthocyanidin molecules by anthocyanidin synthase (ANS, EC 1.14.11.19) and the reduction of anthocyanidins to 2,3-*cis*-flavan-3-ols by anthocyanidin reductase (ANR, EC 1.3.1.77). The *BANYULS* gene, encoding ANR, has been isolated and characterised in *A. thaliana, M. truncatula*, apple, *Lotus corniculatus* and grape.

Legumes offer many opportunities for studying PAs and include species that accumulate a range of PA levels and compositions in different tissues. Extensive genetic and functional genomic resources make *M. truncatula* an ideal model legume for studying PA biosynthesis *M. sativa* and *M. truncatula* plants accumulate a low level of PAs in flowers, stems, roots and leaves.

White clover (*Trifolium repens* L.) is a major component of temperate improved pastures, worldwide, and is a key forage plant in countries with intensive livestock production systems. A low level of proanthocyanidins (3% of dry weight) in forages is beneficial in preventing pasture bloat and increasing nutrient uptake in ruminant livestock. Although white clover plants accumulate a high level of PAs in flowers and seed coats, there is a very low level in vegetative tissues, where PAs, and/or their flavan-3-ol monomers, are restricted to trichome cells.

In spite of the characterization of PA-related genes and biochemical studies of corresponding proteins and metabolites in different species, some steps of PA biosynthesis are still poorly understood. For example, it is not clear whether the role of ANR is restricted only to the biosynthesis of *cis*-flavan-3-ols or if its activity is required for the production of both the *cis* and *trans* 2,3-flavan-3-ol epimers. Six years after isolation and characterization of the first *LAR* gene from *D. uncinatum* molecular aspects of 2,3-*trans*-flavan-3-ol biosynthesis and the contribution of the *LAR* gene to PA biosynthesis are still unclear and based only on *in vitro* activity of recombinant LAR enzymes and expression profiles of *LAR* genes in PA-accumulating tissues. Transgenic approaches are limited to ectopic expression studies in tobacco and white clover plants. Loss-of-function approaches are not suitable in *Arabidopsis* and *M. truncatula*, where *trans* 2,3-flavan-3-ols are absent or produced at a low level.

WO 2008/134372 and WO 2009/003216 disclose flavonoid biosynthetic enzymes useful in modulating the ANT and PA production in plants.

While nucleic acid sequences encoding some flavonoid biosynthetic enzymes have been isolated for certain species of plants, there remains a need for materials useful in modifying flavonoid biosynthesis; in modifying protein binding, metal chelation, antioxidation, and UV-light absorption; in modifying plant pigment production; in modifying plant defense to biotic stresses such as viruses, micro-organisms, insects or fungal pathogens; in modifying forage quality, for example by disrupting protein foam and/or reducing rumen pasture bloat, particularly in forage legumes and grasses, including alfalfa, medics, clovers, ryegrasses and fescues, and for methods for their use.

It is an object of the present invention to overcome, or at least alleviate, one or more of the difficulties or deficiencies associated with the prior art.

### Summary of the invention

The invention is defined by the appended claims.

Applicants have used an extensive transcriptomics approach, in combination with biochemical analysis of selected flavonoids, for molecular dissection of the PA and ANT pathways, co-localized in epidermal cells of floral organs. Spatio-temporal profiles of flavonoid gene expression and accumulation of the corresponding metabolites suggests that components of the ANT and PA pathways may be encoded by distinct members of multigene families. Applicants' gene-to-metabolite approach, integrating transcriptomic and biochemical data from transgenic white clover plants in which the *TrANR* and *TrLAR* genes were down-regulated, suggests that cross-talk occurs between the ANT and PA pathways and that both the ANR- and LAR-specific branches of the PA biosynthetic pathway are active in white clover flowers. Applicants provide the first genetic evidence that LAR activity is required for 2,3-*trans*-flavan-3-ol biosynthesis in white clover flowers.

Applicants propose that metabolic re-programming of the flavonoid pathway to increase the PA level in leaves is an attractive strategy for enhancing bloat safety. Floral PAs in *T. repens* consist of nearly equal proportions of epigallocatechins and gallocatechins. This and the relatively high genetic transformation efficiency of white clover make it a good system for functional analysis of genes involved in biosynthesis of both 2,3-*trans-*flavan-3-ols and 2,3-*cis*-flavan-3-ols. Co-localization of the ANT and PA pathways in floral tissues is another advantage of this system, allowing the possibility of metabolic crosstalk to be investigated.

In one aspect, the present invention provides a method of identifying a gene encoding a polypeptide or polypeptide isoform which is substantially more active in either a proanthocyanidin (PA) or anthocyanin (ANT) pathway of a plant, said method including
providing
   material from said plant at two or more developmental stages; and
   an oligonucleotide probe capable of hybridizing with RNA from a gene encoding a polypeptide which is active in a flavonoid biosynthetic pathway; extracting RNA from said plant material;
hybridizing the oligonucleotide probe with the RNA using a microarray to generate an expression profile;
measuring PA and ANT levels in said plant material to generate a metabolic profile;
comparing said expression profile with said metabolite profile using a computer to identify said gene encoding a polypeptide or polypeptide isoform which is substantially more active in either a PA or ANT pathway.

By a 'polypeptide' is meant a polymer of linked amino acids, which may be an enzyme, regulatory protein or transporter protein. The enzyme may be a biosynthetic enzyme such as CHS, CHI, F3H, F3'H, F3'5'H, DFR, LAR, ANS, ANR, GST, G3T, UFGT, OMT, ART, ANAT or AAT. The regulatory protein may be a transcription factor such as TT1, TT2, TT8, TT16, TTG1, TTG2, MYB, bHLH, MYC, WDR or PAP1. The transporter protein may be a polypeptide involved in the compartmentalisation of flavonoids, such as TT12, TT19 or H⁺-ATPase 10.

By a 'polypeptide isoform' is meant one of two or more different forms of a polypeptide, which may be produced from related genes, or may arise from the same gene by alternative splicing. The isoforms may be produced by single nucleotide polymorphisms (SNPs), small genetic differences between alleles of the same gene.

By 'substantially more active in either a PA or ANT pathway' is meant that the polypeptide or polypeptide isoform has higher activity in either the branch of the flavonoid biosynthetic pathway that produces PAs or the branch of the flavonoid biosynthetic pathway that produces ANTs, when compared with its activity in the other pathway.

In a preferred embodiment the polypeptide or polypeptide isoform has activity at least approximately 15% higher, more preferably at least approximately 25% higher, more preferably at least approximately 35% higher, more preferably at least approximately 50% higher in one pathway relative to the other pathway.

For example, activity may be between approximately 15% and 100% higher, more preferably between approximately 25% and 200% higher, more preferably between approximately 35% and 300% higher, more preferably between approximately 50% and 500% higher in one pathway relative to the other pathway.

In a particularly preferred embodiment, the polypeptide or polypeptide isoform may be active in one pathway, and have no detectable activity in the other pathway.

In a preferred embodiment the polypeptide or polypeptide isoform may be substantially more active in a PA pathway relative to an ANT pathway. In a particularly preferred embodiment, the polypeptide or polypeptide isoform may be active in the PA pathway and have no detectable activity in the ANT pathway.

In a preferred embodiment, the polypeptide or polypeptide isoform may be an enzyme which is active late in the ANT pathway.

By an "enzyme which is active late in the ANT pathway" or a "late ANT pathway enzyme" is meant an enzyme which catalyses one of the final reactions in the synthesis of anthocyganins, after the leucoanthocyanidin branch point.

For example, the late ANT-pathway enzyme may be selected from the group consisting of GST, G3T, UFGT, OMT, ART, ANAT and AAT.

In an alternate preferred embodiment, the polypeptide or polypeptide isoform may be a transcription factor.

For example, the transcription factor may be selected from the group consisting of MYB, bHLH, MYC and WDR.

In preferred embodiments, the plant material may be a plant organ or tissue, such as a flower or inflorescence, or part thereof such as a floret, petal, sepal or stamen, or other floral tissue, or a vegetative organ or part thereof such as a leaf or other plant tissue.

Preferably, the material from said plant is floral material. Preferably, the floral material is at two or more developmental stages, for example immature, partially open (eg. approximately 5 to 35% open, approximately 35 to 65% open, and approximately 65-95% open) and mature stages of development.

By an 'oligonucleotide probe' is meant a short nucleic acid polymer, preferably having between approximately 5 and 200 bases, more preferably between approximately 10 and 100 bases, more preferably between approximately 20 and 50 bases.

By 'nucleic acid' is meant a chain of nucleotides capable of carrying genetic information. The term generally refers to genes or functionally active fragments or variants thereof and or other sequences in the genome of the organism that influence its phenotype. The term 'nucleic acid' includes DNA (such as cDNA or genomic DNA) and RNA (such as mRNA or microRNA) that is single- or double-stranded, optionally containing synthetic, non-natural or altered nucleotide bases, synthetic nucleic acids and combinations thereof.

It will be understood by those of skill in the art that the term 'oligonucleotide probe' applies to one or more oligonucleotide molecules, either identical or non-idential, which are designed, selected, and/or otherwise able to specifically hybridize to a target RNA. Additionally, an oligonucleotide probe as defined herein may comprise a collection of different oligonucleotide molecules targeted to one or more target regions of the same RNA. Thus, the term 'oligonucleotide probe' as used herein may mean either the singular or the plural, such meaning being made clear by the context of usage in the present specification. Preferably a pair of oligonucleotide probes is used.

In a particularly preferred embodiment, the pair of oligonucleotide probes is selected from the pairs shown in Table 5 hereto and functionally active fragments and variants thereof.

By 'functionally active fragment or variant' in relation to an oligonucleotide probe is meant that the fragment or variant (such as an analogue, derivative or mutant) is capable of hybridizing with RNA from the gene encoding a polypeptide active in a flavonoid biosynthetic pathway. Additions, deletions, substitutions and derivatizations of one or more of the nucleotides are contemplated so long as the modifications do not result in loss of functional activity of the fragment or variant. Preferably the functionally active fragment or variant has at least approximately 80% identity to the relevant part of the above mentioned sequence to which the fragment or variant corresponds, more preferably at least approximately 90% identity, even more preferably at least approximately 95% identity, even more preferably at least approximately 98% identity even more preferably at least approximately 99% identity. Preferably the fragment has a size of between approximately 5 and 200 bases, more preferably between approximately 10 and 100 bases, more preferably between approximately 20 and 50 bases.

Preferred fragments and variants include those having a single addition or deletion, or substitution of a single nucleic acid, when compared with an oligonucleotide probe shown in Table 5 hereto.

By 'capable of hybridizing with' is meant that the oligonucleotide probe has a nucleotide sequence sufficiently complementary to a target RNA sequence to permit said oligonucleotide to hybridize therewith under hybridization conditions.

The term 'hybridization' is understood to mean the process during which, under suitable conditions, two nucleotide fragments having sufficiently complementary sequences are capable of forming a double strand with stable and specific hydrogen bonds. A nucleotide fragment 'capable of hybridizing' with a polynucleotide is a fragment which can hybridize with said polynucleotide under hybridization conditions which are determined in a known manner in each case. The hybridization conditions are determined by means of the stringency, ie. the severity of the operating conditions. The higher the stringency at which the hybridization is carried out, the more specific the hybridization is. The stringency is defined in particular according to the base composition of a probe/target duplex, and also by means of the degree of mismatching between two nucleic acids.

The 'stringency' can also depend on the parameters of the reaction, such as the concentration and the type of ion species present in the hybridization solution, the nature and the concentration of denaturing agents and/or the hybridization temperature. The stringency of the conditions under which a hybridization reaction should be carried out will depend mainly on the target probes used. All these data are well known and the appropriate conditions can be determined by those skilled in the art.

Preferably, high stringency conditions may be used. By 'high stringency conditions' is meant the hybridization takes place at a temperature between approximately 35°C and 65°C and at a salt concentration of between approximately 0.5 to 1 m, more preferably at a temperature between 50°C and 65°C and at a salt concentration of between approximately 0.8 to 1M.

By 'RNA from a gene encoding a polypeptide which is active in a flavonoid biosynthetic pathway' is generally meant mRNA transcribed or otherwise generated from the gene.

The gene encoding a polypeptide which is active in a flavonoid biosynthetic pathway may encode any polypeptide which catalyses a reaction or is otherwise involved in flavonoid biosynthesis in a plant, for example an enzyme, regulatory protein or transporter protein, as hereinbefore described. Preferably the polypeptide which is active in a flavonoid biosynthetic pathway is selected from the polypeptides listed in Tables 1-4 hereto.

RNA may be extracted from the plant material by methods known to the person skilled in the art. For example, a CTAB-based extraction method may be employed. Further purification of the extracted RNA may be carried out, again by methods known to those skilled in the art.

The step of hybridizing the oligonucleotide probe with the RNA may also be carried out by methods known to those skilled in the art. A microarray is used to generate an expression profile.

By an 'expression profile' is meant that the activity or level of RNA expression of the gene is measured for the material from the plant, preferably at two or more developmental stages.

The step of measuring PA and ANT levels in the plant material may be carried out qualitatively and/or quantitatively.

A qualitative measurement may be carried out by visualising PA and ANT in untreated or stained plant material.

In a preferred embodiment, plant material may be stained for the presence of PA, for example using DMACA, and then PA visualised.

In a preferred embodiment, ANT may be visualised in untreated plant tissues.

A semi-quantitative measurement of PA may be carried out using a PVPP assay.

PA and/or ANT levels in the plant materials may also be measured quantitatively by measuring metabolites using liquid chromatography mass spectroscopy (LCMS).

The step of comparing said expression profile with said metabolic profile may be carried out by methods known to those skilled in the art. Preferably, the profiles are compared to identity genes that are up- or down-regulated, the up- or down-regulation correlating with PA or ANT accumulation. The step of comparing the expression profile with the metabolic profile is preferably performed using an electronic device, such as a computer.

In a further aspect, the present invention provides a method of manipulating the flavonoid biosynthetic pathway in a plant, said method including identifying a gene encoding a polypeptide or polypeptide isoform which is substantially more active in either a PA or ANT pathway of said plant and up- or down-regulating expression of said gene to increase or decrease the level of PA or ANT in said plant.

In a preferred embodiment, said method includes identifying a gene encoding a polypeptide or polypeptide isoform which is substantially more active in the PA pathway and up-regulating expression of said gene.

In an alternate preferred embodiment, said method includes identifying a gene encoding a polypeptide or polypeptide isoform which is substantially more active in the PA pathway and down-regulating expression of said gene.

In an alternate preferred embodiment, said method includes identifying a gene encoding a polypeptide or polypeptide isoform which is substantially more active in the ANT pathway and up-regulating expression of said gene.

In an alternate preferred embodiment, said method includes identifying a gene encoding a polypeptide or polypeptide isoform which is substantially more active in the ANT pathway and down-regulating expression of said gene.

In a preferred embodiment, said method may include down-regulating expression of a gene encoding a polypeptide or polypeptide isoform which is active late in the ANT pathway.

For example, for targeted modification of the anthocyanin pathway in a red leaf white clover mutant expressing *TrANR* (anthocyanidin reductase) gene, the modification may include down-regulation of late anthocyanin-specific genes.

Particularly preferred genes include those encoding GST, G3T, UFGT, OMT, ART, ANAT and AAT.

In an alternate preferred embodiment, said method may include up- and/or down-regulating expression of one or more genes encoding a transcription factor.

For example, for targeted modification of the anthocyanin pathway in a red leaf white clover mutant expressing *TrANR* (anthocyanidin reductase) gene, the modification may include up- and/or down-regulation of genes encoding transcription factors.

Particularly preferred genes include those encoding MYB, bHLH, MYC and WDR.

By 'manipulating the flavonoid biosynthetic pathway' is meant modifying flavonoid biosynthesis in a plant relative to a control plant. Preferably, flavonoid biosynthesis may be modified to increase PA biosynthesis relative to ANT biosynthesis. However, for some applications it may be desirable to increase ANT biosynthesis relative to PA biosynthesis.

Preferably, the step of identifying a gene encoding a polypeptide or polypeptide isoform which is substantially more active in a PA or ANT pathway is carried out by a method as hereinbefore described. Alternatively, in certain circumstances the gene may already be indentified and the method may omit the identification step.

By 'up-regulating' expression of said gene is meant increasing expression of said gene and, as a result, the protein encoded by the gene, in a plant relative to a control plant.

By 'down-regulating' expression of said gene is meant decreasing expression of said gene and, as a result, the protein encoded by the gene, in a plant relative to a control plant.

The up-regulation or down-regulation may be carried out by methods known to those skilled in the art. For example, a gene may be up-regulated by incorporating additional copies of a sense copy of the gene. A gene may be down-regulated, for example, by incorporating an antisense nucleic acid, a frame-shifted or otherwise modified sense copy of the gene, or a nucleic acid encoding interfering RNA (RNAi).

The up- or down-regulation may be carried out by introducing into said plant an effective amount of a genetic construct including the gene or a modified form thereof, such as an antisense nucleic acid, a frame shifted copy of the gene or a nucleic acid encoding RNAi.

Techniques for incorporating the genetic constructs of the present invention into plant cells (for example by transduction, transfection or transformation) are known to those skilled in the art. Such techniques include *Agrobacterium* mediated introduction, electroporation to tissues, cells and protoplasts, protoplast fusion, injection into reproductive organs, injection into immature embryos and high velocity projectile introduction to cells, tissues, calli, immature and mature embryos. The choice of technique will depend largely on the type of plant to be transformed.

Cells incorporating the genetic constructs of the present invention may be selected, as described above, and then cultured in an appropriate medium to regenerate transformed plants, using techniques well known in the art. The culture conditions, such as temperature, pH and the like, will be apparent to the person skilled in the art. The resulting plants may be reproduced, either sexually or asexually, using methods well known in the art, to produce successive generations of transformed plants.

By 'an effective amount' is meant an amount sufficient to result in an identifiable phenotypic trait in said plant, or in a plant, plant seed or other plant part derived therefrom. Such amounts can be readily determined by an appropriately skilled person, taking into account the type of plant, the route of administration and other relevant factors. Such a person will readily be able to determine a suitable amount and method of administration. See, for example, Maniatis et al, Molecular Cloning: A Laboratory Manual, Cold Spring Harbor Laboratory, Cold Spring Harbor.

In a still further aspect, the present invention provides a method of enhancing bloat safety of a plant, said method including
identifying a gene encoding a polypeptide or polypeptide isoform which is substantially more active in a PA pathway and up-regulating or down-regulating expression of said gene to increase the level of PA in said plant; or
identifying a gene encoding a polypeptide or polypeptide isoform which is substantially more active in an ANT pathway and up-regulating or down-regulating expression of said gene to increase the level of PA in said plant.

In a preferred embodiment, said method may include down-regulating expression of a gene encoding a polypeptide or polypeptide isoform which is active late in the ANT pathway.

For example, for targeted modification of the anthocyanin pathway in a red leaf white clover mutant expressing *TrANR* (anthocyanidin reductase) gene, the modification may include down-regulation of late anthocyanin-specific genes.

Particularly preferred genes include those encoding GST, G3T, UFGT, OMT, ART, ANAT and AAT.

In an alternate preferred embodiment, said method may include up- and/or down-regulating expression of one or more genes encoding a transcription factor.

For example, for targeted modification of the anthocyanin pathway in a red leaf white clover mutant expressing *TrANR* (anthocyanidin reductase) gene, the modification may include up- and/or down-regulation of genes encoding transcription factors.

Particularly preferred genes include those encoding MYB, bHLH, MYC and WDR.

By 'enhancing bloat safety' of a plant is meant reducing the tendency of the plant to cause bloating in an animal which eats the plant.

Preferably, the step of identifying a gene encoding a polypeptide or polypeptide isoform which is substantially more active in a PA or ANT pathway is carried out by a method as hereinbefore described. Alternatively, in certain circumstances the gene may already be indentified and the method may omit the identification step.

In a still further aspect of the present invention, there is provided a genetic construct capable of manipulating the flavonoid biosynthetic pathway in a plant, said genetic construct including a gene encoding a polypeptide or polypeptide isoform which is substantially more active in either a PA or ANT pathway of said plant, or a modified form of said gene.

In a preferred embodiment, the genetic construct according to the present invention may be a vector.

By a 'genetic construct' is meant a recombinant nucleic acid molecule.

By a 'vector' is meant a genetic construct used to transfer genetic material to a target cell.

The vector may be of any suitable type and may be viral or non-viral. The vector may be an expression vector. Such vectors include chromosomal, non-chromosomal and synthetic nucleic acid sequences, eg. derivatives of plant viruses; bacterial plasmids; derivatives of the Ti plasmid from *Agrobacterium tumefaciens*; derivatives of the Ri plasmid from *Agrobacterium rhizogenes*; phage DNA; yeast artificial chromosomes; bacterial artificial chromosomes; binary bacterial artificial chromosomes; vectors derived from combinations of plasmids and phage DNA. However, any other vector may be used as long as it is replicable or integrative or viable in the plant cell.

In a preferred embodiment of this aspect of the invention, the genetic construct may further include a regulatory element and a terminator; said regulatory element, gene and terminator being operably linked.

The regulatory element, gene and terminator may be of any suitable type and may be endogenous to the target plant cell or may be exogenous, provided that they are functional in the target plant cell.

By 'operatively linked' is meant that said regulatory element is capable of causing expression of said gene or modified form thereof in a plant cell and said terminator is capable of terminating expression of gene or modified form thereof in a plant cell. Preferably, said regulatory element is upstream of said gene or modified form thereof and said terminator is downstream of said gene or modified form thereof.

By 'capable of causing expression of said gene' is meant that the gene or modified form thereof and the regulatory element, such as a promoter, are linked in such a way as to permit expression of said gene under appropriate conditions, for example when appropriate molecules such as transcriptional activator proteins are bound to the regulatory sequence.

By 'upstream' is meant in the 3'->5' direction along the nucleic acid.

Preferably the regulatory element is a promoter. A variety of promoters which may be employed in the vectors of the present invention are well known to those skilled in the art. Factors influencing the choice of promoter include the desired tissue specificity of the vector, and whether constitutive or inducible expression is desired and the nature of the plant cell to be transformed (eg. monocotyledon or dicotyledon). Particularly suitable constitutive promoters include the Cauliflower Mosaic Virus 35S (CaMV 35S) promoter.

A variety of terminators which may be employed in the vectors of the present invention are also well known to those skilled in the art. The terminator may be from the same gene as the promoter sequence or a different gene. Particularly suitable terminators are polyadenylation signals, such as the CaMV 35S polyA and other terminators from the nopaline synthase (nos) and the octopine synthase (ocs) genes.

The genetic construct, in addition to the regulatory element, the gene or modified form thereof and the terminator, may include further elements necessary for expression of the gene or modified form thereof, in different combinations, for example vector backbone, origin of replication (ori), multiple cloning sites, spacer sequences, enhancers, introns (such as the maize Ubiquitin *Ubi* intron), antibiotic resistance genes and other selectable marker genes [such as the neomycin phosphotransferase (*npt2*) gene, the hygromycin phosphotransferase (*hph*) gene, the phosphinothricin acetyltransferase (*bar* or *pat*) gene], and reporter genes (such as beta-glucuronidase (GUS) gene (*gusA*)]. The genetic construct may also contain a ribosome binding site for translation initiation. The genetic construct may also include appropriate sequences for amplifying expression.

As an alternative to use of a selectable marker gene to provide a phenotypic trait for selection of transformed host cells, the presence of the genetic construct in transformed cells may be determined by other techniques well known in the art, such as PCR (polymerase chain reaction), Southern blot hybridisation analysis, histochemical assays (e.g. GUS assays), thin layer chromatography (TLC), northern and western blot hybridisation analyses.

Those skilled in the art will appreciate that the various components of the genetic construct are operatively linked, so as to result in expression of said gene or modified form thereof. Techniques for operatively linking the components of the genetic construct of the present invention are well known to those skilled in the art. Such techniques include the use of linkers, such as synthetic linkers, for example including one or more restriction enzyme sites.

Preferably, the genetic construct is substantially purified or isolated.

By 'substantially purified' is meant that the genetic construct is free of the genes, which, in the naturally-occurring genome of the organism from which the nucleic acid or promoter is derived, flank the nucleic acid or promoter. The term therefore includes, for example, a genetic construct which is incorporated into a vector; into an autonomously replicating plasmid or virus; or into the genomic DNA of a prokaryote or eukaryote; or which exists as a separate molecule (eg. a cDNA or a genomic or cDNA fragment produced by PCR or restriction endonuclease digestion) independent of other sequences. It also includes a genetic construct which is part of a hybrid gene encoding additional polypeptide sequence.

Preferably, the substantially purified genetic construct is at least approximately 90% pure, more preferably at least approximately 95% pure, even more preferably at least approximately 98% pure.

The term "isolated" means that the material is removed from its original environment (eg. the natural environment if it is naturally occurring). For example, a naturally occurring nucleic acid present in a living plant is not isolated, but the same nucleic acid separated from some or all of the coexisting materials in the natural system, is isolated. Such nucleic acids could be part of a vector and/or such nucleic acids could be part of a composition, and still be isolated in that such a vector or composition is not part of its natural environment.

Preferably, the gene included in the genetic construct of the present invention is identified by a method as hereinbefore described.

In a preferred embodiment, the gene may encode a polypeptide or polypeptide isoform which is active late in the ANT pathway. For example, the late ANT-pathway enzyme may be selected from the group consisting of GST, G3T, UFGT, OMT, ART, ANAT and AAT.

In an alternate preferred embodiment, the gene may encode a transcription factor. For example, the transcription factor may be selected from the group consisting of MYB, bHLH, MYC and WDR.

In a further aspect of the present invention there is provided a transgenic plant cell, plant, plant seed or other plant part with modified flavonoid biosynthetic characteristics relative to an untransformed control plant; said plant cell, plant, plant seed or other plant part including a genetic construct or vector according to the present invention.

By 'modified flavonoid biosynthetic characteristics' is meant that the transformed plant exhibits increased flavonoid biosynthesis and/or contains increased levels of soluble carbohydrate relative to an untransformed control plant.

Preferably, said transformed plant exhibits increased PA biosynthesis and/or contains increased levels of PA relative to an untransformed control plant.

In a preferred embodiment, the transgenic plant cell, plant, plant seed or other plant part with modified flavonoid biosynthetic characteristics has an increase in soluble carbohydrate, preferably an increase in PA, of least approximately 15%, more preferably at least approximately 25%, more preferably at least approximately 35%, more preferably at least approximately 50% relative to an untransformed control plant.

For example, soluble carbohydrate, preferably PA, may be increased by between approximately 15% and 500%, more preferably between approximately 25% and 300%, more preferably between approximately 35% and 200%, more preferably between approximately 50% and 100% relative to an untransformed control plant.

Preferably the transgenic plant cell, plant, plant seed or other plant part is produced by a method according to the present invention.

The present invention also provides a transgenic plant, plant seed or other plant part derived from a plant cell of the present invention and including a genetic construct or vector of the present invention.

The present invention also provides a transgenic plant, plant seed or other plant part derived from a plant of the present invention and including a genetic construct or vector of the present invention.

The plant cell, plant, plant seed or other plant part may be from any suitable species, including dicotyledons, moncotyledons and gymnosperms. In a preferred embodiment the plant cell, plant, plant seed or other plant part may be from a dicotyledon, preferably forage legume species such as clovers (*Trifolium* species) and medics (*Medicago* species), more preferably white clover (*Trifolium repens*), red clover (*Trifolium pratense*), subterranean clover (*Trifolium subterraneum*) and alfalfa (*Medicago sativa*).

Preferably, the transgenic plant cell, plant, plant seed or other plant part is a clover species, more preferably white clover, or an alfalfa species.

For example, the present invention enables the production of clover plants with increased PA in leaf blades, for improved nutrition for grazing animals.

By 'plant cell' is meant any self-propagating cell bounded by a semi-permeable membrane and containing a plastid. Such a cell also requires a cell wall if further propagation is desired. Plant cell, as used herein includes, without limitation, algae, cyanobacteria, seeds suspension cultures, embryos, meristematic regions, callus tissue, leaves, roots, shoots, gametophytes, sporophytes, pollen and microspores.

By 'transgenic' is meant any cell which includes a DNA sequence which is inserted by artifice into a cell and becomes part of the genome of the organism which develops from that cell. As used herein, the transgenic organisms are generally transgenic plants and the DNA (transgene) is inserted by artifice into either the nuclear or plastidic genome.

The methods of the present invention may be applied to a variety of plants, including monocotyledons [such as grasses (e.g. forage and bioenergy grasses including perennial ryegrass, tall fescue, Italian ryegrass, red fescue, reed canarygrass, big bluestem, cordgrass, napiergrass, wildrye, wild sugarcane, *Miscanthus,* switchgrass), corn or maize, rice, wheat, barley, sorghum, sugarcane, rye, oat) and energy crops (e.g. energy cane, energy sorghum)], dicotyledons [such as *Arabidopsis,* tobacco, soybean, canola, alfalfa, potato, cassava, clovers (e.g. white clover, red clover, subterranean clover), vegetable brassicas, lettuce, spinach] and gymnosperms.

Preferably, the methods are applied to alfalfa and clover, more preferably white clover.

As used herein, except where the context requires otherwise, the term "comprise" and variations of the term, such as "comprising", "comprises" and "comprised", are not intended to exclude further additives, components, integers or steps.

As used herein, except where the context requires otherwise, the term "include" and variations of the term, such as "including", "includes" and "included", may have the same meaning as the term "comprise" and variations of the term.

### Detailed description of the embodiments

The present invention will now be more fully described with reference to the accompanying examples and drawings. It should be understood, however, that the description following is illustrative only and should not be taken in any way as a restriction on the generality of the invention described above.

### Brief description of the drawings / figures

In the figures:

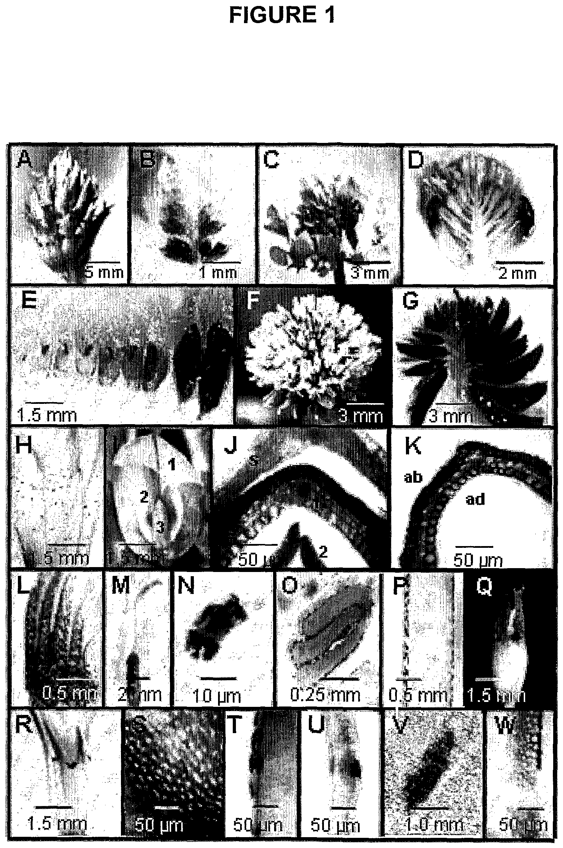

**Figure 1****.** Accumulation of Proanthocyanidins and Anthocyanins in White Clover Organs and Tissues at Different Stages of Development. **(A)** to **(M)** 4-Dimethylaminocinnemaldehyde staining of PA in inflorescences and flowers. **(N)** to **(P)** Staining of PA in vegetative organs. **(Q)** to **(W)** Visualisation of anthocyanins in florets and leaves. ab-abaxial; ad-adaxial; c-carpels; s-sepals, st-stamens; 1-standard petal; 2-lateral wing petals; 3-keel petals.

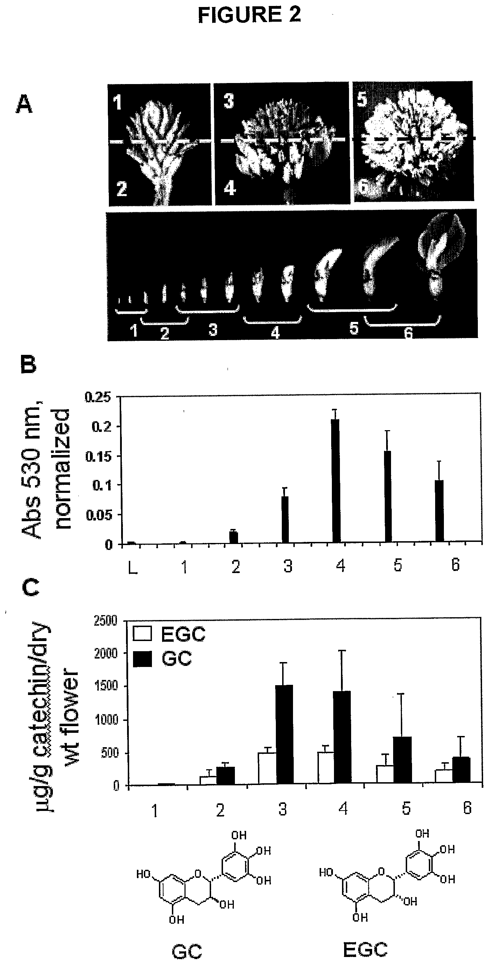

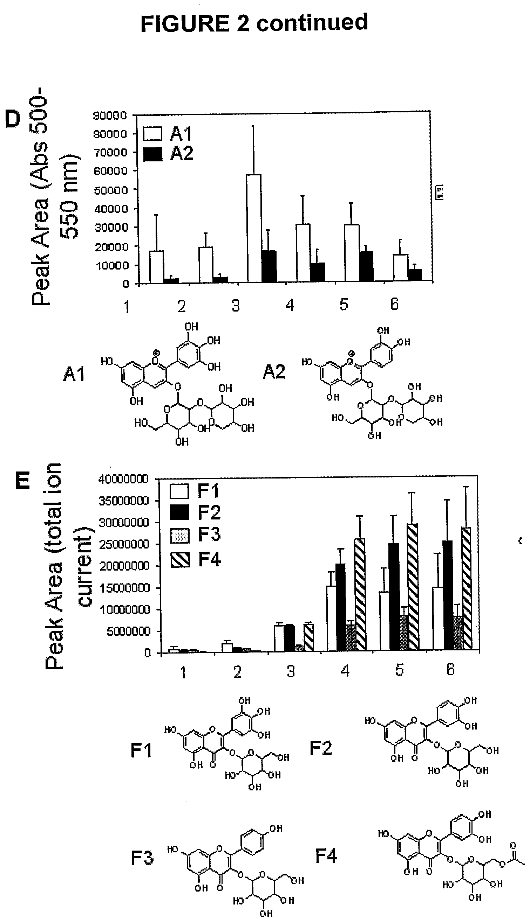

**Figure 2****.** Analysis of Flavonoid Levels in White Clover Inflorescences. **(A)** Appearance of white clover inflorescences and flowers at six stages of development. **(B)** Proanthocyanidin level. **(C)** Level and composition of free 2,3-flavan-3-ols. Black bars- GC, open bars-EGC. **(D)** Level and composition of anthocyanins. Open bars-A1, black bars- A2. **(E)** Level and composition of flavonol glycosides. Open bars-F1, black bars-F2, grey bars-F3, cross-hatched bars-F4. GC-gallocatechin, EGC-epigallocatechin. A1-delphinidin-3-sambudioside, A2-cyanidin-3-sambudioside; F1-myricetin glycoside, m/z 479; F2-quercetin glycoside, m/z 463; F3-kaempferol glycoside, m/z 477; F4-quercetin quercetin acetyl-glycoside, m/z 505.

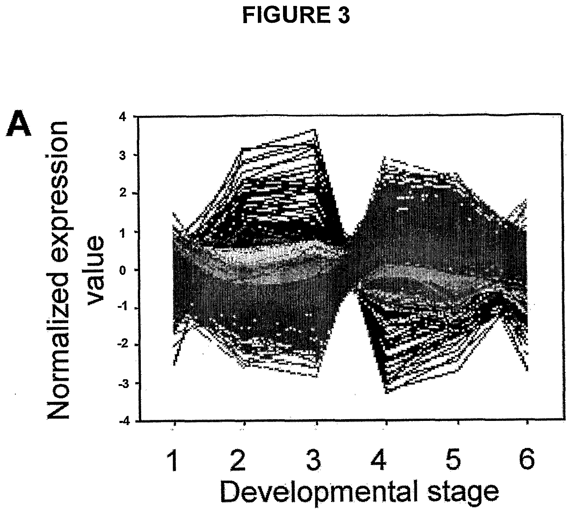

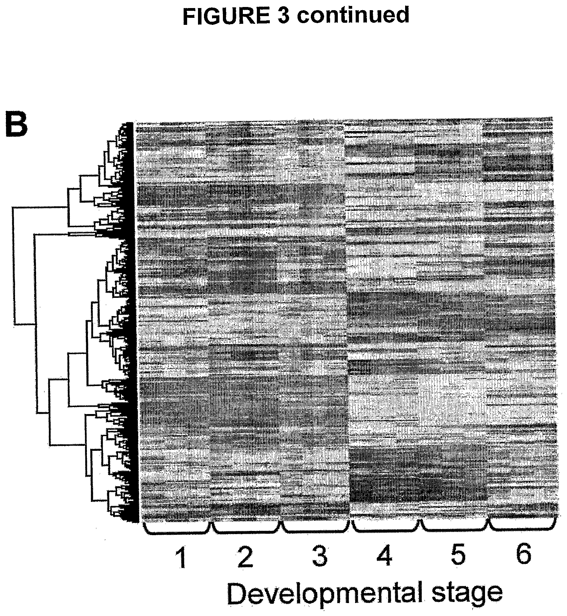

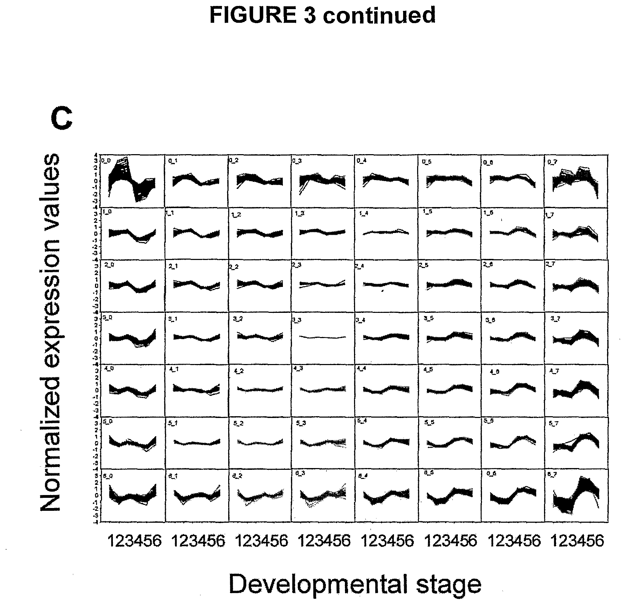

**Figure 3****.** Normalized Expression Data from Genes Differentially-Expressed (*p*≥ 0.05). **(A)** Line plot showing gene expression patterns across 6 developmental stages of white clover flowers. The black lines represent genes up-regulated at stages 1-3 and down-regulated at stages 4-6. The medium grey lines represent genes down-regulated at stages 1-3 and up-regulated at stages 4-6. The light grey lines represent genes constitutively expressed. **(B)** Heat map derived from hierarchical clustering of genes showing similar expression patterns across the six developmental stages. For each of the stages represented the darker to grey colour depicted the higher the observed gene expression. **(C)** A self organising map with 7 X 8 output nodes organising genes into 56 clusters of similar gene expression.

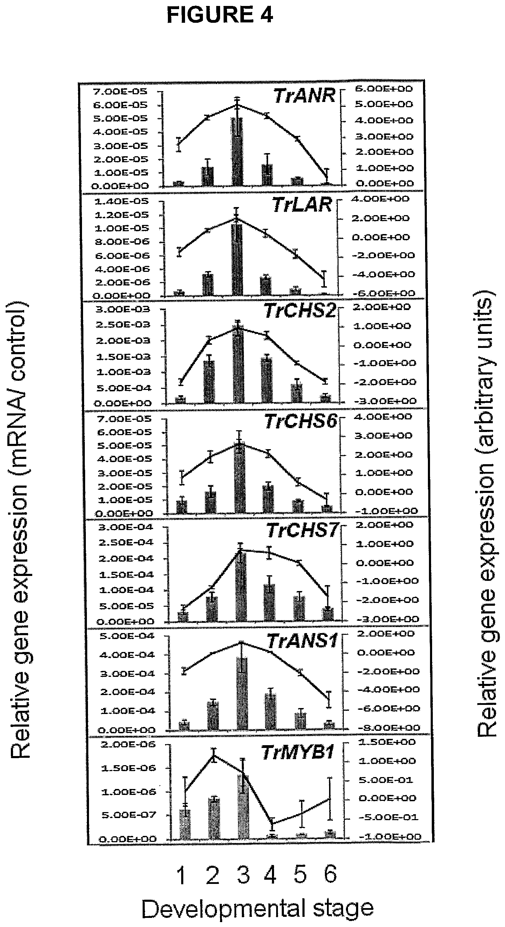

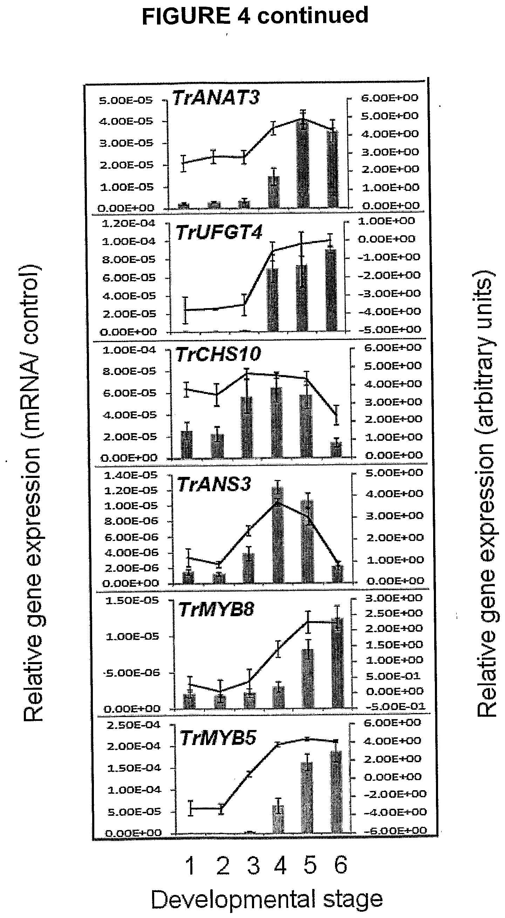

**Figure 4****.** Transcript Levels of Selected Genes at Six Stages of White Clover Flower Development. Normalized relative transcript levels of indicated genes determined by real-time RT-PCR are shown as bars (scale on the left) and microarray results (scale on the right), as lines. Numbers on the x-axes represent developmental stages.

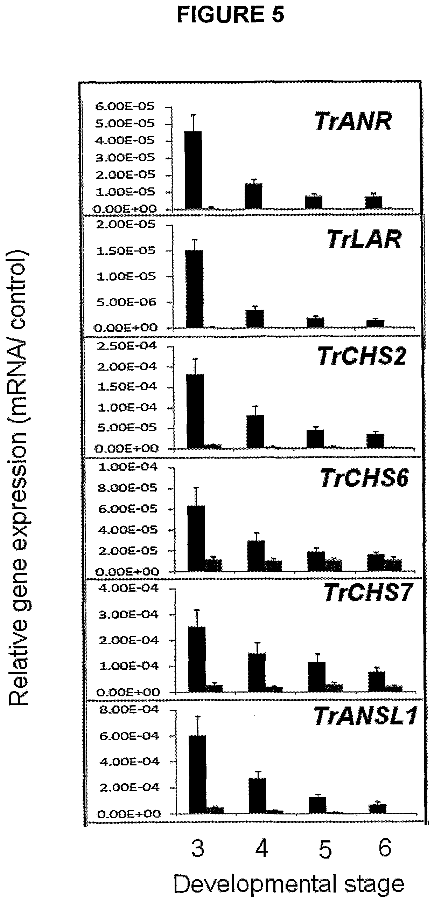

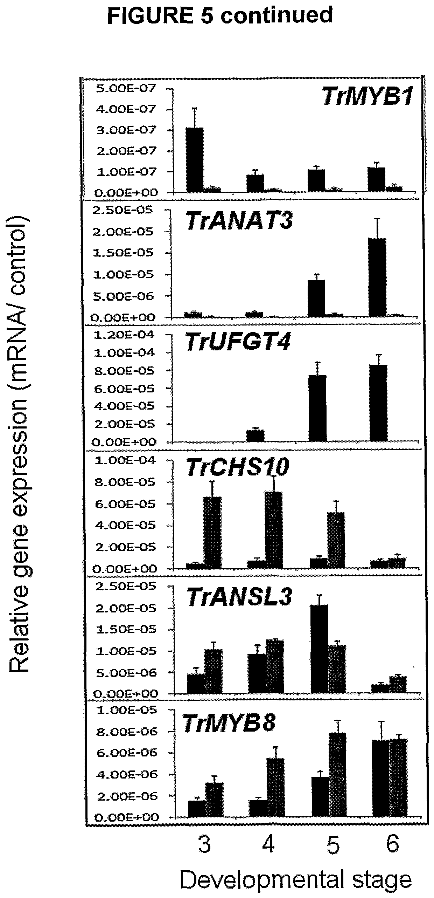

**Figure 5****.** Organ-Specific Expression of Selected Genes at Developmental Stages 3, 4, 5 and 6 of White Clover Flower Development. Normalized relative transcript levels of indicated genes were determined by real-time RT-PCR. Black bars represent expression in inner whorls (petals, carpel and stamens). Grey bars represent expression in sepals. Numbers on the x-axes represent developmental stages.

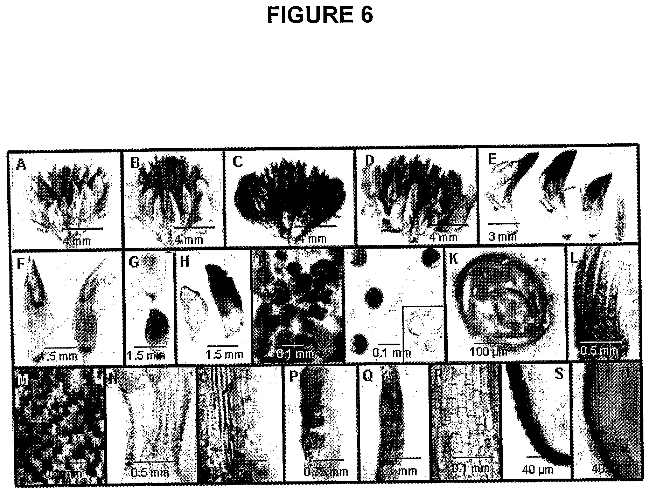

**Figure 6****.** Phenotypes of Flowers from White Clover Lines Containing a dsRNAi Construct Targeting *TrANR.* **(A)** to **(C)** 50% open inflorescences and flowers of *TrANR* dsRNAi lines showing white (6-10A), pink (6-8A) and red (6-14D) flower phenotypes, respectively. **(D)** Inflorescence of the 6-14D line at stages 5 and 6. **(E)** Individual flowers of the 6-14D line at different developmental stages. **(F)** Immature inflorescences at stage 3 of the wild-type (left) and 6-14D line (right). **(G)** Standard petals of wild-type (upper image) and line 6-14D (lower image) at stage 3. **(H)** Wing (right) and keel (left) petals at stage 3. **(I)** Petal epidermal cells under x X magnification. **(J)** Protoplasts isolated from petals of transgenic and wild-type plants (bottom left). **(K)** Cross-section of a line 6-14D flower at stage 3. **(L-M)** Anther filaments of wild-type plants stained with DMACA (stage 3) under X3.2 and X16 magnification, respectively. **(N-O)** Anther filaments of line 6-14D (stage 3) under X3.2 and X16 magnification, respectively. **(P)** Carpels of wild-type plants stained with DMACA (stage 3). **(Q-R)** Carpel of line 6-14D under X1.5 and X16 magnification, respectively. **(S-T)** Cross-section of a carpel from a wild-type plant stained with DMACA and an unstained carpel from line 6-14D (stage 4) under x40 magnification.

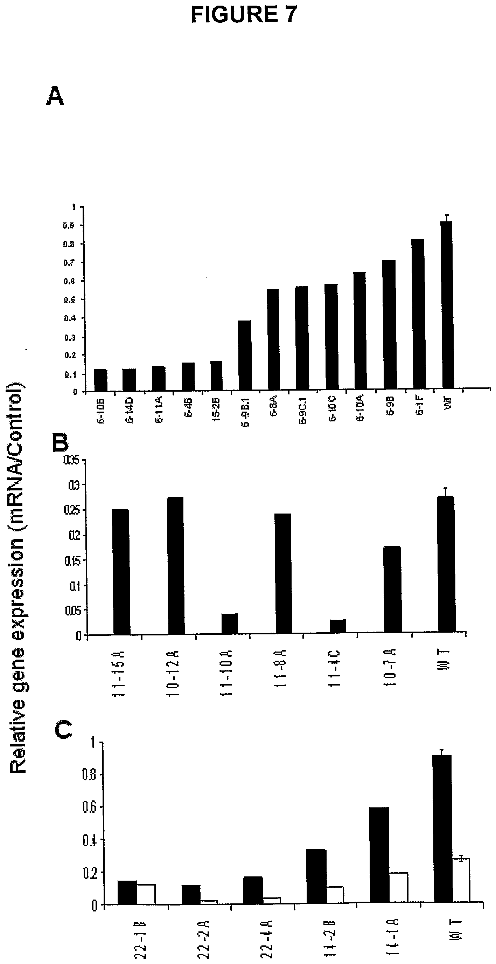

**Figure 7****.** Analysis of *TrANR* and *TrLAR* Transcript Levels in White Clover Lines Containing dsRNAi Constructs Targeting *TrANR* and *TrLAR.*
   **(A)** Transcript levels of the *TrANR* gene in *TrANR* dsRNAi lines.
   **(B)** Transcript levels of the *TrLAR* gene in *TrLAR* dsRNAi lines.
   **(C)** Transcript levels of the *TrLAR* and *TrANR* genes in *TrANR-TrLAR* dsRNAi lines. Normalized relative transcript levels were determined in 50% open inflorescences of the indicated lines by real-time RT-PCR. Black bars: *TrANR*, open bars: *TrLAR.* WT-wild-type.

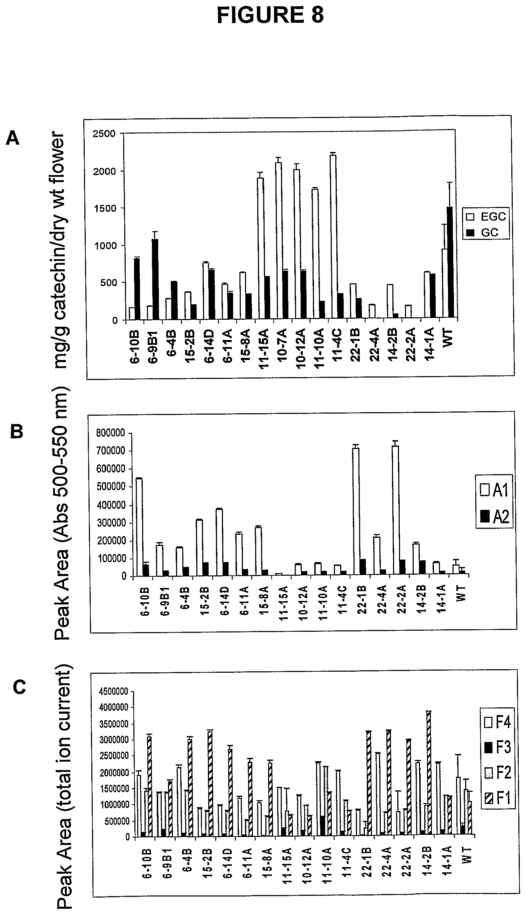

**Figure 8****.** Analysis of Flavonoid Levels in White Clover Lines Containing dsRNAi Constructs Targeting *TrANR* and *TrLAR.*
   **(A)** to **(C)** Level and composition of flavonoid pathway products in 50% open inflorescences of wild-type lines and transgenic lines in which *TrANR*, *TrLAR* or both genes were targeted by dsRNAi constructs. **(A)** Free flavan-3-ols. **(B)** Anthocyanins. **(C)** Flavonol glycosides. GC-gallocatechin, EGC-epigallocatechin. A1-delphinidin-3-sambudioside, A2-cyanidin-3-sambudioside; F1- myricetin glycoside, m/z 479; F2-quercetin glycoside, m/z 463; F3-kaempferol glycoside, m/z 477; F4-quercetin glycoside, m/z 505. Lines 6 and15-TrANR dsRNAi; lines10 and 11-TrLAR dsRNAi; lines 14 and *22-TrANR-TrLAR* dsRNAi; wt-wild-type cv 'Mink'.

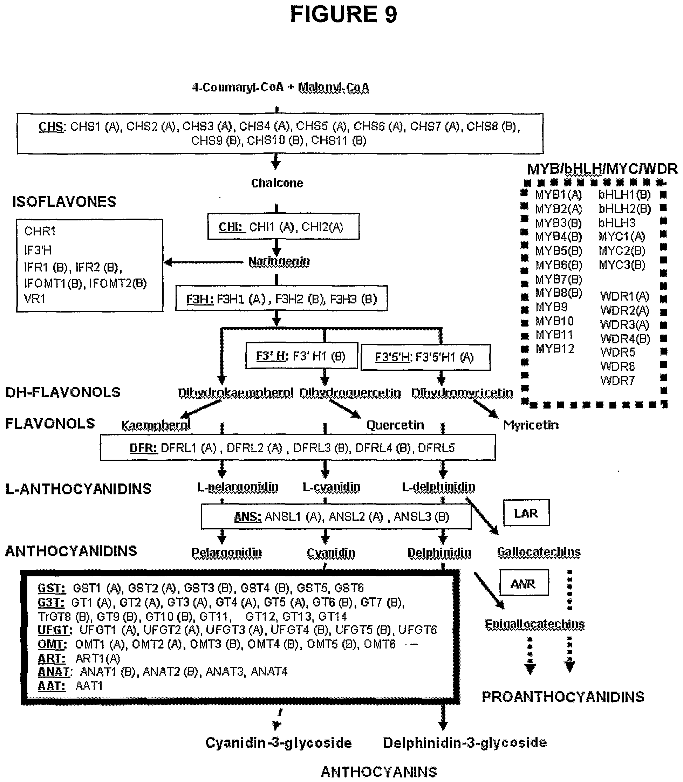

**Figure 9****.** A Model for Flavonoid Biosynthesis in White Clover Flowers Based on Biochemical and Transcriptomic Data.
   Specific compounds are listed in the lower case. Classes of compounds are listed in bold type. Enzymes are shown as open boxes, with preferred late-anthocyanin-specific genes highlighted in the heavy box and preferred transcription factors highlighted in the dotted box. Compounds and genes marked with an asterisk (*) were up-regulated in *TrANR* dsRNAi lines. Those marked with a hash (#) were down-regulated in *TrANR* dsRNAi lines.

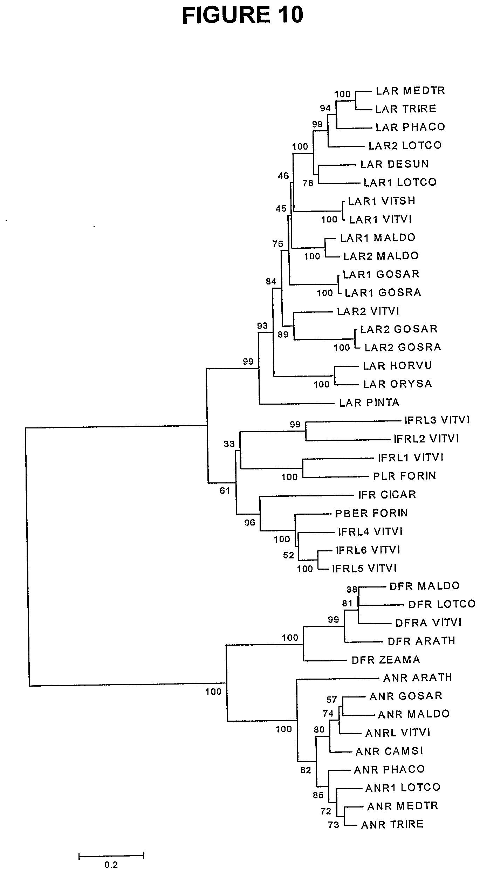

**Figure 10****.** Phylogenetic tree of several classes of reductase-epimerase-dehydrogenase (RED) proteins: flavonol synthases (FLS), isoflavone reductases (IFR) and isoflavone reductase-like proteins (IFRL), phenylcoumaran benzylic ether reductases (PBER), (+)-pinoresinol/(+)-lariciresinol reductase protein (PLR), flavanone 3-hydroxylases (F3H), leucoanthocyanidin reductases (LAR), anthocyanidin reductases (ANR) and anthocyanidin reductase-like proteins (ANRL) involved in flavonoid biosynthesis. The phylogenetic tree was constructed from a ClustalW alignment using the neighbor-joining method in the MEGA4.0.2 package. VITV*-Vitis vinifera,* PINTA-*Pinus taeda,* LOTCO*-Lotus corniculatus,* TRIRE*-Trifolium repens,* MEDTR*-Medicago truncatula,* PHACO*-Phaseolus coccineus,* ORYSA-*Oryza sativa,* HORVU*-Hordeum vulgare,* GOSAR-*Gossypium arboretum,* GOSRA-Gossypium *raimondii,* VITSH-*Vitis shuttleworthii* , MALDO-*Malus x domestica,* ARATH*-Arabidopsis thaliana,* DESUN-*Desmodium uncinatum*, ZEAMA-Zea *mays*, CAMSI-*Camellia sinensis FORIN-Forsythia x intermedia*, CICAR-*Cicer arietinum.* The accession numbers are as follows. Swissprot: Q84V83.1, Q00016.1. P51110.1. Genbank: CAI56335.1, CAI56334.1, CAI56333.1, CAI56332.1, CAI56330.1, CAI56331.1, AAC49608.1, AAF64174.1, CAD91910.1, CAI56323.1, CAI56324.1, CA156319.1, CAI56325.1, CAI56320.1, AAN77735.1, CAI56327.1, CAI56328.1, FJ842544, FJ842546, CAD91909.1, CAI56322.1, CAI56321.1, CAD91911.1, CAI56326.1, CAI26310.1, CAI26308.1, CAA28734.1, AAZ79363.1, AAZ79364.1, AAZ79365.1, BAB92999.1, AAT68773.1, ABC71337.1, AAV71171.1, ABC71324.1, ABC71328.1, AAV71171.1. RefSeq: NP_199094.1, NP_176365.1.

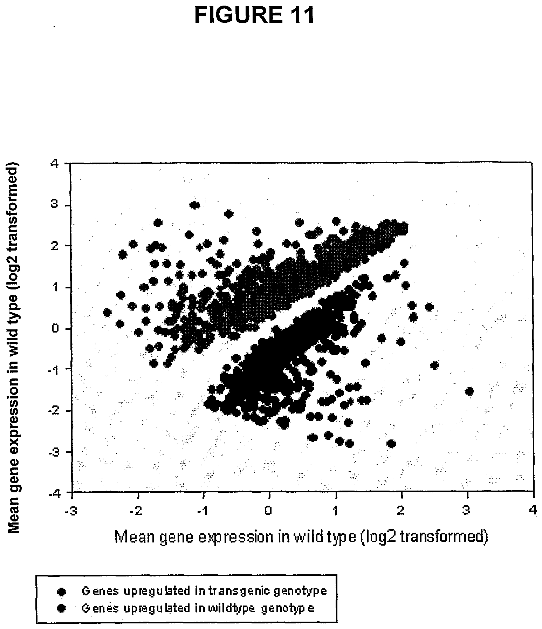

**Figure 11****.** Normalised gene expression data from genes showing a significantly different expression (*p*≥ 0.05) in flowers of 3 *TrANR* dsRNAi lines in comparison to 3 wild-type white clover plants. Grey dots represent genes up-regulated in *TrANR* dsRNAi lines compared to wild-type plants. Black dots represent genes down-regulated in *TrANR* dsRNAi lines compared to wild-type plants.

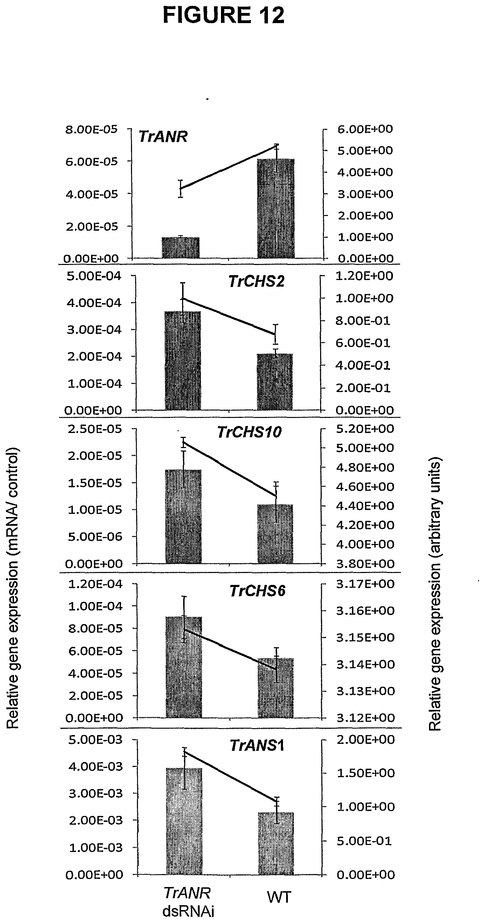

**Figure 12****.** Transcript Levels of Selected Genes in *TrANR* dsRNAi Lines in Comparison to Wild-Type Plants. Normalized relative transcript levels of indicated genes, as determined by real-time RT-PCR, are shown as bars (scale on the left) and microarray results (scale on the right) as lines.
**Table 1.** Transcripts Induced at Stages 1-3 of Flower Development in White Clover
**Table 2.** Transcripts Induced at Stages 4-6 of Flower Development in White Clover
**Table 3.** Transcripts Up-Regulated in Flowers of *TrANR* dsRNAi Lines, Relative to Wild-Type Plants. Genes marked with an asterisk (*) were up-regulated at flower stages 1-3 in wild-type plants. Genes marked with a hash (#) were up-regulated at stages 4-6 in wild-type plants.
**Table 4.** Transcripts Down-regulated in in Flowers of *TrANR* dsRNAi Lines, Relative to Wild-Type Plants. Genes marked with an asterisk (*) were up-regulated at flower stages 1-3 in wild-type plants. Genes marked with a hash (#) were up-regulated at stages 4-6 in wild-type plants.
**Table 5.** List of Primers Used for Real Time RT-PCR Analysis

### Example 1 - Methods

### Plant Growth Conditions

Wild type and transgenic white clover lines were vernalised in a controlled growth room for 6 weeks at 5°C with an 8 h photoperiod and a light intensity of 41+/-5 µmol-m⁻¹-s⁻¹ at canopy height. Flowering was then induced in a controlled growth cabinet (Enconair) by growing plants for 4 weeks at 22°C with a 16 hour photoperiod and a light intensity of 240+/-30 µmol-m⁻¹-s⁻¹ at canopy height.

### Generation of Transgenic Plants

Transgenic white clover plants (*Trifolium repens* L. cv Mink) were generated by *Agrobacterium*-mediated transformation using cotyledonary explants and selection with 50 mg/L kanamycin sulfate as previously described (Ding et al., 2003). DNA was extracted from leaf tissue of putative transgenic lines using the Wizard DNA purification kit (Promega) and screened by real-time PCR for the presence of the *npt2* selectable marker gene using the primers 5'-GGCTATGACTGGGCACAACA-3' and 5'-ACCGGACAGGTCGGTCTTG-3'. PCR mixtures were set up in a laminar flow hood with aerosol-free pipette tips using SYBR Green PCR Master Mix (cat# 4309155, Applied Biosystems), according to the manufacturers instructions, using at least 2 technical replicates and a 25 µl reaction volume. Thermal cycling was performed with a MX3000P thermal cycler (Stratagene) using the following cycling conditions for the detection of the *npt2* gene: 10 mins at 95°C; 40 cycles of 30 sec at 95°C, 30 sec at 60°C and 30 sec at 72°C; 1 min at 95°C, 30 sec at 55°C and 30 sec at 95°C.

### Visualisation of Proanthocyanidins and Anthocyanins

Plant material was stained for the presence of proanthocyanidins and monomeric flavan-3-ols using 0.01% (w/v) 4-dimethylaminocinnemaldehyde (DMACA) in absolute ethanol containing 1% (w/v) concentrated hydrochloric acid (McMurrough and McDowell, 1978). Anthocyanins were visualized in untreated white clover tissues. Images were captured using a Leica MZFLIII light microscope (Leica Microsystems) fitted with a CCD camera.

### Biochemical Analysis of Flavonoids

A semi-quantitative PVPP-butanol-HCl assay was used to measure total proanthocyanidin levels in 5 -10 mg samples of freeze-dried, finely ground white clover material (Ray et al., 2003). Samples were analyzed using a spectrophotometer (Nanodrop) and final values were normalized against the mass of individual samples. Three biological replicates were performed. Flavonol glycosides, flavan-3-ols and anthocyanins were identified and quantified by LC-MS analysis. Three technical replicates of freeze dried, finely ground plant material (approximately 5 mg) were extracted three times in 0.5 ml aliquots of 80% methanol in water. The combined extracts were dried with gentle warming under a stream of nitrogen and reconstituted in 200 µl of 80% methanol/water. An Agilent 1100 series HPLC system (Waldbronn) equipped with a quaternary gradient pump, column heater, autosampler with sample cooler (maintained at 4°C), and diode array detector (data acquired over 190-800 nm), coupled to a Thermo Electron LTQ ion trap mass spectrometer was used for LC-MS analysis. 5 µl aliquots of each sample were injected onto a 150 x 2.1 mm id., 3µ, Thermo BDS Hypersil C18 column maintained at 40°C. The mobile phase consisted of two components: A (water with 0.1% formic acid) and B (acetonitrile with 0.1% formic acid); and followed the gradients at a flow rate of 0.2 ml/min: Gradient 1: 0-5 min, 98% A; 5-25 min, 62% A; 26-35 min, (0.3ml/min) 98% A.

For identification of metabolites, LC-MS was run in polarity switching mode with MSⁿ data acquired in both negative and positive modes. Analysis of the ESI negative mode MS and MSⁿ data allowed the identification of four flavonol glycosides, and analysis of the ESI positive mode MS and MSⁿ data along with the PDA data allowed the identification of two anthocyanins. For enhanced sensitivity needed to quantify metabolites, LC-MS data was acquired in ESI negative mode with a mass range limited to 200 to 1000 amu. Prior to data acquisition the system was tuned using a 20 µg/ml standard of epicatechin (EC). Standard curves for EC and epigallocatechin (EGC) were prepared by serial dilution of stock solutions and analysed in conjunction with the samples. The results were linear over the range examined (8-285 ng for EGC, 5-81 ng for GC). Standards for the flavonol glycosides and anthocyanins were not obtained and absolute quantitation was not possible. Results were based on relative levels of the metabolites in each sample, based on the area of the peak for the [M-H]⁻ ion for the flavonols and for the UV-Vis absorption (500-550 nm) peak area for the anthocyanins.

### Characterisation of the White Clover ANR and LAR Genes

cDNA clones containing the white clover *ANR* and *LAR* genes were identified using the sequences of the *Arabidopsis thaliana BANYULS* gene and the *Desmodium unicinatum LAR* gene as input data for BLAST searches of a white clover EST database (Altschul et al., 1997; Sawbridge et al. 2003). The deduced protein sequences of the white clover *ANR* and *LAR* genes were compared to sequences of related genes in the reductase-epimerase-dehydrogenase (RED) superfamily by constructing a phylogenetic tree with bootstrapping from a ClustalW alignment using the neighbor-joining method in the MEGA4.0.2 package (Tamura et al., 2007; Kumar et al., 2008).

### Preparation of Constructs for Plant Transformation

cDNA clones in pGEM-T Easy (Promega, Madison, USA) encoding the white clover *ANR* and *LAR* genes were previously generated as part of an EST discovery project (Sawbridge et al., 2003). The characterized *TrANR* and *TrLAR* cDNA clones were used as templates for PCR reactions. A 331 bp fragment from the 3' end of *TrANR* was amplified using the primers 5'-attB1-ATGCAGTTTCTGTCGGGTTC-3' and 5'-attB2-ATCAAAATCTAATTCTTCAGTGC-3'. A 386 bp fragment from the 3' end of *TrLAR* was amplified using the primers 5'-attB1-TGAATGAGCTTGCTTCTTTGTG-3' and 5'-attB2-TAGATCCACCTCAGGTGAACC-3'. These PCR products were inserted into pDONR221 and fully sequenced clones were introduced into a GATEWAY®-enabled plant expression vector containing *TrANR* and *TrLAR* in hairpin constructs under the control of an enhanced CaMV 35S promoter and the 35S terminator and named *TrANR* dsRNAi and *TrLAR* dsRNAi, respectively. A 335 bp PCR fragment amplified from *TrANR* using the primers 5'-ATGCAGTTTCTGTCGGGTTC-3' and 5'-AGCAAGCTCATTCAATCAAAATCTAATTCTTCAGTGC-3' and a 371 bp PCR fragment amplified from *TrLAR* using the primers 5'-GAATTAGATTTTGATTGAATGAGCTTGCTTCTTTGTG-3' and 5'-TGAACCTTTTCAACAGGAAGC-3' were used as a template for a secondary PCR reaction using the GATEWAY primers 5'-attB1-ATGCAGTTTCTGTCGGGTTC-3' and 5'-attB2-TAGATCCACCTCAGGTGAACC-3'. The 706 bp product, containing sequences from *TrANR* and *TrLAR,* was inserted into pDONR221 and a fully-sequenced clone was introduced into the GATEWAY®-enabled plant expression vector to produce *TrANR-TrLAR* dsRNAi.

### Analysis of Gene Expression in White Clover

Proprietary Combimatrix CustomArray software was used to design single oligonucleotide probes of 35 to 40 bases in length for each white clover unigene. The resulting probe set was then assigned to a Combimatrix Custom 12k array.

To analyse differential expression of the genes at six developmental stages, samples were taken from the upper and lower halves of immature, 50% open and mature inflorescences in wild-type white clover, *cv* Mink. In order to test the effect of down-regulating *TrANR* on global gene expression, the 50% open inflorescences were harvested from wild-type white clover and red-flowered *TrANR* dsRNAi lines. Both microarray experiments involved three biological replicates, represented by different genotypes or transformation events, and two technical replicates.

RNAs were extracted using the CTAB-based method of Chang et al. (1993) and were further purified using an RNeasy® Mini kit following the manufacturer's protocol (QIAGEN). The RNA samples were amplified and labelling was performed using the MessageAmp™II aRNA Amplification Kit (Ambion) and Biotin-ULS aRNA Fluorescent Labelling Kit (Kreatech), according to the manufacturers' instructions. Each sample was hybridized to a separate array following the protocol recommended by the manufacturer (CombiMatrix). Slides were labeled, post-hybridisation, with streptavidin-cy5 according to the manufacturer's protocols (http://www.combimatrix.com). Slides were re-used up to 4 times and were stripped between uses with the Combimatrix stripping reagent as per http://www.combimatrix.com. The hybridized arrays were scanned with an Axon GenePix4000B instrument. Data was extracted using Combimatrix Microarray Imager software (http://webapps.combimatrix.com/customarray/customarrayHome.jsp).

Background subtraction was performed by computing the mean signal intensity from the faintest 5% of all probes plus two standard deviation units, and deducting this value from all spots on the array. A minimum floor value was then set at 20 to eliminate any zero or negative spot values. The data on each array was then normalized using global median normalization (Draghici 2003) prior to being LOG₂ transformed. Significant differences in gene expression levels between treatments were identified using analysis of variance (ANOVA) using the MAANOVA Bioconductor package (http://cran.r-project.org/src/contrib/Descriptions/maanova.html) (Wu *et al.* 2003). Genes that showed a difference with a significance of P≥0.05 were identified as showing markedly different gene expression between the treatments. Genes showing similar expression profiles across the 6 phenological ranges of flower development in the first experiment were identified using self organizing maps in the SOM package from the R statistical programming environment (http://www.r-project.org/) and hierarchical clustering from the Bioconductor package (http://www.bioconductor.org).

Thirteen white clover flavonoid genes representing different expression profiles and four internal control genes were selected for validation of microarray data. The housekeeping gene, elongation factor 1-alpha (Ef1-α), was also included. A standard curve method for absolute quantitation was used with DNA standards of known concentration for each gene. Reverse transcription of 1 µg of RNA was performed using Transcriptor First Strand cDNA Synthesis kit (Roche) according to the manufacturer's recommendations. A list of the primers is shown in Table S5. The thermal profile: 95°C 10 min, [95.0°C 30 sec, 60.0°C 30 sec]× 40, melting curve protocol began immediately after amplification and consisted of 95°C 1 min, 60°C 1 min, 20 min ramp time from 60°C to 95°C followed by 95°C for 30 sec. Duplicate controls included RT-PCR reactions lacking reverse transcriptase or no template. Expression values were normalised by geometric averaging of four internal control genes encoding glyceraldehyde-3P-dehydrogenase (*GAPDH*), elongation factor 1-alpha (*EF1α*), histone H4 (*HH4*) and S-adenosylmethionine (*SAMS*), using geNorm software (PrimerDesign Ltd).

### Accession Numbers

Sequence data can be found in the GenBank/EMBL database under the following accession numbers: *TrANR*, FJ842544 and TrLAR, FJ842546.

### Example 2

### Proanthocyanidins and Anthocyanins are Co-Localized in Floral Epidermal Cells

PAs and their monomers were histochemically stained in white clover organs and tissues using DMACA (Figure 1). Floral organs stained strongly indicating that a high level of PA and 2,3-flavan-3-ol monomers were present. Accumulation of PAs in inflorescences at immature, partially (50%) open and mature stages of development is shown in Figure 1 (A-G). The accumulation of PAs appeared to be developmentally regulated within all three developmental stages as indicated by intense staining of the oldest florets located at the base of each inflorescence (Figure 1B, D,E,G). White clover flowers have a calyx that consists of 5 fused sepals in which PAs or their monomers were detected only in multicellular trichomes (Figure 1H). The white or pale pink asymmetrical corolla contains 5 petals: a single large standard petal and two lateral wing petals, which enclose two interior keel petals (Figure 1I). At early stages of flower development, PA accumulation was most clearly seen in the standard petal (Figure 1J-K), followed by the inner, wing and keel petals. PA accumulation appeared to start in epidermal cells located on the abaxial side of the petal and proceed to epidermal cells on the adaxial side during development (Figure 1J-K). The bases of all five petals are fused to a tube of 10 stamens. A mosaic pattern of PA accumulation was detected on the abaxial side of stamen filaments (Figure 1L). A single carpel is located within the staminal tube. Figure 1M shows accumulation of PA in carpels. PAs or their monomers were detected only in multicellular trichomes of aerial vegetative organs of white clover, including peduncles, stolons, stipules, petioles and leaves (Figure 1N-P). Trichomes staining heavily with DMACA were seen in leaves at stage 0.2 (Thomas, 1987, Figure 1O). Accumulation of PAs in peduncles was similarly restricted to trichomes (Figure 1P).

ANTs accumulated in both epidermal and sub-epidermal cells of aerial vegetative organs with no detectable accumulation in trichomes. The accumulation of ANTs in floral organs was restricted to epidermal cells, mainly in a small group of cells on the sepals (Figure 1Q-E) and in petals (Figure 1R, U). ANTs were virtually undetectable in inner floral whorls including carpels and stamens under normal conditions, but could be synthesized in these whorls under stress conditions of low temperature and high light intensity. In leaves ANT mainly accumulate in epidermal cells located on adaxial side (Figure 1V, W), but could be found also on abaxial side under stress conditions of low temperature and high light intensity. Thus, the epidermal cells of petals are the main location where PAs and ANTs are likely to be spatially co-localized.

### Example 3

Flavonoid Levels and Composition Change During Floral Development in White Clover We divided the inflorescences transversely at three selected developmental stages, namely, immature inflorescences, 50% open and mature inflorescences, for quantitative analyses of flavonols, PA, flavan-3-ols and ANT during flower development. This allowed the less developed flowers (upper part of inflorescence) and more developed flowers (lower part of inflorescence) within each inflorescence to be analysed separately (Figure 2A). As a result, flower development was represented by six stages, the youngest being the upper part of immature inflorescences (stage 1) and the most developed being the lower part of mature inflorescences (stage 6) (Figure 2A).

PAs were extracted in butanol-HCl, bound to PVPP and heated to release colored anthocyanidins as degradation products of PAs. This method showed that a very low level of PAs accumulated in leaves, reflecting their presence only in trichomes (Figure 2B). A higher level of PA was detected at flower stages 2 and 3, peaking at stage 4. Analysis of the free 2,3-flavan-3-ol level and composition in inflorescences using LC-MS revealed the presence of only gallocatechin (GC) and epigallocatechin (EGC) monomeric units (Figure 2C). The accumulation of EGC and GC was found to be developmentally regulated in flowers with detectable levels of free monomers at the stage 2 and the highest levels recorded at stage 3. A higher level of GC than EGC was seen at all six stages of flower development.

Analysis of anthocyanins in developing flowers revealed two major molecules, delphinidin-3-sambudioside (A1) and cyanidin-3-sambudioside (A2), the level of A1 being approximately two- to three-fold that of A2 (Figure 2D). Both ANTs showed the highest level of accumulation at stage 3, reflecting ANTs visible in sepals and emerging parts of the petals (Figure 1). Analysis of the level and composition of flavonols revealed 4 main flavonol glycoside species with myricetin (F1, m/z 479), quercetin (F2, m/z 463, F4, m/z 505) and kaempferol (F3, m/z 477) backbones (Figure 2E). The stereochemistry of the sugar unit and the position of the acetate moiety in these molecules was not established. Levels of the four flavonol glycosides increased during flower development, showing the highest level in mature flowers. The myricetin glycosides (F1, m/z 479, R3'=OH, R5'=OH) were predominant in immature inflorescences (stages 1-2), almost equal levels of myricetin and quercetin glycosides (F2, m/z 463 and F4, m/z 505, R3'=OH, R5'=H) were found at flower stage 3, and quercetin glycosides were most abundant at later developmental stages.

### Example 4

### Flavonoid Gene Expression is Developmentally Regulated in White Clover Flowers

We monitored the transcript accumulation patterns of 12,000 *T. repens* genes at the six stages of flower development. Figure 3 (A and B) shows graphical views of the normalized expression data with the expression value of each gene plotted on a log scale against the six developmental stages. All of these profiles passed the significance filter at p≤5 0.05. A total of 2398 genes showed expression differences when at least two of the six developmental stages were compared. The expression profiles for significantly differentially expressed genes across the 6 development stages were clustered using a self organising map. This map had 7 X 8 output nodes and organised genes into 56 clusters of similar gene expression (Figure 3C). We were interested in identifying groups of genes with expression profiles that temporally coincided with patterns of PA accumulation (stages 1-3) or ANT production in epidermal cells of developing white clover flowers (stages 4-6). Eleven clusters (654 genes) showed higher expression at stages 1-3 (dark & light blue) (Figure 3C). 21 clusters (928 genes) showed higher expression at stages 4-6 (red and brown) (Figure 3C). There were no clear differences in expression of the remaining genes between stages 1-3 and 4-6. Lists of genes with expression peaks between stages 1 and 3 (expression profile A), and those with expression peaks between stages 4 and 6 (expression profile B), are shown in Tables 1 and 2. We grouped the genes in terms of seven classes of potential functions, namely, flavonoid enzymes, transcription factors, mediators of protein-protein interactions and protein stability, transporters, mediators of auxin biosynthesis and signal transduction, proteins involved in cell signalling and metabolic enzymes not involved in flavonoid biosynthesis.

### Example 5

### Genes Expressed Between Stages 1 and 3 of Flower Development

Most members of the early and late flavonoid biosynthesis gene (EBG and LBG) families showed expression profile A (see Table 1, online). Seven chalcone synthase (CHS) homologs showed expression profile A. The deduced amino acid sequences of these homologs, apart from *TrCHS1*, contain amino acids required for correct substrate binding, based on the crystal structure of *Medicago sativa* CHS (Jez et al., 2000). Five of these CHS-like genes, *TrCHS1, TrCHS2, TrCHS3, TrCHS4, TrCHS6* and *TrCHS7,* showed expression profiles that peaked sharply at stages 2 and 3. *TrCHS5* showed equally high levels of expression at stages 2, 3 and 4. The expression profiles of two chalcone isomerase (CHI)-like genes, *TrCHI1* and *TrCHI2,* peaked sharply at stage 3 and showed equally high expression levels at stages 2 and 4, respectively. The expression of white clover homologs of flavonoid-3-hydroxylase (*TrF3H1*) and flavonoid-3',5'-hydroxylase (*TrF3*'*5*'*H1*) genes peaked at stage 3 and declined at later developmental stages. *TrCytB5-1,* a homolog of a flower-specific cytochrome b5 gene, which is known to regulate F3'5'H activity and the accumulation of 5'-substituted anthocyanins (de Vetten et al., 1999), showed an expression profile very similar to that of *TrF3'S'H-1.* Interestingly the expression of a second cytochrome b5 gene, (*TrCytB5-*2) showed a broader expression profile, peaking at stages 2, 3 and 4. Two dihydroflavonol 4-reductase-like genes, *TrDFRL1* and *TrDFRL2*, showed expression that peaked between stages 1 and 3 and sharply declined at later stages. Expression of two anthocyanidin synthase-like genes, (*TrANSL1* and *TrANSL2*), was also up-regulated during early stages of flower development, with the highest level at stage 3. Two genes homologous to anthocyanidin reductase (*TrANR*) and leucoanthocyanidin reductase (*TrLAR*), showed developmentally regulated expression profiles with the highest levels of gene expression at stage 3, correlating well with accumulation of the corresponding flavan-3-ols. The expression of *ANR* was higher than that of *LAR* at all stages of flower development. LBGs, most of which encode enzymes involved in the modification of flavonoids, including flavonol 3-O-glucosyltransferases, UDP-glucose glucosyltransferases, O-methyltransferases, anthocyanidin rhamnosyl-transferases and UDP-glucose 4-epimerases, were well represented in profile A. Three genes homologous to an *Arabidopsis* laccase (o-diphenol and para(p)-diphenol:dioxygen oxidoreductase, *TT10*) involved in the oxidative polymerization of flavonoids (Pourcel et al., 2005), were also detected in the profile A group. Two of these genes, (*TrLAC1* and *TrLAC*2) displayed a sharp expression peak at stage 3 and expression of a third laccase-like gene (*TrLAC3*) peaked at stages 1-2.

We found 19 transcription factors in the list of profile A genes. Among them were members of the R2R3-MYB/bHLH/WDR module involved in regulation of flavonoid genes. These included two R2R3 MYB transcription factors, (*TrMYB1*)*,* one MYC factor (*TrMYC1*) and three WDR proteins (*TrWDR1-3*). Genes similar to those encoding other regulatory proteins involved in flavonoid biosynthesis (GLABRA2, TT1, MADS-box, WRKY and GRAS), were also found to be expressed at early stages of white clover flower development. The expression of *TrMYB2, TrWDR1*-*3* and *TrTT1* peaked very early during flower development (stage 1).

Transporters were represented by 16 candidate genes potentially involved in the compartmentalization of flavonoids into the vacuole. These included ABC transporters, a glutathione S-transferase and a vacuolar sorting protein. Most showed an expression peak at stage 3.

### Example 6

### Genes Expressed Between Stages 4-6 of Flower Development

Members of some gene families with representatives up-regulated at stages 1-3 were found to be induced at later stages of flower development (see Table 2). For example, two CHS-like genes (*TrCHS9* and *TrCHS11*), showed a sharp peak at stage 4 and two others, (*TrCHS8* and *TrCHS10*), showed broad expression peaks at stages 3-5. Two F3H candidates, *TrF3H2* and *TrF3H3,* showed distinct expression profiles. Expression of *TrF3H2* was almost the same between stages 4-6 and that of *TrF3H3* peaked at stage 6. Two DFR-like genes, *TrDFRL3* and *TrDFRL4*, showed sharp up-regulation at stage 5. An F3'H homolog (*TrF3'H1*) and an ANS-like gene (*TrANSL3*) had expression peaks at stage 4. Genes encoding anthocyanin 5-aromatic acyltransferase, isoflavone-7-O-methytransferase, methyltransferase, glucosyltransferase, UDP-glucuronosyl/UDP-glucosyltransferase and UTP-glucose glucosyltransferase enzymes conformed to expression profile B. Two isoflavone-7-O-methytransferase genes and two NADPH:isoflavone reductase candidate genes were identified among profile B genes. Some profile B genes potentially encoded transporters involved in vacuolar sequestration of flavonoids including a multidrug resistance-associated protein, a H+-transporting ATPase, ATP-binding cassette (ABC) transporters, a glutathione S-transferase and a vacuolar sorting protein. Interestingly a number of auxin-regulated genes and genes involved in auxin transport were up-regulated at stages 4-6 but not at earlier developmental stages.

White clover homologs of genes encoding components of the MYBR2R3-MYB/bHLH/WDR module, which potentially regulates flavonoid production, were also well represented within the profile B genes. These include six R2R3-MYB candidates (*TrMYB3-6,8*) four MYC/bHLH candidates (*TrMYC2-3, TrbHLH1-2*) and one WDR factor (*TrWDR4*). Genes encoding proteins similar to the YABBY, MADS-box, WRKY and GAI/GRAS classes of transcription factors, potentially involved in flavonoid biosynthesis, were also found to be expressed at late stages of white clover flower development.

Real-time RT-PCR was used to validate the microarray data, with an emphasis on the expression of molecular markers of PA biosynthesis (*TrANR* and *TrLAR*), ANT biosynthesis (ANT 5' aromatic acetylase and UDP-glucosyltransferase), as well as CHS and ANS-like genes, representing EBGs and LBGs. Profile A genes included: *TrANR, TrLAR, TrCHS7, TrCHS6, TrCHS2, TrANSL1* and *TrMYB1.* Profile B genes included: *TrANAT3, TrUFGT4, TrCHS10, TrANSL3, TrMYB8* and *TrMYB5*. In all cases, there was a good correlation between real-time RT-PCR and microarray results (Figure 4).

To test both the spatial and temporal expression patterns of profile A and B genes we separated the sepals, which accumulate a high level of ANT and a low level of PA, from the inner floral whorls, which accumulate a high level of PA and a low level of ANT, sampling flowers at stages 3, 4, 5 and 6. Expression of the PA pathway-specific genes, *TrANR* and *TrLAR*, was highest within the inner whorls, correlating well with histochemical DMACA staining for PA accumulation in flowers (Figure 5 and Figure 1). *TrANSL1* and *TrMYB1* showed a similar expression profile. The four selected *CHS* genes showed a range of spatial expression profiles within the flowers. *TrCHS7* and *TrCHS2* were expressed mainly in inner whorls. Among the selected profile B genes, expression of *TrCHS10* was found to be sepal-enhanced and *TrCHS6* showed an intermediate expression profile, with most expression within the inner whorls, but relatively high expression in sepals. *TrANAT3 and TrUFGT* were found to be expressed specifically in the inner whorls. *TrANS3* and *TrMYB8* were expressed in both inner whorls and sepals.

### Example 7

### Characterization of the White Clover ANR and LAR Genes

The translation product of a 1,014-bp *TrANR* cDNA (338 amino acids) shared 92.4% sequence similarity (88.2% identity) with a functionally characterized ANR from *M. truncatula* and 84% similarity (75.4% identity) with the BANYULS protein of *A. thaliana* (Xie et al., 2003). The position of *TrANR* in a phylogenetic tree of the superfamily of reductase-epimerase-dehydrogenase (RED) proteins is shown in Figure 10. The ANR family is most closely related to DFRs, forming a separate branch in the RED family and sharing a core 315- to 320-amino acid region. ANRs differ from DFRs by having 6-8 extra amino acids at the N-terminus and longer carboxy-terminal regions. Three putative *LAR* genes were identified among white clover expressed sequence tags (EST) sequences on the basis of similarity to the *D. uncinatum* LAR sequence (AJ550154; Tanner et al., 2003). All three have ORFs of 1,071 bp that are predicted to encode proteins of 356 amino acids in length. The *TrLARa* and *TrLARb* ORFs are distinguished by a single nucleotide difference and encode proteins with 99.7% amino acid identity. The *TrLARa* ORF also contains a frameshift, most likely caused by the loss of G418 during cDNA synthesis. The ORFs of *TrLARb* and *TrLARc* are distinguished by 9 nucleotide differences, encoding proteins with 98.9% amino acid identity. The four amino acids that discriminate *TrLARb* from *TrLARc* are not located within conserved motifs previously described (Tanner et al., 2003; Bogs et al., 2005). Since white clover is an allotetraploid species, these sequence variants may represent homeologs or allelic variation. Consequently, *TrLARb* was selected for further analysis and will henceforth be referred to as *TrLAR.* The deduced amino acid sequence of *TrLAR* is similar to LAR proteins from *M. truncatula* (86.5% amino acid identity), *L. corniculatus* LAR2-1 (71.6%) and *Phaseolus coccineus* (71.7%) (Bogs et al., 2005; Pang et al., 2007; Paolocci et al., 2007). *TrLAR* also shares 64.6% amino acid identity with *L. corniculatus* LAR1-1 and 62% identity with *D. uncinatum* LAR. Four motifs conserved in LAR sequences but absent from closely related isoflavone reductases (IFR) were identified in *TrLAR.* Three of these motifs, KRFLPSEFGHD (residues 116-126), ICCNSIA(g/a/s)WPY (residues 160-170), and THDIFI(n/k)GCQ (residues 276-285), are present in functionally active LAR enzymes. A fourth shorter motif, DIGKFT, is located between residues 203-208. Figure 10 shows the relationship between the sequences of *TrLAR* and other enzymes in the reductase-epimerase-dehydrogenase (RED) superfamily (Paolocci et al., 2007).

### Example 8

### Down-Regulation of TrANR Correlates with the Accumulation of ANT in Floral Organs

Transgenic white clover plants ectopically expressing dsRNAi silencing constructs containing 3' end sequences of the *TrANR* (18 plants) and *TrLAR* (10 plants) cDNA sequences, and a fusion between the *TrANR* and *TrLAR* fragments, (9 plants) under control of the 35S RNA promoter from CaMV were generated to elucidate the function of *TrANR* and *TrLAR* in white clover flowers. The presence of transgenes in the To generation of transformed plants was verified by real-time PCR.

No significant phenotypic differences were found between the transgenic and wild-type plants in vegetative organs and sepals sampled at different stages of development. The main differences were seen in petals, carpels and stamens of flowers from stages 2 and 3 of development. The petals of *TrANR* dsRNAi lines displayed three main colour phenotypes, white/light pink, resembling wild-type flowers (lines 6-9B, 6-10A, 6-1F), pink (lines, 6-8A, 6-9C1 and 6-10C) and dark red (lines 6-10B, 6-14D, 6-11A, 6-9B1, 6-4B, 15-2B) (Figure 6A-C, respectively). The strongest level of ANT accumulation in the red flowered lines was observed at flower stages 3 and 4. The colour of the petals was paler at later developmental stages (Figure 6D-E). In contrast to wild-type plants, the uppermost flowers of inflorescences in the red-flowered *TrANR* dsRNAi lines did not fully develop at stage 6 (Figure 1F and Figure 6C). No significant differences were seen between sepals of the wild-type and transgenic lines at any developmental stages (Figure 6E-F). Light microscopy revealed a high level of ANT accumulation in the epidermal cells of petals, carpels, stamens and protoplasts of *TrANR* dsRNAi lines from early stages (2-3) of flower development (Figure 6G-K) in red-flowered lines. No ANT was detected in these organs in wild-type plants at corresponding stages. A mosaic pattern of ANT accumulation in epidermal cells of stamen filaments and carpels in *TrANR* dsRNAi lines correlated with the distribution of PA-producing cells in wild-type plants, as shown in Figure 6L-T. Transgenic *TrANR-TrLAR* dsRNAi plants showed flower phenotypes similar to those of *TrANR* dsRNAi lines. However, no red flower phenotype was seen among the *TrLAR* dsRNAi lines. The inflorescences of these lines resembled those of wild-type plants at all stages of development.

Transcript levels of the *TrANR* gene were measured in 50% open inflorescences (stages 3 and 4) of transgenic dsRNAi and wild-type plants using real-time RT-PCR. A red-flowered phenotype correlated with reduction in the level of *TrANR* expression in *TrANR* dsRNAi lines (Figure 7A, lines 6-10B, 6-14D, 6-11A, 6-4B, 15-2B). Pink-flowered *TrANR* dsRNAi lines (Figure 7A, lines 6-9B1, 6-8A, 6-9C1, 6-10C) showed an intermediate level of *TrANR* expression that was higher than that of red-flowered transgenics, but lower than that of most transgenic lines with white or light pink flowers (lines 6-9B, 6-10A, 6-1F) and the wild-type (Figure 7A).

Four of the six tested *TrLAR* dsRNAi lines showed a reduced level of *TrLAR* expression in comparison to the wild-type and two lines, 11-10A and 11-4C, showed almost a 10-fold reduction in expression. (Figure 7B).

Four of the five tested *TrANR-TrLAR* dsRNAi lines with red-flowered phenotypes (lines 22-2A, 22-4A, 22-1B, 14-2B) were found to have reduced levels of both *TrANR* and *TrLAR* transcripts (Figure 7C). A higher level of both of these genes was found in *TrANR-TrLAR* dsRNAi lines with white/light pink flowers (14-1A, 22-9A, 14-3A), but this level was still significantly lower than that of control plants.

### Example 9

### Down-Regulation of TrANR and TrLAR Correlates with Changed Levels of Flavonoids in White Clover Flowers

Biochemical analysis of flavan-3-ols in 50% open inflorescences (stages 3 and 4) showed a reduction in the level of EGC in 4 out of seven tested *TrANR* dsRNAi lines (6-10B, 6-9.B.1, 6-4B and 15-2B) with red-flowered phenotypes and a reduced level of *TrANR* transcript, in comparison to wild-type plants (Figure 8A). Four tested *TrANR* dsRNAi lines (6-4B, 15-2B, 6-11A and 15-8A) showed a decreased level of GC in comparison to control plants. The 6-4B and 15-2B lines showed a reduced level of both EGC and GC, when compared to control plants. Line 6-11A did not show a reduced level of EGC, relative to control plants, but the level of GC was significantly reduced.

Interestingly, all tested *TrLAR* dsRNAi lines showed lower levels of GCs than wild-type plants. Two out of five analyzed *TrLAR* dsRNAi lines (11-10A and 11-4C) that down-regulated *TrLAR* expression also showed a significantly reduced GC level than those of control plants. All *TrLAR* dsRNAi lines showed dramatically higher levels of EGC in comparison to control plants.

All tested *TrANR-TrLAR* dsRNAi lines with red petals (22-2A, 22-4A, 22-1B,14-2B) that showed strong down-regulation of *TrANR* and *TrLAR* expression also had reduced GC levels, relative to control plants. Two lines, (22-2A and 22-4A) also had reduced EGC levels. GC was virtually absent in these two lines. Conversely, a pink flowered line (14-1A) with a higher level of *TrANR* and *TrLAR* expression did not have significantly reduced levels of EGC and GC in comparison to control plants.

There was a positive correlation between ANT levels in 50% open inflorescences of transgenic plants and the intensity of petal coloration (Figure 8B). All lines with red-flowered phenotypes had higher levels of both delphinidin-3-sambudioside (A1) and cyanidin-3-sambudioside (A2), relative to wild-type plants, with a much higher level of A1 than A2. No significant differences were observed in A1 and A2 levels between white/light pink-flowered transgenic lines and wild-type plants.

The level and relative abundance of four major flavonol glycosides was modified in flowers from some *TrANR* dsRNAi lines, in comparison to those of wild-type plants (Figure 8C). The level of myricetin glycoside (F1, m/z 479), was up to 3-fold higher in red-flowered transgenic *TrANR dsRNAi* and *TrANR-TrLAR* dsRNAi lines than in the wild-type. An intermediate level of myricetin glycoside accumulation was detected in transgenic lines with a pink-flowered phenotype. Production of kaempferol glycoside (F3, m/z 477) was slightly lower in the red-flowered transgenic lines than in transgenic lines with pink and white flowers and wild-type plants. There was no significant correlation between the accumulation of two quercetin glycosides (F2, m/z 463 and F4, m/z 505), ANT and flavan-3-ols in white clover flowers.

### Example 10

### Down-regulation of TrANR Correlates with Global Changes in the Expression of Flavonoid-Related Genes

We compared the transcript accumulation patterns of 12,000 *T. repens* genes in 50% open inflorescences of three red-flowered *TrANR* dsRNAi lines and three wild-type lines using CombiMatrix™ custom oligonucleotide arrays. Only expression profiles that passed the significance filter at p ≤ 0.05 were analyzed (see Tables 3 and 4). Approximately 900 genes were up-regulated and 600 genes were down-regulated in the red flowered *TrANR* dsRNAi lines, relative to wild-type plants (see Figure 11). A large proportion of these genes (approx 400) showed no BLAST hits or matched only hypothetical proteins. Of the annotated genes potentially involved in metabolic pathways, 150 were up-regulated and 123 were down-regulated in the *TrANR* dsRNAi lines relative to wild-type plants. These comprised gene classes encoding putative flavonoid enzymes, transcription factors, transporters, cell signaling proteins, proteins involved in protein-protein interactions or protein stability, mediators of auxin biosynthesis and signal transduction, proteins involved in transcription and translation and enzymes of other metabolic pathways (see Tables 3 and 4).

Twenty eight flavonoid pathway genes were up-regulated in red flowered *TrANR* dsRNAi lines, relative to wild-type clover plants (see Table 3). Most of the genes encoded enzymes involved in modification of ANTs. Two genes involved in the late steps of ANT biosynthesis, flavonoid 3-O-glucosyltransferase and UDP-glucuronosyl/UDP-glucosyltransferase, showed the highest rates of induction, 8.2- and 7.5-fold, respectively in *TrANR* dsRNAi lines. Other ANT-related genes up-regulated in *TrANR* dsRNAi lines included those encoding four glucosyltransferases, two glutathione S-transferases, two *o*-methyltransferases, anthocyanidin rhamnosyl-transferase and anthocyanin 5-aromatic acyltransferase.

Genes encoding eleven flavonoid enzymes, representing both EBG and LBG and functioning upstream of the *TrANR* gene, were also up-regulated in inflorescences of *TrANR* dsRNAi lines, relative to wild-type plants. These genes included three dihydroflavonol-4-reductase homologs (*TrDFRL5, TrDFRL2, TrDFRL3*), seven chalcone synthase homologs (*TrCHS9, TrCHS11, TrCHS5, TrCHS2, TrCHS6, TrCHS10*), an anthocyanidin synthase-like gene (*TrANSL1*), a flavanone 3-hydroxylase homolog (*TrF3H2*) and a chalcone isomerase homolog (*TrCHI2*). Genes encoding two homologs of cytochrome b5 DIF were also up-regulated 1.8- and 1.5-fold. Interestingly, homologs of genes encoding some enzymes of the isoflavonoid pathway, namely isoflavone 3'-hydroxylase, vestitone reductase, NADPH:isoflavone reductase, chalcone reductase, and isoflavone-7-O-methytransferase were also up-regulated in the red-flowered *TrANR* dsRNAi lines, relative to wild-type plants.

Genes encoding 19 transcription factors were up-regulated in inflorescences of red-flowered *TrANR* dsRNAi plants, relative to wild-type plants. Representatives of two components of the R2R3-MYB/bHLH/WDR module, namely *TrWDR5, TrWDR6, TrMYB2, TrMYB6* and *TrMYB3,* showed the highest levels of up-regulation (X5, X1,2, X4.5, X1.7 and X1.4, respectively). Genes encoding representatives of the bHLH and MYC families of transcription factors, *TrMYC3* and *TrbHLH2,* were up-regulated 1.5-and 1.4-fold, respectively, in red-flowered *TrANR* dsRNAi plants. Genes encoding homologs of the circadian clock-associated genes, *CCA1 (TrMYB10)* and *LHY (TrMYB11)* were up-regulated 2.9- and 2.4-fold respectively, in the transgenic lines.

Thirty genes encoding proteins involved in protein-protein interactions and protein stability, 22 genes involved in cell signaling and 13 transporters were expressed at higher levels in red-flowered *TrANR* dsRNAi lines, relative to wild-type plants. Lipid transfer proteins, vesicle-associated membrane proteins and vacuolar sorting proteins involved in intracellular compartmentalization of the flavonoid enzymes and/or their products were strongly represented among genes highly expressed in inflorescences of red-flowered *TrANR* dsRNAi lines.

Approximately 500 genes were down-regulated in the inflorescences of *TrANR* dsRNAi lines, relative to wild-type plants (see Table 4). Approximately 300 of these genes showed no BLAST hits or matched only hypothetical proteins. Ten genes down-regulated in *TrANR* dsRNAi plants encoded flavonoid-related enzymes. As expected, the expression of *TrANR* was much lower in these lines. Surprisingly, two genes involved in isoflavonoid biosynthesis, NADPH:isoflavone reductase (*TrlFR1,* X24) and isoflavone-7-O-methytransferase (*TrlFOMT1,* X3.7) were strongly down-regulated. Homologs of flavonoid 3'-hydroxylase (*TrF3'H1,* X2.4), dihydroflavonol 4-reductase (*TrDFR4,* X1.7), UDP-glucose 6-dehydrogenase (X2.3), anthocyanin 5-aromatic acyltransferase (X3.3), UDP glucuronosyl/UDP glucosyltransferase (X2.9), flavonoid 3-*O*-glucosyltransferase (X1.88), and methyltransferase (X1.5), genes were also down-regulated in *TrANR* dsRNAi lines.

Expression of 18 transcription factors was suppressed in Tr*ANR* dsRNAi plants, among them members of the MYB/bHLH/WDR module, namely *TrWDR7* (x3.5) and *TrMYB12* (x1.37). These genes had not been differentially expressed during the development of white clover flowers.

Real-time RT-PCR was used to validate data from the second microarray experiment. A sample of genes up-regulated or down-regulated in *TrANR* dsRNAi lines relative to wild-type plants was selected, namely, *TrANS1, TrCHS10, TrCHS2, TrCHS6,* and *TrANR* (see Figure 12). The expression profiles of these genes correlated well with the results of the microarray experiment (see Tables 3 and 4).

A comparison of the two microarray data sets revealed that 22% (33 out of 150) of the genes up-regulated in inflorescences of red-flowered *TrANR* dsRNAi lines showed differential expression during flower development in wild-type plants. Genes in this subset corresponding to expression profiles A and B are highlighted in blue and yellow, respectively, in Tables 3 and 4. It is interesting that the highest proportion of these genes (14) are flavonoid-related, including six chalcone synthase homologs (*TrCHS2,* profile A, *TrCHS6,* profile A, *TrCHS5,* profile A; *TrCHS9,* profile B; *TrCHS10,* profile B; *TrCHS11,* profile B), two out of three dihydroflavonol-4-reductase homologs (*TrDFRL2,* profile A; *TrDFRL3,* profile B), one chalcone isomerase homolog (*TrCHI2*, profile A), one flavonoid 3-hydroxylase homolog (*TrF3H2,* profile B), one anthocyanidin synthase homolog (*TrANS1,* profile A), and one cytochrome b5 DIF homolog (*TrCytB5-1,* profile A). Of the 12 homologs of transferases involved in ANT modification and up-regulated in *TrANR* dsRNAi lines, just three showed differential expression during wild-type flower development. These were a UDP-glucuronosyl/UDP-glucosyltransferase homolog, *TrUFGT4* (profile B), a methyltransferase, *TrOMT5* (profile B) and anthocyanidin rhamnosyl-transferase, *TrART1* (profile A).

Six transcription factors up-regulated in *TrANR* dsRNAi lines were differentially expressed during the development of wild-type flowers, including the R2R3 MYB-related genes *TrMYB2* (profile A), *TrMYB3* (profile B) and *TrMYB6* (profile B). Other transcription factors up-regulated in *TrANR* dsRNAi lines and differentially expressed during flower development included *TrMYC2* (profile B), a CONSTANS-like zinc finger protein (profile B) and a SQUAMOSA promoter-binding protein (profile A).

Six out of 8 of the genes encoding flavonoid pathway enzymes that were down-regulated in *TrANR* dsRNAi lines showed differential expression between at least two flower stages. The remaining two were candidate anthocyanin 5-aromatic acyltransferase (*TrANAT3*) and UDP-glucose glucosyltransferase (*TrUFGT6*) genes. The transcription factors *TrWDR7* and *TrMYB12,* which were down-regulated in *TrANR* dsRNAi plants, did not show differential expression during flower development.

### Example 11

### Distinct Representatives of Flavonoid-Related Multigene Families Contribute to Spatio-Temporal Profiles of ANT and PA Accumulation in Floral Organs

The developmentally-regulated anthocyanin and proanthocyanidin pathways were found to be spatially co-localized in epidermal cells of white clover flower petals. Accumulation of 2,3-flavan-3-ol monomers and PA started in immature inflorescences and peaked at stages 3 and 4, respectively. ANT accumulation began in epidermal cells of petals when they emerged from the sepals and were exposed to light (stages 3-6, Figure 2A, D). The onset of light-induced pigmentation coincided with declining levels of 2,3-flavan-3-ols and *TrANR* and *TrLAR* transcripts (Figure 2C and Figure 4). This suggests that the activities of the PA and ANT pathways are separated temporally, with the PA pathway active at stages 1-3 and the accumulation of ANT occurring in the same cells at later stages of floral development. The activities of the two pathways appear to overlap at stage 3. This raises questions about the molecular organization of these pathways and their potential cross-talk in epidermal cells.

PAs and ANTs are produced by two related but distinct branches of the flavonoid pathway. Both branches involve the conversion of 4-coumaroyl CoA and malonyl CoA to flavan-3,4-diol and 3-OH-anthocyanidin molecules. Activation of both pathways requires the recruitment of R2R3-MYB, WDR and bHLH transcription factors for the transcriptional activation of early and late flavonoid biosynthesis genes. Molecular studies in a range of plant species have revealed that almost all of the flavonoid enzymes are encoded by members of multigene families. The ANT and PA pathways in white clover flowers may recruit exactly the same enzymes or distinct isoforms of enzymes encoded by different members of multigene families, for shared steps in flavonoid production.

The expression of homologues of PA pathway-specific genes, including *TrANR*, *TrLAR, TrTT1* and *TrTT10,* showed strict profile A expression, peaking during flower stages 1-3 and declining at later developmental stages. This correlated well with the production of 2,3-flavan-3-ol monomers in the inner floral whorls. Homologs of ANT pathway-specific genes involved in conversion of 3-OH-anthocyanidin molecules to anthocyanins were found in both profiles, correlating with ANT production in sepals at all stages of development and the increase in ANT biosynthesis in inner floral whorls at stage 3 (Figure 2C-D). Representatives of multigene families encoding CHS, DFR, ANS and F3H enzymes, MYB, bHLH and WDR transcription factors and transporters were also found in both expression profiles. Representatives of some of the genes shared by both the PA and ANT biosynthesis pathways, such as *CHI* (profile A), *F3'H* (profile B) and F3'5'H (profile A) were found only in one profile, although expression of these genes was detectable at both early and late stages of flower development. For example, expression of the single *F3'5'H* candidate gene peaked at stages 2 and 3 but remained high through stages 4 and 5. This correlated well with expression of homologs of the flower-specific cytochrome b5 gene, which regulates F3'5'H activity, and with the main increase in production of 5'-hydroxylated flavan-3-ols throughout flower development (Figure 2C).

ANS represents a branch point between the PA and ANT pathways converting flavan-3,4-diols to 3-OH-anthocyanidins, potential substrates for both pathways. The ANT pathway modifies 3-OH-anthocyanidins by a chain of glycosylation and esterification reactions and the PA pathway involves the reduction of 3-OH-anthocyanidins to 2,3-cis-flavan-3-ols by ANR. Three ANS-like proteins from white clover, TrANS1, TrANS2 and TrANS3, show 94.4%, 94.4%, and 70% deduced amino acid sequence identity to M. *truncatula* ANS, respectively. Multiple sequence alignment confirmed the presence of three conserved residues (His-232, His-288, and Asp-234) required to coordinate ferrous iron at the catalytic center of iron-containing soluble oxygenases, and Arg-298, Y-217 and S-300, which are assumed to contribute to the specific binding of 2-oxoglutarate in the TrANS proteins. However, only TrANS1 contains the DHQ1-, DHQ2-and MES/ascorbate-binding domains specific to ANS enzymes, but not other 2-oxoglutarate iron-dependent oxygenases, including flavanone 3-□-hydroxylases (F3H) and flavonol synthases (FLS). TrANS2 and TrANS3 share a low level of amino acid identity with Arabidopsis FLS (41.9% and 35%, respectively) and F3H (31.1% and 26.5%, respectively). The *TrANS2* and *TrANS3* genes showed distinct profile A and profile B-specific expression, respectively, whilst *TrANS1* expression peaked at stage 3, but remained relatively high during stages 4 and 5.

R2R3-MYB, bHLH and WDR transcription factors have redundant functions in plant development. The Arabidopsis representatives of these families (TT2, TT8 and TTG1) are involved in PA, ANT and mucilage biosynthesis, root-hair patterning and trichome development. Six candidate genes encoding white clover MYB factors were closely related to R2R3-MYB proteins identified in other plant species. The R2R3 repeat region of white clover MYBs is highly conserved and contains the motif [D/E]Lx2[R/K]x3Lx6Lx3R for interaction with bHLH proteins, whereas the C-terminal regions show a low level of similarity to other MYB factors. *TrMYB3* (profile B) is closely related to the Arabidopsis subgroup 10 MYBs and clustered with other R2R3-MYB gene products involved in anthocyanin biosynthesis, including PAP1, PAP2, PhAN2, LeANT1, VvMYBA2 and VvMYBA1. The deduced amino acid sequence of TrMYB6 (profile A) clustered with MIXTA and PhMYB1, sharing 89% amino acid sequence similarity in the R2R3 DNA-binding domain. Representatives of the bHLH, WDR, MADS box and WRKY box gene families were also found in both expression profiles. Both bHLH and WDR factors are components of the R2R3-MYB/bHLH/WDR transcription factor complex that regulates enzymes in both the PA and ANT pathways in different plants. Arabidopsis TTG2, a WRKY box factor, regulates at least three separate morphogenetic processes in L1-derived cells: trichome development and the production of mucilage and condensed tannin in seed coats. *BANYULS* promoter activity is not affected in *ttg2* mutants and both TTG2 and TT1, a zinc finger protein, may be involved in post-transcriptional regulation of *BANYULS* expression. Therefore, the white clover *TTG2* homolog could potentially regulate *TrANR* expression in trichomes and epidermal cells. The Arabidopsis MADS-box factor TT16/ABS is known to be expressed in the ovule, mediating *BANYULS* expression and PA accumulation in the endothelium of seed coats. One of the white clover MADS box factors up-regulated early in flower development might similarly control PA production by transcriptionally activating the *TrANR* gene.

The PA and ANT pathways are localized within different groups of epidermal cells in sepals: a low level of PA accumulates in trichomes and a high level of ANT is present in a subset of epidermal cells at stages 1-6 (Figure 1). The fact that some of the selected flavonoid genes, including molecular markers of the PA (*TrANR* and *TrLAR*) and ANT (*ANT acetylase, UFGT*) pathways, early (*CHS*) and late (ANS-like) flavonoid biosynthetic genes as well as R2R3-MYB transcription factors, displayed organ-specific expression profiles (Figure 5) suggests that the PA and ANT pathways in sepals and petals recruit (at least partially) different flavonoid-specific members of multigene families. Taken together, the data suggest that spatio-temporal patterns of ANT and PA accumulation in floral organs reflect developmentally-regulated and organ-specific expression profiles of distinct isoforms of flavonoid-related enzymes encoded by multigene families.

### Example 12

### Roles of the White Clover ANR and LAR Genes in PA Biosynthesis

According to the most recent models, *ANR* and *LAR* participate in two separate branches of the PA pathway in most PA-producing species. LAR functions downstream of DFR, catalyzing the conversion of 2,3-flavan-3,4-diols to 2,3-*trans*-flavan-3-ols. ANR acts immediately downstream of ANS catalyzing the conversion 3-OH-anthocyanidins to 2,3-*cis*-flavan-3-ols. Expression of both genes has been found to be developmentally regulated in PA-accumulating tissues of different species. In grapes, *VvANR* and *VvLAR1* are up-regulated at early stages of berry development, 7 weeks before veraison, which correlates well with accumulation of the corresponding flavan-3-ols and PA. The expression of *MdLAR1* and *MdANR* was also shown to be highest during early development of apple (*Malus x domestica Borkh.* cv. 'Cripps Red') fruit. Furthermore, transcript levels of both the *ANR* and *LAR* genes are higher in immature leaves than in mature leaves of *L. corniculatus,* correlating well with increased accumulation of PAs at early stages of leaf development. The spatio-temporal expression patterns of the *TrANR* and *TrLAR* genes in white clover flowers also correlate with the pattern of *cis-* and trans-flavan-3-ol accumulation. Interestingly, a higher level of *TrANR* than *TrLAR* expression correlates with higher levels of GC than EGC monomers at all tested stages of flower development. Despite the higher level of GC we observed in comparison to EGC, prodelphinidin polymers in *T. repens* flowers consist of terminal and extender units with nearly equal proportions of the two epimers. A higher expression level of *ANR,* relative to *LAR,* has also been seen in *L. corniculatus* herbage and in the skin of red apples. A higher level of *VvANR* expression than *VvLAR1* and *VvLAR2* expression in grape flowers was found to correlate with a higher level of catechins than epicatechins. The expression of *VvANR* correlates with a high level of flavan-3-ols and extension subunits with 2,3-*cis*- stereochemistry only in grape leaves, where *VvLAR1* is not expressed and *VvLAR2* is only expressed late in development. Interestingly, in spite of the high level of catechin monomers, most grape tissues accumulate significant levels of epicatechin-based PAs.

### Example 13

### TrLAR Gene Activity is Necessary but Insufficient for 2,3-trans-flavan-3-ol Production in White Clover Flowers

Although the role of the ANR gene in biosynthesis of the 2,3-*cis*-flavan-3-ols has been clearly demonstrated using molecular, genetic and biochemical approaches, the contribution of the *LAR* gene to PA biosynthesis is still unclear, mainly due to the lack of genetic studies. Most functional information is based on the *in vitro* activity of recombinant LAR proteins, ectopic expression of LAR genes in tobacco and white clover and the expression profiles of *LAR* and *ANR* genes in PA-accumulating tissues. *LAR* and *ANR* genes have been found to be co-expressed in tissues producing PA, namely *L. corniculatus* leaves, apple fruit, grape berries, seed coats of *M. truncatula* and white clover flowers (this study). The expression of *ANR* and *LAR* genes is coordinately regulated by the same family of transcription factors in grape berries and *L. corniculatus* tissues. The absence of *LAR* genes in plants producing only 2,3-*cis-*flavan-3-ols, such as Arabidopsis may suggest that *LAR* genes are involved in the biosynthesis of 2,3-*trans*-flavan-3-ols.

On the other hand, a relatively high level of *MtLAR* expression contrasted with the virtual absence of 2,3-*trans*-flavan-3-ol subunits in PA from *M. truncatula* plants. The ectopic expression of *LAR* genes from *M. truncatula* and D. *uncinatum* in tobacco and white clover did not increase levels of *trans*-flavan-3-ols in leaves or flowers. The PA level was actually lower in transgenic tobacco lines than in control plants. Alternative or multiple functions of the *LAR* gene have been suggested. The *LAR* gene is encoded by multigene families in some PA-producing species, including grape and *L. corniculatus.* Only one of the *L. corniculatus LAR* genes showed *in vitro* activity in *E*. *coli.*

Transgenic approaches aiming to characterize the function of *LAR* genes have not been successful. Gain-of-function experiments failed to show increased levels of *2,3-trans-*flavan-3-ol subunits in transgenic tobacco and white clover lines ectopically expressing *LAR* genes. The function of *LAR* genes has not been successfully characterized by loss-of-function approaches in the model plants, *A. thaliana* and *M. truncatula,* which have PA that lacks, or contains virtually no *trans*-flavan-3-ol monomers. Floral PAs and free flavan-3-ols in *T. repens* contain both epigallocatechins and gallocatechins. This feature and the high genetic transformation efficiency of *T. repens* make it an attractive system for the functional analysis of PA-related genes, including *ANR* and *LAR*. Phylogenetic analysis showed that *TrLAR* is most similar to LAR proteins from *M. truncatula* and *P. coccineus,* species which, like white clover, lack appreciable PA biosynthesis in leaf, stem and root tissues. When compared to *Lotus corniculatus* LAR proteins, the amino acid sequence of TrLAR is more similar to LcLAR2 than to LcLAR1. It is interesting that LcLAR2 did not show specific LAR activity when expressed in *E*. *coli.* The spatio-temporal profile of white clover *LAR* expression correlates well with accumulation of GC in PA-producing organs. Down-regulation of the *TrLAR* gene in *TrLAR* dsRNAi and *TrANR-TrLAR* dsRNAi lines significantly decreased the level of *TrLAR* transcripts and correspondingly, the GC level in white clover flowers. The much more pronounced reduction in GC level, compared to the *TrLAR* transcript level in some *TrLAR* dsRNAi lines (11-15A, 10-12A and 11-8A) suggests that expression of another *TrLAR* gene(s) could be affected in *TrLAR* dsRNAi lines. Our phenotypic, molecular and biochemical data suggest that *LAR* activity is necessary for GC biosynthesis in white clover flowers. However, the fact that ectopic expression of *LAR* genes in tobacco and white clover plants resulted in no changes in GC production suggests that LAR activity alone is not sufficient for the biosynthesis of 2,3-*trans*-flavan-3-ols.

Down-regulation of the *TrLAR* gene leads to a dramatic increase in the level of EGC in *TrLAR* dsRNAi lines. However, there were no significant changes in the levels of the two main anthocyanins in the petals. This suggests that the pool of intermediate 3-OH-anthocyanidin molecules appeared to be diverted towards 2,3-*cis*-flavan-3-ol rather than anthocyanin production when *TrLAR* was down-regulated, in contrast to the down-regulation of *TrANR* in white clover plants.

### Example 14

### TrANR Gene Activity is Necessary and Sufficient for 2,3-cis-flavan-3-ol Production in White Clover Flowers

Loss of ANR function in the Arabidopsis *banyuls* mutant results in a *transparent testa* phenotype with a decreased level of 2,3-*cis*-flavan-3-ols and accumulation of anthocyanin in the seed coat. Combined with the finding that expression of recombinant MtANR protein converts cyanidin, delphinidin and pelargonidin molecules into epicatechines, epigallocatechin and epiafzelechin, respectively, suggests that ANR activity is necessary and sufficient for the production of 2,3-*cis*-flavan-3-ols. As in the *banyuls* mutant, down-regulation of *TrANR* reduced the level of *ANR* transcripts and EGC molecules and increased the level of ANT in PA producing cells of white clover. An intriguing finding was that down-regulation of the *TrANR* gene correlated with reduced levels of both EGC and GC in *TrANR* dsRNAi and *TrANR-TrLAR* dsRNAi lines. Interestingly, the levels of *TrLAR* transcripts were twice as high in *TrANR-TrLAR* dsRNAi lines 22-1 B and 14-2B as in *TrLAR* dsRNAi lines 11-10A and 11-4C, but GC levels were lower in the *TrANR-TrLAR* dsRNAi lines. GC was virtually undetectable in the 22-2A and 22-4A *TrANR-TrLAR* dsRNAi lines, suggesting that the effect of silencing the *TrANR* and *TrLAR* genes on 2,3-*trans*-flavan-3-ol production was additive. A reduced level of GC in *TrANR* dsRNAi and *TrANR-TrLAR* dsRNAi lines might be explained by ANR having an additional direct or indirect role in the biosynthesis of 2,3-*trans*-flavan-3-ols. Interestingly, the *trans* (*ent*) epimers of catechin, gallocatechin and afzelechin were detected as minor products after incubation of recombinant MtANR protein with cyanidin, delphinidin and pelargonidin molecules, respectively. This finding was explained as an artifact caused by epimerization of the thermodynamically less stable 2,3-cis diastereoisomers into more stable *2,3-trans-* (*ent*) forms. Further experiments are needed to clarify whether this reaction occurs naturally in wild-type plants or is triggered only by an artificially high level of pathway intermediates.

### Example 15

### Cross-Talk within the Flavonoid Pathway

The ANT and PA pathways share dihydroflavonols as precursor molecules. Three classes of these molecules, differing only in the extent of B-ring hydroxylation, have been identified in legumes. Modification of dihydrokaempferols (R3'=H, R5'=H), dihydroquercetins (R3'=OH, R5'=H), and dihydromyricetins (R3'=OH, R5'=OH) by DFR, ANS and a range of anthocyanidin-modifiying enzymes leads to the biosynthesis of ANTs with pelargonidin (R3'=H, R5'=H), cyanidin (R3'=OH, R5'=H) and delphinidin (R3'=OH, R5'=OH) backbones, respectively. Alternatively dihydroflavonols can be converted to *cis* and *trans* epimeric forms of afzelechins (R3'=H, R5'=H), catechins (R3'=OH, R5'=H) and gallocatechins (R3'=OH, R5'=OH) by DFR, LAR, ANS and ANR enzymes in the PA pathway. Glycosylated forms of three flavonols, representing all three B-ring hydroxylated variants of dihydroflavonols, were found in white clover flowers with an abundance of myricetin glycosides (F1, m/z 479, R3'=OH, R5'=OH) at stages 1-2 and an increased level of quercetin glycosides (F2, m/z 463 and F4, m/z 505, R3'=OH, R5'=H) at later developmental stages (3-6). ANT composition at all these stages showed the predominance of delphinidin-based ANTs and a much lower level of cyanidin-based ANTs. A low level of kaempferol-based ANTs (F3, m/z 477, R3'=H, R5'=H) and virtually no pelargonidin-based ANTs were found at all developmental stages. Analysis of 2,3-flavan-3-ols detected only gallocatechins and epigallocatechins at all developmental stages, with a higher level of gallocatechins.

Down-regulation of *TrANR* led to a decrease in the level of epigallocatechin and an increase in the level of products of the flavonol and anthocyanin pathways with hydroxylation of the B-ring at the 3' and 5' positions (Figure 9). The level of myricetin glycosides and delphinidin-3-sambudioside was enhanced up to 3 and 5-7 fold, respectively, in red-flowered transgenic *TrANR* dsRNAi and *TrANR-TrLAR* dsRNAi lines. Enhanced accumulation of delphinidin-based ANTs could be explained by diversion of intermediates, such as delphinidins, from 2,3-flavan-3-ol to ANT production. The finding that ANT accumulation was clearly enhanced in epidermal cells of the inner whorls of immature flowers, which normally produce only PA (Figure 6K), supports a metabolic diversion model. Light-induced up-regulation of ANT biosynthesis in carpels and stamens at stages 2 and 3, when they were covered by petals and sepals, is very unlikely to have occurred. The finding that an increased level of ANT in red-flowered *TrANR* dsRNAi and *TrANR-TrLAR* dsRNAi lines is developmentally regulated, showing the most intense coloration at stage 3 and fading later (Figure 6D,E), suggests that a temporary excess of intermediate molecules at stages 1-3, due to down-regulation of the *TrANR* gene, may trigger this metabolic change.

Enhanced expression of genes encoding potential glucosyltransferases, UDP-glucuronosyl/UDP-glucosyltransferases, glutathione transferases, methyltransferases and anthocyanidin rhamnosyl-transferases functioning downstream of ANS in *TrANR* dsRNAi lines suggests that their transcriptional regulation was triggered by a reduced level of the *TrANR* transcript and/or an excess of unused metabolic intermediates. Of these genes, *TrUFGT4* and *TrGT12* showed the highest levels of up-regulation in red-flowered *TrANR* dsRNAi lines (8.2- and 7.5-fold, respectively). The expression of some of these genes was not detected or not shown to vary significantly between the six developmental stages of wild-type flowers studied in the first microarray experiment (Figure 9). This subset of genes included *TrGST5, TrGST6, TrGT11, TrGT12, TrGT13, TrGT14, TrUFGT6, TrOMT6, TrANAT4* and *TrAAT1.*

Down-regulation of *TrANR* led to a 3 fold increase in the level of myricetin glycosides produced by the flavonol pathway, which branches from the PA and ANT pathways up-stream of ANR. It is difficult to explain these changes simply by metabolic spillover or diversion of delphinidin intermediates. Moreover, changes in the expression levels of most EBG, LBG and genes encoding transcription factors in *TrANR* dsRNAi lines, in comparison to wild-type plants, suggest that re-programming of the whole flavonoid pathway had occurred. Ectopic expression of one R2-R3 MYB transcription factor, PAP1, in Arabidopsis resulted in an elevated level of cyanidin-type ANTs and quercetin type flavonols as well as the up-regulation of almost all genes encoding ANT biosynthetic enzymes. Down-regulation of a single gene encoding a metabolic enzyme, TrANR, also led to dramatic changes in the levels of flavonoids and changes in the expression of almost all the genes encoding enzymes known to be involved in ANT and PA biosynthesis in white clover (Figure 9). Genes up-regulated in *TrANR* dsRNAi lines included representatives of genes functioning upstream of Tr*ANR* in the general flavonoid pathway or in another branch, such as isoflavone biosynthesis, namely *TrCHS, TrCHI, TrF3H2, TrF3'H1, TrDFRL, TrANS, TrCHR, TrIF3'H, TrIFOMT* and *TrVR* candidate genes. However, not all of these changes in gene expression were accompanied by changes in the levels of flavonoid products. Although down-regulation of a *TrF3'H* gene correlated with an increase in the level of delphinidin-3-sambudioside, this was not accompanied by increased expression of *TrF3'5'H,* suggesting that the level of this transcript does not limit flux in the pathway producing delphinidin-based ANTs.

Some transcription factors were also up-regulated in Tr*ANR* dsRNAi lines, providing further support for the metabolic re-programming model. Six *MYB* genes, two *MYC* or *bHLH* genes and three *WDR* genes were up-regulated in flowers of *TrANR* dsRNAi lines in comparison to wild-type plants. *TrWDR5, TrWDR6, TrMYB10 TrMYB11, TrMYB9* and *TrbHLH3* were up-regulated in red flowered *TrANR* dsRNAi lines. *TrMYB12* and Tr*WDR7* were down-regulated in these transgenic lines.

Another interesting outcome of down-regulating the *TrANR* gene was the differential expression of members of the same gene family. Among *DFR*-like genes, expression of *TrDFRL1* (profile A) did not change, expression of *TrDFRL2* (profile A), *TrDFRL3* (profile B) and *TrDFRL5* (neither profile) was up-regulated, and expression of *TrDFR4* (profile B) was down-regulated in Tr*ANR* dsRNAi lines. Only one member of each of the white clover *F3H* and *ANS* gene families was up-regulated in *TrANR* dsRNAi lines. Differential expression was also detected among members of the white clover *OMT, GT* and *ANAT* gene families. Two representatives of the *TrIFOMT* gene showed contrasting expression patterns: *TrIFOMT1* was down-regulated and *TrIFOMT2* was up-regulated in *TrANR* dsRNAi lines, relative to wild-type plants.

A possible mechanism for the re-programming of the flavonoid pathway in *TrANR* dsRNAi lines involves changes in gene expression in response to the accumulation of intermediate molecules, such as 2,3-flavan-3,4-diols and 3-OH-anthocyanidins. Flavonoids have been implicated in direct and indirect interactions with transcription/translation machinery, trafficking, anion channels, mediators of cell signaling and cell-to-cell communication. Flavonoids are involved in polar auxin transport, responses to wounding and pathogens, interactions between plants or between plants and animals, embryonic development and seed germination. Loss of *ANS*/*TT18*/*TDS4 (TANNIN DEFICIENT SEED 4*) function in *A. thaliana* resulted in the appearance of multiple small vacuoles, suggesting that PA or intermediate accumulation is a signal for vacuolar maturation. The molecular targets of flavonoids include transcription factors, kinases, ABC transporters, hydrolases, peptidases, tyrosine phosphatases and serine/threonine kinases. Some of these proteins are transcriptionally up-regulated by a R2-R3-MYB/bHLH/WDR transcription factor complex in other species. Hence, it is interesting that candidate *MYB, bHLH* and *WDR* genes showed enhanced expression in *TrANR* dsRNAi lines. A number of metabolism-related transcription factors (MTFs) have been recently described. MTFs are metabolic enzymes or their homologs that use NAD, FAD and CoA as cofactors and directly link metabolism with gene regulation binding directly to DNA, or regulating gene expression by interacting with other transcription factors. Both ANR and LAR proteins require NADPH/NADH cofactors for their activities. Nuclear localization is a key requirement for regulation of transcription. Transient expression of the 35S::*MtANR* in tobacco and 35S::*TrANR* in Arabidopsis leaf epidermal cells demonstrated cytosolic localization. Moreover, both ANR and LAR proteins lack the known nuclear localization signals and domains involved in protein-protein interaction. Subcellular localization of these proteins in PA-producing cells could provide crucial information about their potential function as MTFs.

### Example 16

### Metabolic Channeling

The metabolic channeling model suggests that sequential enzymes in a metabolic pathway are organised into macromolecular complexes. The movement of intermediates directly between enzymes within these structures increases catalytic efficiency by limiting their diffusion and interaction with other cell components. The channeling model also allows the possibility of combinatorial regulation, resulting in a variety of enzyme complexes producing related but distinct metabolites. The spatial and temporal profiles of PA and ANT biosynthesis in white clover epidermal cells suggests two possible channeling models: (i) the existence of independent channels producing PA and ANT; and (ii) the existence of a single core channel branching at the ANS point allowing production of PA and ANT pathways potentially competing for the anthocyanidin substrate. Spatio-temporal expression profiles of flavonoid-related genes suggest that the first model may be valid in white clover flowers. In support of this model, different representatives of the *CHS, DFR, ANS, F3H, R2R3-MYB, bHLH* and *WDR* and transporter gene families were identified in both expression profiles. In support of the second model, only one likely *ANS* homolog has been found in *Medicago* and white clover. Single homologs of the *F3'H, CHI* and *F3'5'H* genes may be also shared between the ANT and PA biosynthetic pathways. The first model suggests that the ANT and PA channels may be spatially and temporally separated. Modification of the one of the metabolic channels in this case may not necessarily affect the other pathway. The second model could function when ANT and PA channels are located in the same spatial vicinity. In this case, modification of one of the metabolic channels would re-direct the flow of intermediate molecules, resulting in quantitative changes in the final products. Results from both white clover and Arabidopsis lines lacking functional ANR provide evidence for the second model by showing that down-regulation of the *ANR* gene leads to enhanced ANT accumulation in tissues that normally produce PA (Figure 6). Furthermore, a reduced level of ANT in tobacco petals ectopically expressing *ANR* genes suggests a flexible mechanism(s) of flux diversion at the branch-point between the ANT and PA pathways, with competition between ANR and anthocyanidin glucosyltransferase enzymes for the anthocyanidin substrate.

In summary, we present experimental data correlating spatio-temporal patterns of ANT and PA biosynthesis with differential expression patterns of flavonoid-related genes in developing white clover flowers. Our findings support a model where the ANT and PA pathways are spatially co-localized within epidermal cells of petals, temporally overlap at stages 2-4 and recruit distinct isoforms of flavonoid-related enzymes encoded by multigene families. Altered levels of flavonoid pathway products and changes in the expression of many flavonoid-related genes provide evidence for metabolic re-programming in *TrANR* dsRNAi lines and the possibility of cross-talk between metabolic channels producing PAs, ANTs and flavonol glycosides. We also present the first *in vivo* genetic evidence that a plant LAR protein is required for the biosynthesis of 2,3-*trans*-flavan-3-ols. Our findings support the idea of a role for the ANR enzyme in the biosynthesis of 2,3-*trans*-flavan-3-ols, in addition to its known function in the reduction of anthocyanidins to 2,3-*cis*-flavan-3-ols. Our work will facilitate genetic modification of the flavonoid pathway to increase PA levels in herbage for enhancing bloat safety in key forage legumes, such as alfalfa and white clover.

### Tables

**Table 1**

| Probe number | Name | Annotation | UniRef90 | Score | E-value | p.val | Stage 1 | Stage 2 | Stage 3 | Stage 4 | Stage 5 | Stage 6 |
|---|---|---|---|---|---|---|---|---|---|---|---|---|
| **Flavonoid pathway enzymes** | | | | | | | | | | | | |
| 3577 | *TrCHS1* | Chalcone synthase | O04111 | 249 | 1.60E-19 | 0.00000000000 | 3.355169905 | 5.03411559 | 3.7330603 | -4.3607844 | -4.798735499 | -4.803917483 |
| 4173 | *TrDFRL1* | Dihydro-flavanol reductase | Q1SP66 | 792 | 2.90E-77 | 0.00000000000 | 2.487777119 | 3.044301198 | 1.5950363 | -4.7495303 | -4.89267673 | -5.223023485 |
| 3588 | *TrDFRL2* | Dihydroflavonol-4-reductase | Q6TQT0 | 1432 | 4.60E-145 | 0.00000000003 | 0.741677048 | 2.037415225 | 3.1525918 | 2.6746614 | 0.881878388 | -2.801387091 |
| 9064 | *TrF3'5'H1* | Flavonoid 3',5'-hdyroxylase | Q2PF26 | 563 | 5.40E-53 | 0.00000000034 | 1.975027665 | 3.299829885 | 4.1313213 | 3.5980586 | 2.394774897 | -0.320587888 |
| 2585 | *TrANR* | Anthocyanidin reductase | Q84XT1 | 1143 | 1.80E-114 | 0.00000000180 | 2.654476017 | 4.361090909 | 5.1703438 | 4.4630822 | 2.978170803 | 0.510461064 |
| 10563 | *TrCHS2* | Chalcone synthase | P17957 | 1710 | 1.60E-174 | 0.00000000192 | -1.837951355 | 0.366152346 | 0.9953488 | 0.592085 | -0.87568155 | -1.870876835 |
| 2320 | *TrANSL1* | Anthocyanidin synthase | Q2TUV8 | 1343 | 1.20E-135 | 0.00000001710 | -1.703117068 | 0.193240414 | 1.2605585 | 0.253017 | -1.99434523 | -4.889406581 |
| 7371 | *TrLAR* | Leucoanthocyanidin reductase | AJ550154.3 | 640 | 1.00E-180 | 0.00000036100 | -1.313001132 | 0.974402948 | 2.2180532 | 0.5963576 | -1.61451624 | -4.309620288 |
| 1112 | *TrF3H1* | Flavanoid 3-hydroxylase | O04112 | 1591 | 5.80E-162 | 0.00000364385 | 2.422201036 | 3.392642394 | 4.333794 | 4.1982455 | 3.767242237 | 2.610168422 |
| 1210 | *TrCHI1* | Chalcone isomerase | Q8H0G1 | 926 | 1.90E-91 | 0.00000499000 | 3.042360975 | 3.495062453 | 3.9582301 | 3.361407 | 1.816263548 | 0.665422814 |
| 9320 | *TrLac1* | Laccase | Q8RYM9 | 285 | 7.90E-23 | 0.00000770000 | -1.685349967 | -0.436841078 | 1.2795463 | 0.6975313 | -0.007464909 | -0.868883482 |
| 10566 | *TrCHS3* | Chalcone synthase | P17957 | 715 | 4.20E-69 | 0.00001345686 | -1.183055121 | 0.529839512 | 2.3049658 | 1.9125735 | 0.964177288 | -0.275261814 |
| 3311 | *TrCytB5-1* | Cytochrome b5 DIF-F | Q9M5B0 | 417 | 1.50E-37 | 0.00001610000 | 2.716065602 | 4.187998789 | 4.328347 | 3.7906774 | 2.376137296 | 1.357477372 |
| 10567 | *TrCHS7* | Chalcone synthase | P51086 | 960 | 4.60E-95 | 0.00002326741 | -2.309915463 | -1.183785501 | 0.7548042 | 0.6169887 | 0.076903988 | -1.73316044 |
| 8348 | *TrLac1* | Laccase | Q53NW2 | 680 | 2.30E-65 | 0.00002480000 | -1.836825183 | -0.993164642 | -0.4881446 | -1.9351985 | -1.701440114 | -1.415979512 |
| 9735 | *TrGT5* | Flavonoid 3-O-glucosyltransferase related | O04114 | 380 | 1.30E-33 | 0.00003983068 | 2.727638349 | 2.810213897 | 3.4165825 | 3.1106163 | 1.985179277 | 0.632431935 |
| 10562 | *TrCHS4* | Chalcone synthase 6-4 | P51079 | 151 | 4.80E-09 | 0.00016566500 | 3.331165198 | 3.796057225 | 4.7120491 | 4.0454517 | 2.801725613 | 2.229079517 |
| 10571 | *TrCHS5* | Chalcone synthase | P17957 | 895 | 3.60E-88 | 0.00019590700 | -1.632622874 | 1.172349219 | 1.2410926 | 1.0397242 | -1.290256955 | -2.38741553 |
| 8033 | *TrGST1* | Glutathione S-transferase GST 7 | Q9FQF1 | 690 | 1.80E-66 | 0.00024253893 | 3.775187526 | 3.698402516 | 4.4517278 | 3.8212356 | 3.075289539 | 1.571296587 |
| 8940 | *TrGST2* | Putative glutathione S-transferase | Q8H8E0 | 128 | 8.70E-07 | 0.00064167700 | 3.106375653 | 2.787413155 | 3.1043808 | 1.9585072 | 1.30350352 | 1.16629159 |
| 4237 | *TrGT1* | Putative flavonol 3-O-glucosyltransferase | O81010 | 345 | 6.40E-30 | 0.00070456700 | 1.388343164 | 1.808359985 | 1.9800678 | 0.7791704 | 0.948487668 | 1.530733693 |
| 10564 | *TrCHS6* | Chalcone synthase | P30081 | 1668 | 4.40E-170 | 0.00080783200 | 0.903128281 | 2.002306483 | 2.6737985 | 2.1467123 | 0.628968613 | -0.29831704 |
| 1763 | *TrUFGT1* | UDP-glucose glucosyltransferase | Q9LVR1 | 431 | 5.40E-39 | 0.00105015300 | 3.170964363 | 2.576500022 | 2.5255572 | 1.1404227 | 1.578963547 | 0.91616233 |
| 4847 | *TROMT1* | O-methyltrarisferase, family 2 | Q2HTB5 | 1411 | 7.50E-143 | 0.00107496900 | 2.40287562 | 2.886527785 | 2.4407377 | 1.666127 | 1.368773046 | 1.52621636 |
| 984 | *TrCytB5-2* | Cytochrome b5 | P49098 | 400 | 1.00E-35 | 0.00169837900 | 2.238644629 | 2.553288409 | 2.6323499 | 2.1717374 | 2.056643887 | 1.594585524 |
| 5174 | *TrGT2* | Glycosyl transferase, family 31 | Q1SGZ2 | 646 | 8.70E-62 | 0.00327826474 | 0.840512589 | 0.925310082 | 0.7647323 | 0.0928043 | 0.042152182 | 0.345209637 |
| 9306 | *TrANSL2* | Anthocyanidin synthase | Q9FFF6 | 349 | 6.80E-35 | 0.00333332500 | 0.090690607 | 0.297013091 | 0.5465757 | -0.440799 | -0.765211274 | -0.773588141 |
| 1186 | *TrCHI2* | Chalcone-flavonone isomerase | O22604 | 184 | 7.80E-13 | 0.00486386171 | 3.781814829 | 4.66003136 | 4.7573918 | 4.5920329 | 4.023287173 | 3.66644844 |
| 2106 | *TrUFGT2* | UDP-glucose glucosyltransferase | O04930 | 387 | 2.40E-34 | 0.00735053800 | -0.378609873 | -1.303647964 | -0.9898406 | -2.3490026 | -2.673143843 | -2.746032742 |
| 4370 | *TrOMT2* | O-methyltransferase, family 2 | Q1S8W2 | 144 | 7.00E-08 | 0.01870341800 | 4.491943929 | 4.382175124 | 3.9265657 | 3.316574 | 1.919562837 | 2.351266911 |
| 6720 | *TrUFGT3* | UTP-glucose glucosyltransferase | O23205 | 131 | 2.60E-06 | 0.02378190600 | -2.663078454 | -1.984268775 | -2.1262866 | -2.9618972 | -2.287142707 | -2.341763776 |
| 1793 | *TrGT3* | Glucosyltransferase-like protein | O49492 | 166 | 5.00E-10 | 0.02813340900 | -1.450600699 | -1.933213119 | -1.6369657 | -3.4455076 | -2.42730273 | -2.311084381 |
| 4232 | *TrGT4* | O-linked GIcNAc transferase | O26186 | 128 | 4.10E-06 | 0.03102278500 | 2.237417488 | 2.68200662 | 2.4123661 | 1.785924 | 1.631157875 | 1.460475849 |
| 10644 | *TrART1* | anthocyanidine rhamnosyl-transferase | Q8S342 | 681 | 1.70E-65 | 0.03318746000 | 3.093639962 | 3.064225079 | 2.965625 | 1.3893601 | -0.426162741 | -1.051226447 |
| 6504 | *TrLac3* | Laccase | O22917 | 367 | 7.00E-32 | 0.04562514800 | -5.002555419 | -4.019037915 | -4.6814486 | -4.8321705 | -4.703723902 | -4.698797732 |

| **Transcriptional factors** | | | | | | | | | | | | |
|---|---|---|---|---|---|---|---|---|---|---|---|---|
| 8138 | | TGACG-sequence-specific DNA-binding protein | O24160 | 253 | 1.30E-19 | 0.00000000079 | 1.865175654 | 1.119620895 | 1.0461317 | -1.4875006 | -0.986519753 | -0.330680465 |
| 9405 | | Transcription activator | Q8L8A8 | 139 | 1.00E-13 | 0.00000016200 | 1.178274225 | 0.640475872 | 0.2566204 | -1.0653888 | -1.2395845 | -0.510626212 |
| 10472 | | Zinc finger transcription factor | O65036 | 437 | 1.20E-39 | 0.00005900000 | 1.040163465 | 0.941506641 | 0.1549588 | -0.4668842 | 0.003699524 | 0.127676762 |
| 6820 | | Squamosa promoter-binding-like protein 3 | P93015 | 142 | 2.10E-08 | 0.00012152500 | 2.864251171 | 2.701065988 | 2.3037749 | 0.7053791 | 0.498217436 | 0.059074569 |
| 3641 | | GLABRA2 like protein | O23611 | 203 | 1.70E-31 | 0.00013640600 | 1.787705468 | 1.07772996 | 1.4670592 | -0.5758142 | -1.165866751 | -1.744076731 |
| 10471 | | Zinc finger transcription factor | O65036 | 425 | 2.10E-38 | 0.00018232200 | 0.932182831 | 0.019581429 | 0.1113621 | -1.7648071 | 0.01021813 | 0.556230041 |

| Probe number | Name | Annotation | UniRef90 | Score | E-value | p.val | Stage 1 | Stage 2 | Stage 3 | Stage 4 | Stage 5 | Stage 6 |
|---|---|---|---|---|---|---|---|---|---|---|---|---|
| 5438 | *TrMYB1* | MYB24 | Q2LME0 | 492 | 1.80E-45 | 0.00036309400 | 0.264124386 | 1.212814163 | 0.745303 | -0.6247157 | -0.362648219 | 0.025971698 |
| 2153 | *TrMYB2* | MYB59 related | Q4JL84 | 134 | 2.40E-07 | 0.00212627900 | 1.924334773 | 1.369501837 | 1.5078738 | 1.5166078 | 1.366545571 | 0.640299585 |
| 7738 | | Putative CCCH-type zinc finger protein | O82199 | 168 | 1.50E-10 | 0.00513551200 | 3.574535057 | 2.732027991 | 2.8510478 | 1.7473991 | 2.932212171 | 3.098670542 |
| 1514 | | GRAS transcription factor | Q1S6U1 | 288 | 6.40E-25 | 0.01490600700 | -3.099350618 | -2.790536615 | -2.7951157 | -4.4039577 | -4.400590734 | -3.404672562 |
| 7815 | | GRAS family transcription factor | Q1S255 | 188 | 3.00E-12 | 0.01947737300 | 1.873559136 | 1.489003503 | 1.1692643 | 0.1700182 | 0.399414368 | 0.209690299 |
| 4596 | *TrWDR1* | WD40-like | Q1SUQ3 | 199 | 1.10E-13 | 0.02030418300 | -1.410414117 | -1.238728347 | -1.4807259 | -2.3597923 | -2.025239292 | -2.082347404 |
| 9685 | *TrWDR2* | WD-40 repeat protein | O22467 | 153 | 9.10E-09 | 0.02041123400 | 4.100814111 | 3.454419383 | 3.4035321 | 2.6013488 | 2.652294312 | 2.53846547 |
| 7792 | *TrMYC1* | AtMYC2 | Q39204 | 281 | 2.70E-22 | 0.02425909600 | 0.717892501 | 0.85031338 | 0.9635264 | 0.1477635 | 0.344727897 | 0.239958587 |
| 10656 | *TrTT1* | Transparent testa 1 protein TT1 | Q1SGF6 | 237 | 1.80E-18 | 0.02682476200 | 0.476229301 | 0.95096276 | 0.7832894 | 0.0655802 | 0.300619845 | 0.295767657 |
| 6487 | | Helix-loop-helix DNA-binding | Q1SCX7 | 126 | 2.30E-06 | 0.02993908200 | -1.331444782 | -1.1632329 | -1.1739826 | -2.0970584 | -1.781917421 | -1.568745378 |
| 812 | *TrWDR3* | WD-repeat protein | O23919 | 216 | 1.30E-15 | 0.03031877100 | 1.517700195 | 0.70812978 | 0.6191753 | -0.4607095 | -0.380221258 | -0.578927004 |
| 7934 | | Transcriptional factor B3; Cupredoxin; TonB box | Q1S2L8 | 187 | 4.00E-12 | 0.04554921400 | 1.532484855 | 1.231365605 | 1.4111784 | 0.4924264 | 0.725621672 | 1.039777775 |
| 2885 | | WRKY-type DNA binding protein | Q5IZC7 | 356 | 4.70E-31 | 0.04979756400 | -0.716461123 | -0.454970468 | -0.5213443 | -0.7926652 | -0.949510533 | -0.922300624 |

| **Protein-protein interaction/Protein stability** | | | | | | | | | | | | |
|---|---|---|---|---|---|---|---|---|---|---|---|---|
| 8398 | | ZF-HD homeobox protein | Q9SB61 | 260 | 6.30E-21 | 0.00000000000 | 2.917579719 | 2.41208071 | 1.58734 | -3.47965 | -2.54466579 | -2.39632793 |
| 6955 | | Zinc finger, RING-type | Q2HRJ4 | 541 | 9.90E-51 | 0.00034805100 | -1.317245586 | -0.2132285 | 0.730291 | -0.09191 | -0.41963813 | -1.38337892 |
| 9830 | | Cyclin-like F-box | Q1SPN8 | 349 | 2.50E-30 | 0.00114909400 | -2.244729216 | -2.8804263 | -2.50292 | -4.30092 | -3.53578226 | -4.26338582 |
| 3510 | | Zinc finger protein | Q1RPX5 | 94 | 1.50E-06 | 0.00157821500 | 1.889610084 | 1.50729984 | 1.461671 | 0.814281 | 0.735505654 | 0.86723045 |
| 1927 | | RING-H2 finger protein RHG1a-like | Q5N7R4 | 157 | 2.20E-09 | 0.00429985700 | 1.055480583 | 0.59739798 | 0.584351 | -0.20521 | -0.1783458 | 0.3685362 |
| 3923 | | Homeobox domain, ZF-HD class protein | Q1RVW1 | 202 | 9.80E-15 | 0.00768176600 | -0.036151633 | -0.393332 | -0.3564 | -1.1131 | -0.76205755 | -0.46640585 |
| 187 | | PHD finger protein | O81488 | 334 | 5.90E-39 | 0.00879598700 | 1.58588998 | 1.03026863 | 0.916608 | 0.299459 | 0.362980411 | 0.465875 |
| 7123 | | HD-Zip protein | O04291 | 120 | 1.90E-13 | 0.02820591600 | -2.857439899 | -3.591211 | -2.90581 | -4.516 | -3.57309136 | -4.12004908 |
| 2321 | | F-box family protein | Q1PEN2 | 128 | 1.40E-06 | 0.03206785800 | 0.670603573 | 0.30729099 | 0.568291 | -0.2059 | 0.325926164 | 0.36462149 |
| 272 | | Cyclin-like F-box | Q1T2D4 | 128 | 3.80E-06 | 0.03262094400 | -1.382481168 | -1.3737679 | -1.3208 | -2.2972 | -1.8327159 | -1.83170906 |
| 152 | | Ubiquitin related | O96951 | 557 | 2.10E-52 | 0.03433194300 | 4.326076876 | 4.14730291 | 4.362717 | 3.519191 | 3.216720715 | 3.43269419 |

| Probe number | Name | Annotation | UniRef90 | Score | E-value | p.val | Stage 1 | Stage 2 | Stage 3 | Stage 4 | Stage 5 | Stage 6 |
|---|---|---|---|---|---|---|---|---|---|---|---|---|
| 3338 | | Coiled-coil protein | Q53JH6 | 148 | 1.10E-07 | 0.04250694000 | 1.697647417 | 0.96667017 | 1.025332 | 0.061005 | -0.36525057 | -0.629163 |

| **Transporters** | | | | | | | | | | | | |
|---|---|---|---|---|---|---|---|---|---|---|---|---|
| 5368 | | Aquaporin-7 | O14520 | 149 | 1.60E-08 | 0.00000000227 | -1.968749895 | -0.0240636 | -0.06103 | -4.23084 | -4.47944462 | -4.35003412 |
| 8181 | | Putative nitrate transporter NRT1-3 | Q9FRU4 | 515 | 1.10E-51 | 0.00000025700 | 1.038967004 | 2.49613923 | 2.701683 | 0.878395 | 1.312987803 | 1.37476525 |
| 10735 | | Putative ABC transporter | O80946 | 641 | 2.90E-61 | 0.00000087000 | -3.294318862 | -0.792273 | 1.275504 | 0.236861 | -0.92284402 | -1.61977005 |
| 3925 | | Putative peptide/amino acid transporter | Q7XJQ3 | 367 | 6.80E-32 | 0.00005910000 | 1.083437106 | 0.7737491 | 0.73981 | -0.70961 | -1.03698706 | -0.46382144 |
| 1559 | | Lipid transfer protein | O22110 | 117 | 9.80E-06 | 0.00044650300 | 1.869134794 | 2.75294269 | 2.830493 | 1.769676 | -0.40405179 | -0.92710864 |
| 5021 | | Lipid transfer protein | O22110 | 155 | 8.80E-10 | 0.00054652500 | -4.145000083 | -2.1410899 | -3.71468 | -4.83217 | -4.99002567 | -5.6006332 |
| 10076 | | Potassium transporter 1 | O22397 | 301 | 2.70E-24 | 0.00180806000 | -2.617550432 | -1.96788 | -2.07191 | -3.3922 | -2.80110247 | -2.52123239 |
| 8973 | | High-affinity potassium transporter | Q3V5P7 | 232 | 4.70E-17 | 0.00293218800 | -2.184294808 | -1.2328235 | -0.93375 | -2.47444 | -1.43627325 | -1.61639613 |
| 3836 | | ABC transporter, transmembrane region, type 1 | Q1RSS5 | 783 | 3.50E-75 | 0.00297302800 | 2.766144705 | 2.00725501 | 2.132366 | 1.545695 | 1.650469887 | 1.76971828 |
| 10295 | | Lipid transfer protein | O64431 | 212 | 8.50E-16 | 0.00504234900 | 1.185377611 | 1.37500426 | 1.064649 | 0.011351 | 0.741227839 | 1.11887917 |
| 1377 | | Outer envelope membrane protein | Q41041 | 185 | 5.50E-13 | 0.00853821500 | 2.17520816 | 2.05917165 | 2.542805 | 1.236224 | 1.631169658 | 2.01948204 |
| 9523 | | Vacuolar protein sorting 26 | O01258 | 613 | 2.50E-58 | 0.01226584800 | -3.594863962 | -3.0829089 | -2.93241 | -4.26324 | -4.35257326 | -3.39232487 |
| 4821 | | Putative membrane related protein CP5 | Q6Z705 | 283 | 2.50E-23 | 0.01694889700 | 1.651698606 | 1.23973613 | 1.44573 | 0.921058 | 1.459948228 | 1.57035598 |
| 3280 | | Outer membrane protein, OMP85 family | Q2RBM6 | 423 | 3.90E-38 | 0.03878474200 | 1.089545492 | 1.00674035 | 1.353441 | 0.5735 | 0.289741634 | 0.29985128 |

| **Cell signalling** | | | | | | | | | | | | |
|---|---|---|---|---|---|---|---|---|---|---|---|---|
| 8094 | | Similarity to elicitor-inducible receptor-like protein | Q9FH56 | 253 | 8.80E-20 | 0.00000000003 | 0.101740553 | 2.21086836 | 1.125783 | -3.64913 | -3.8252675 | -2.09967215 |
| 2740 | | Receptor protein kinase-like protein | O49483 | 373 | 1.00E-31 | 0.00000139000 | 1.205633699 | 0.84929486 | 0.764088 | -0.15207 | -0.7016875 | -0.33353066 |
| 7805 | | Putative receptor protein kinase | Q69SP5 | 171 | 4.30E-10 | 0.00080635900 | 4.003058694 | 3.8801436 | 3.578249 | 2.598014 | 2.171140345 | 1.65663638 |
| 1745 | | Receptor protein kinase-like protein | Q8LA44 | 705 | 4.90E-68 | 0.00216724400 | 2.144741442 | 1.89486445 | 1.844677 | 0.976641 | 0.682397103 | 1.19534427 |
| 6776 | | Protein kinase r | Q1SDD6 | 705 | 4.80E-68 | 0.00288470600 | 0.525010045 | 0.46258586 | 0.481454 | -0.26149 | -0.13817257 | -0.00793303 |
| 1508 | | Protein kinase | Q1ST94 | 759 | 8.90E-74 | 0.00861234600 | -0.342503804 | -0.2931435 | 0.239749 | -1.39972 | -2.23945056 | -2.5858146 |
| 1910 | | Kinase like protein | O23334 | 346 | 5.40E-30 | 0.00904263800 | 1.170335392 | 0.83535418 | 0.763816 | -0.29759 | -1.14377959 | -1.04943362 |
| 2943 | | Receptor-kinase | O04098 | 131 | 4.30E-06 | 0.01061665248 | 1.171339072 | 0.73206235 | 0.969261 | -0.52571 | -0.10857917 | -0.23741244 |
| 531 | | Putative receptor-like protein kinase | O81069 | 136 | 1.70E-06 | 0.02496065400 | 3.221141985 | 3.33692224 | 3.4499 | 2.628687 | 2.512394524 | 2.97364395 |
| 3140 | | Protein kinase related | Q1SMH9 | 663 | 1.40E-63 | 0.02702405700 | 1.716967142 | 1.55045231 | 1.295714 | 0.686379 | 0.428201226 | 0.90755351 |
| 8618 | | Cdc2 cyclin-dependent kinase | O13379 | 381 | 1.00E-33 | 0.02816313800 | 3.184492717 | 2.56892364 | 2.458818 | 1.772137 | 1.678094659 | 1.70784117 |
| 2822 | | Protein kinase | Q1SQL4 | 151 | 1.30E-08 | 0.03164401200 | -2.918763359 | -2.6262791 | -2.94466 | -4.4761 | -4.59933972 | -4.14614927 |
| 8349 | | Receptor protein kinase | O49545 | 850 | 2.00E-83 | 0.03442350100 | 0.87618951 | 0.36559413 | 0.885606 | 0.052315 | 0.162632507 | 0.24726914 |
| 698 | | Lectin-like protein kinase | Q9FG33 | 288 | 5.70E-23 | 0.03712385400 | -3.551089127 | -2.6577382 | -3.58957 | -4.58073 | -4.32456627 | -3.68115131 |
| 2884 | | Protein kinase; amino acid-binding ACT | Q1RX25 | 417 | 5.40E-37 | 0.04319084000 | 1.347968197 | 1.59937983 | 1.360195 | 0.945737 | 1.009151951 | 1.43771012 |
| 4116 | | S-receptor kinase related protein | Q05970 | 292 | 1.50E-34 | 0.04992617000 | -2.39154687 | -1.5808777 | -2.15774 | -2.99469 | -3.75675321 | -2.73950557 |

| **Metabolic enzymes** | | | | | | | | | | | | |
|---|---|---|---|---|---|---|---|---|---|---|---|---|
| 7466 | | Cytochrome P450 | O22162 | 317 | 1.20E-26 | 0.00000000004 | 0.203651944 | 1.88142986 | 1.461009 | -3.14798 | -3.13960175 | -2.26854776 |
| 3458 | | 4-coumarate-CoA ligase | O24540 | 183 | 8.80E-12 | 0.00000000202 | 1.238961277 | 2.78283064 | 1.324656 | -4.08216 | -4.10758667 | -4.07150353 |
| 6509 | | (R)-mandelonitrile lyase 1 precursor (EC 4.1.2.10) | O24243 | 273 | 1.60E-21 | 0.00000061433 | -0.506505437 | 1.09756747 | 1.453386 | -0.43376 | -1.46157148 | -0.4417474 |
| 3579 | | Putative steroid dehydrogenase | O16925 | 166 | 1.80E-10 | 0.00002559599 | 1.619932489 | 1.80784466 | 1.841471 | 0.46391 | -0.89511457 | -0.69072362 |
| 7829 | | Putative strictosidine synthase | O81484 | 296 | 1.20E-24 | 0.00006543894 | -0.855368371 | 0.1953185 | -1.64507 | -4.83217 | -4.30173477 | -5.6006332 |
| 8884 | | Putative strictosidine synthase | O81484 | 241 | 1.10E-18 | 0.00007422973 | 0.55917067 | 1.72871541 | 1.18475 | -1.78854 | -2.79012051 | -3.51211877 |
| 5702 | | Dihydrofolate reductase | P36591 | 165 | 6.00E-10 | 0.00011442540 | 0.493139132 | 1.07031204 | 1.492219 | 0.397031 | -0.50368779 | -0.84666126 |
| 6768 | | 4-coumarate-CoA ligase 2 | P31687 | 722 | 7.20E-70 | 0.00018069333 | -2.42175446 | -1.2518912 | -0.31969 | -0.80211 | -2.04809075 | -4.155764 |
| 9988 | | Cytochrome P450 monooxygenase CYP711A | Q2MIZ8 | 510 | 2.30E-47 | 0.00120167300 | 3.245067154 | 2.0266807 | 2.128803 | 0.234667 | -0.49954028 | -0.97601417 |
| 7901 | | Cytochrome P450 | O22162 | 157 | 4.70E-09 | 0.00228282900 | 3.685995351 | 3.84329402 | 3.603733 | 3.520901 | 2.158644892 | 1.39744574 |

| Probe number | Name | Annotation | UniRef90 | Score | E-value | p.val | Stage 1 | Stage 2 | Stage 3 | Stage 4 | Stage 5 | Stage 6 |
|---|---|---|---|---|---|---|---|---|---|---|---|---|
| 4634 | | Oxygenase | O82031 | 700 | 1.70E-67 | 0.00611212514 | -4.412641106 | -2.999877 | -2.91296 | -3.69866 | -3.62852401 | -3.27352365 |
| 10398 | | Phenylalanine ammonia-lyase | O04058 | 559 | 1.50E-52 | 0.01251835036 | 0.766038743 | 0.06124215 | 0.559555 | -0.63327 | -1.6973529 | -4.25134318 |
| 1557 | | Cytochrome P450 | O65624 | 173 | 7.00E-11 | 0.01273526700 | -4.117508164 | -3.4958991 | -3.78859 | -4.76992 | -4.4528391 | -4.26328698 |
| 7238 | | Cytochrome P450 monooxygenase CYP83C | Q2MJ14 | 481 | 2.40E-44 | 0.01380961100 | -2.921544347 | -2.8123123 | -2.50855 | -3.95605 | -3.95040451 | -3.04928061 |
| 2010 | | Putative plastid glucose 6 phosphate/phosphate translocator | Q58J24 | 878 | 2.40E-86 | 0.01821603533 | -3.185146237 | -2.0008183 | -2.72482 | -3.59116 | -4.00403093 | -3.8658431 |
| 1114 | | Cytochrome P450 | O81346 | 132 | 2.80E-06 | 0.01856459000 | 1.029604342 | -0.2633534 | -0.06268 | -1.84737 | -4.99002567 | -5.6006332 |
| 5577 | | Cinnamyl alcohol dehydrogenase | O04391 | 131 | 1.40E-06 | 0.01899200506 | 4.426760644 | 4.53382638 | 4.290437 | 3.937205 | 3.684932953 | 2.89097629 |
| 7028 | | Cytochrome P450 | O04163 | 137 | 7.30E-07 | 0.02427402800 | 0.672464007 | -0.9720905 | -0.49147 | -2.57424 | -4.99002567 | -5.6006332 |
| 3741 | | Anthranilate phosphoribosyltransfe rase | Q9LXU2 | 422 | 2.80E-37 | 0.03717674881 | -3.760790736 | -2.5333262 | -3.83917 | -4.71984 | -4.52772646 | -4.31775019 |
| 8645 | | Cytochrome P450 | O22307 | 135 | 1.10E-06 | 0.03813879000 | 1.918509487 | 1.00848128 | 1.831241 | -0.0572 | -0.7835104 | -1.48914346 |

**Table 2**

| Probe number | Name | Annotation | UniRef90 | Score | E-value | p.val | Stage 1 | Stage 2 | Stage 3 | Stage 4 | Stage 5 | Stage 6 |
|---|---|---|---|---|---|---|---|---|---|---|---|---|
| **Flavonoid pathway enzymes** | | | | | | | | | | | | |
| 10565 | *TrCHS8* | Chalcone synthase | P51086 | 1236 | 2.70E-124 | 0.00000001174 | 0.96480281 | 3.322898114 | 4.00228616 | 3.87549408 | 3.89820985 | 0.992977007 |
| 3706 | *TrANSL3* | Anthocyanidin synthase | Q9FFF6 | 859 | 2.30E-84 | 0.00000001865 | 1.199326818 | 0.884659537 | 2.41666348 | 3.71364005 | 3.03810313 | 0.886900586 |
| 2895 | *TrIFR1* | NADPH:isoflavone reductase | 048601 | 536 | 3.60E-50 | 0.00000002998 | -0.297447867 | -0.380412074 | 0.73368331 | 4.21773799 | 3.79393938 | 4.022554639 |
| 2307 | *TrANAT3* | Anthocyanin 5-aromatic acyltransferase | 004201 | 786 | 1.20E-76 | 0.00000003024 | -0.074903868 | -0.026842085 | -0.3497689 | 0.88152019 | 1.08204864 | 0.146152232 |
| 10572 | *TrCHS9* | Chalcone synthase | P51090 | 748 | 1.30E-72 | 0.00000003357 | 1.615774262 | 0.76353607 | 2.44172631 | 2.7839425 | 2.54239333 | -0.071725969 |
| 6634 | *TrF3H2* | Flavonoid 3-hydroxylase | Q9M547 | 657 | 5.70E-63 | 0.00000036320 | 2.853117289 | 3.620093921 | 3.23878971 | 3.61629055 | 3.48207534 | 2.044546364 |
| 6728 | *TrANAT2* | Putative anthocyanin 5-aromatic acyltransferase | 004201 | 260 | 2.50E-20 | 0.00000304676 | 3.156309766 | 2.468618795 | 3.43904183 | 3.88240531 | 1.97262062 | -0.328420282 |
| 10479 | *TrGST3* | Glutathione S-transferase GST 24 | Q9FQD4 | 611 | 4.30E-58 | 0.00000448128 | -1.452691356 | -1.475286047 | -0.9609693 | 1.62118397 | 2.42074299 | 2.401772302 |
| 9976 | *TrIFOMT2* | Isoflavone-7-O-methytransferase 6 | 022308 | 388 | 1.90E-34 | 0.00001167326 | -0.003358252 | 0.30887472 | 0.24615345 | 1.72179526 | 3.44905357 | 2.787650887 |
| 2504 | *TrOMT3* | Methyltransferase | Q2QWY0 | 220 | 1.90E-15 | 0.00004396764 | 0.930179656 | 0.476379221 | 0.85660499 | 1.48323672 | 0.6931032 | -0.516035689 |
| 4494 | *TrGT6* | Glucosyltransferase-like protein | 049492 | 169 | 2.20E-10 | 0.00005706762 | -1.619272881 | -3.351752637 | -2.4189755 | -1.2628073 | -0.2879552 | -0.1501615 |
| 3607 | | NAD-dependent epimerase/dehydrata se | Q1S9W4 | 709 | 1.80E-68 | 0.00011426118 | 0.661266219 | 0.023141031 | 1.20854474 | 2.43590222 | 2.40155015 | 1.724341354 |
| 7304 | *TrDFRL3* | Dihydroflavonol-4-reductase | Q94HG6 | 557 | 2.20E-52 | 0.00015593417 | -0.797705723 | -1.477727917 | -0.8264102 | 0.84424338 | 1.37714907 | 0.740888282 |
| 10081 | *TrGT7* | Predicted glycosyl transferase | 004253 | 188 | 8.60E-13 | 0.00015791672 | 1.692117272 | 1.684134902 | 1.8992005 | 2.33488343 | 1.10255051 | 0.263966487 |
| 6717 | *TrGT8* | Glucosyltransferase | 023380 | 116 | 8.70E-10 | 0.00018282375 | -3.483224587 | -4.764589884 | -2.6146152 | -1.4419509 | -0.6694048 | -0.598396371 |
| 10569 | *TrCHS10* | Chalcone synthase | Q2HZ40 | 865 | 5.10E-85 | 0.00030343478 | 3.833193484 | 3.506048515 | 4.57847039 | 4.56629248 | 4.39119592 | 2.329268404 |
| 7334 | *TrGT9* | Predicted glycosyl transferase | 004253 | 476 | 8.40E-44 | 0.00037981397 | 0.042295454 | -0.547052858 | 0.19052962 | 1.00993581 | 0.19179559 | -0.157802666 |
| 7860 | *TrCHS11* | Chalcone and stilbene synthases | Q1S1C0 | 135 | 8.70E-07 | 0.00051863932 | -2.089094162 | -2.414482679 | -1.3307444 | -0.0254641 | -2.5453191 | -5.107719208 |
| 2662 | *TrANAT1* | Anthocyanin 5-aromatic acyltransferase | 004201 | 548 | 1.80E-51 | 0.00055146926 | -1.039611383 | -1.202796446 | -0.5426719 | 1.46754821 | 2.38170633 | 1.827445657 |
| 1225 | *TrF3H3* | Flavanone 3-hydroxylase | 004112 | 139 | 2.40E-07 | 0.00065780800 | -0.706356896 | 0.094170285 | 0.06589967 | -0.4453039 | 0.81765065 | 1.318535477 |
| 941 | *TrGT10* | Glycosyl transferase | Q1SRU1 | 1419 | 1.10E-143 | 0.00099818754 | 2.835253034 | 2.457960805 | 2.94216882 | 3.13210235 | 1.98334874 | 0.830380394 |
| 3177 | *TrOMT4* | Methyltransferase | Q1SBL8 | 1396 | 3.90E-224 | 0.00315479959 | 2.198504188 | 1.80603273 | 2.2301984 | 3.04496157 | 2.66881565 | 1.640115831 |
| 9557 | *TrIFR2* | NADPH:isoflavone reductase | 048601 | 707 | 2.60E-68 | 0.00327387448 | -0.027000121 | -0.355600794 | -0.4678631 | 0.73090779 | 0.4880981 | 0.254200173 |
| 3922 | *TrF3'H1* | Flavonoid 3'-hydroxylase | Q2PEY1 | 835 | 5.00E-84 | 0.00344262445 | -0.847191939 | -0.169172096 | 0.52741679 | 1.07814156 | 0.57889385 | -0.772705301 |
| 4090 | *TrUFGT4* | UDP-glucuronosyl/UDP-glucosyltransferase | Q1RXH1 | 491 | 6.30E-72 | 0.00577774051 | -3.768085386 | -3.705417463 | -3.4931921 | -0.5801375 | -0.1868898 | -0.003302962 |
| 7303 | *TrDFRL4* | Putative dihydroflavonol-4-reductase | P73212 | 152 | 3.8e-12 s | 0.00780987057 | 0.161062926 | 0.544211727 | 0.90993658 | 1.68268973 | 2.13577877 | 1.753272879 |
| 3886 | *TrIFOMT1* | Isoflavone-7-O-methytransferase 6 | 022308 | 323 | 1.50E-27 | 0.00967695180 | -4.544662109 | -3.947053001 | -4.0845466 | -1.6450444 | -0.4021697 | -0.405646303 |
| 4998 | *TrUFGT5* | UDP-glucose glucosyltransferase | 023205 | 168 | 2.30E-10 | 0.01112955946 | 0.63042667 | -0.077188457 | 1.10777477 | 1.87843084 | 2.04644237 | 1.487967102 |
| 8039 | *TrGST4* | Glutathione S-transferase | P32110 | 672 | 1.40E-64 | 0.01358354612 | -2.705125398 | -2.312519763 | -2.6856047 | -2.0493654 | -1.7850778 | -2.489887792 |
| 5123 | | UDP-glucose 4-epimerase | Q43070 | 860 | 1.90E-84 | 0.01459701948 | 1.92065834 | 2.2401848 | 2.51781835 | 3.88339203 | 4.04372151 | 3.99993236 |
| 9144 | | 2-hydroxyisoflavanone dehydratase | Q5NUF3 | 132 | 1.10E-06 | 0.01470554943 | -4.648104276 | -4.236008056 | -4.797643 | -2.9031246 | -2.4688888 | -2.391390287 |
| 2607 | *TrFS1* | Flavonol synthase-like protein | Q8LCJ7 | 707 | 1.90E-15 | 0.02002049077 | -0.242762166 | -0.002955064 | -0.1571982 | 0.51751962 | 0.55574443 | 0.118328464 |
| 3363 | | UTP-glucose-1-phosphate uridylyltransferase | 059819 | 492 | 1.80E-45 | 0.04040425184 | 2.165179633 | 1.60480234 | 2.51048762 | 2.7480727 | 2.6332539 | 2.151637792 |
| 9456 | *TrOMT5* | Putative methyltransferase | Q1SBL8 | 528 | 2.70E-49 | 0.04721257054 | 1.086497942 | 2.054048383 | 3.056356 | 4.17232741 | 3.15307818 | 1.490082949 |
| **Transcriptional factors** | | | | | | | | | | | | |
| 4631 | *TrMYB3* | MYB10 protein | Q70RD0 | 497 | 5.00E-46 | 0.00000000000 | -0.596335089 | 1.752717145 | 2.48151852 | 3.00807738 | 1.98067054 | 0.277930088 |
| 4321 | | LIM, zinc-binding; Zinc finger, RING-type | Q1T3L7 | 698 | 2.80E-67 | 0.00000000038 | -0.824258566 | -0.908764425 | -0.1156481 | 4.63422241 | 4.9905896 | 4.825375159 |
| 5762 | *TrMYB4* | Myb26 | P93474 | 523 | 9.10E-49 | 0.00000000116 | -4.666499493 | -4.72759484 | -0.9145317 | 3.30684646 | 3.55574034 | 2.357025239 |
| 10018 | | Zinc-finger protein BcZFP1 | 004177 | 206 | 3.70E-15 | 0.00000001204 | -2.81746627 | -5.143540905 | -4.6611458 | -0.3911345 | -0.3220759 | 0.337640289 |
| 5691 | | Homeobox domain, ZF-HD class | Q1RVW1 | 197 | 3.00E-14 | 0.00000001213 | -4.846316664 | -4.186031134 | -1.3927238 | 3.52042503 | 3.10546325 | 2.659971278 |
| 7441 | | GTL1 protein | 048590 | 195 | 4.60E-13 | 0.00000011830 | 0.816984771 | 0.342323508 | 1.25896382 | 2.81691966 | 3.13894675 | 2.552565241 |
| 349 | *TrMYC2* | MYC1 | Q71SQ1 | 142 | 5.30E-08 | 0.00000241106 | 1.504896555 | 1.041040244 | 3.22856425 | 3.93886124 | 2.8166249 | 0.73766203 |
| 8253 | | Zinc finger protein CONSTANS-LIKE 14 | 022800 | 137 | 5.00E-07 | 0.00001693928 | -2.411596494 | -1.809863973 | -2.0738604 | 0.2520513 | -0.8056079 | -1.189449305 |
| 5759 | *TrMYB5* | Myb26 | P93474 | 123 | 2.80E-81 | 0.00003084487 | -3.223797578 | -3.287452461 | 0.54843483 | 3.82369429 | 4.40010663 | 4.063304283 |
| 347 | *TrbHLH1* | bHLH protein | Q69WS3 | 115 | 4.30E-28 | 0.00004871938 | -1.119001735 | -1.438735514 | -0.206187 | 0.71445574 | 0.46240282 | 0.3084384 |
| 1156 | | Mob1-like protein | Q949G5 | 826 | 7.60E-81 | 0.00005214373 | 1.161666368 | 0.865861484 | 1.66191497 | 3.57279279 | 3.92034792 | 3.636329143 |
| 8780 | domain-containing | Zinc finger A20 domain-containing protein 2 | 076080 | 173 | 1.10E-18 | 0.00009046884 | 0.62106855 | 0.263827257 | 1.20632222 | 2.73026814 | 3.18877843 | 2.965145906 |
| 4110 | | Zinc finger protein CONSTANS-LIKE 14 | 022800 | 172 | 6.90E-11 | 0.00013128398 | -1.111610901 | -0.183070509 | 0.12916419 | 1.85301386 | 1.51993445 | 1.037780577 |
| 5756 | *TrMYB6* | MYB48 | Q9LX82 | 351 | 1.70E-46 | 0.00027381600 | 0.678459497 | 0.85352738 | 0.62140192 | -0.0135602 | 1.11826672 | 2.294190967 |
| 5760 | *TrMYB7* | MYB59 | Q4JL84 | 410 | 5.40E-53 | 0.00054415300 | -2.388504016 | -2.752156769 | -3.2565379 | -4.6547908 | -3.1579039 | -0.494292045 |
| 6636 | | Zinc finger, RING-type; RINGv | Q1S8X5 | 127 | 4.30E-06 | 0.00094161454 | -3.807638081 | -3.050462259 | -4.2228028 | -0.4693914 | -0.3876906 | -0.270577761 |
| 4588 | | ARF GAP-like zinc finger-containing protein | Q69QY4 | 329 | 3.40E-28 | 0.00140755899 | -3.763765845 | -4.301434883 | -4.4336404 | -1.9269059 | -1.6606546 | -2.019324028 |
| 816 | | BSD domain, putative | Q2R2M4 | 96 | 1.10E-06 | 0.00283644260 | -2.80418398 | -3.056753723 | -2.7728556 | -1.361118 | -0.8328079 | -1.226019351 |
| 3001 | *TrWDR4* | WD-40 repeat | Q1SAJ3 | 328 | 4.30E-28 | 0.00294894217 | -1.00220639 | -1.038085758 | -0.6360313 | 0.32582121 | 0.23008991 | -0.046119735 |
| 350 | *TrMYC3* | MYC1 | Q71SQ1 | 123 | 6.90E-06 | 0.00355653234 | -0.201989055 | -0.70451272 | 0.65710861 | 1.30584118 | 0.67074996 | -0.911343679 |
| 7368 | *TrbHLH2* | bHLH transcription factor | Q5N802 | 621 | 3.50E-59 | 0.00425985840 | -1.324644041 | -1.527806096 | -0.6365379 | 0.60032412 | 1.08038561 | 0.829192383 |
| 6531 | | WRKY65 | Q2PJS0 | 279 | 6.30E-23 | 0.00607014680 | 0.909565985 | 0.361602153 | 0.5462086 | 1.06739188 | 1.08906683 | 0.869138013 |
| 2327 | | Zinc finger, RING-type; RINGv | Q1SJS5 | 186 | 3.40E-24 | 0.00963864052 | 0.962929201 | 0.559770449 | 0.85974036 | 1.56222975 | 1.71712685 | 1.333033793 |
| 2245 | | MADS-box transcription factor | 065874 | 1195 | 5.90E-120 | 0.01365747100 | 2.056148179 | 2.236120629 | 3.11351409 | 3.7416017 | 3.33000085 | 3.087985555 |
| 5391 | | YABBY protein | Q1S622 | 228 | 1.50E-17 | 0.01668545474 | 1.457812389 | 1.913856205 | 2.69882433 | 3.06596091 | 3.14819209 | 2.584173997 |
| 419 | *TrMADS1* | MADS-box protein | Q5VKS3 | 529 | 2.10E-49 | 0.02535192704 | 0.906291107 | 0.836052456 | 1.24358833 | 1.95680382 | 1.9282514 | 2.046237241 |
| 3244 | | TGACGsequence-specific DNA-binding protein | P14232 | 126 | 6.00E-06 | 0.02866272128 | -2.493642446 | -1.931788781 | -0.9592391 | -0.5188558 | -1.8038804 | -2.662751943 |
| 5696 | *TrMYB8* | Myb-like protein | Q69WS3 | 547 | 2.30E-51 | 0.02958517601 | 0.308078039 | 0.066782055 | 0.38264333 | 1.42609898 | 2.2713874 | 2.241017064 |
| 7050 | | Zinc finger family protein | Q3ZDI4 | 247 | 1.70E-19 | 0.03357164146 | 2.603900793 | 2.645239551 | 2.55064734 | 3.15642289 | 3.18498219 | 2.623072857 |
| 8783 | | Zinc finger, AN1-type; Zinc finger | Q1 SGE4 | 576 | 1.90E-54 | 0.03561264401 | -1.106720305 | -1.320243681 | -0.5844656 | 0.70249722 | 1.21010793 | 1.235902756 |
| 4808 | | GAI-like protein GAI2 | Q20CJ6 | 124 | 1.20E-14 | 0.03613666507 | -1.392364905 | -1.698069015 | -0.8182756 | -0.2071728 | -1.312731 | -2.180975501 |
| 986 | | C2H2 zinc-finger protein ZPT2-10 | 022082 | 135 | 2.50E-07 | 0.03865252300 | 1.065925362 | 1.055480905 | 0.87810999 | 2.07873026 | 2.2198447 | 2.189261557 |

| **Protein-protein interaction, Protein stability** | | | | | | | | | | | | |
|---|---|---|---|---|---|---|---|---|---|---|---|---|
| 6196 | | BRCA1-associated RING domain protein | 004097 | 194 | 5.90E-13 | 0.00000001163 | -3.693146005 | -3.42169137 | -4.2676622 | 1.43653738 | 1.84930971 | 1.95852578 |
| 2272 | | Kelch repeat containing F-box protein | Q69NY4 | 189 | 1.10E-12 | 0.00109274258 | -0.661515831 | -0.520385266 | -0.2041594 | 1.11030556 | 1.63577187 | 1.638108952 |
| 7445 | | Zinc finger, RING-finger | Q1RYX6 | 682 | 1.70E-77 | 0.00146560100 | 2.074862868 | 1.838152848 | 1.99121661 | 2.5848772 | 2.14686311 | 1.484045322 |
| 8788 | | Ubiquitin ligase SINAT5 | Q60EU0 | 134 | 7.80E-07 | 0.00322390058 | 1.052736492 | 0.896253238 | 1.01382719 | 2.30784104 | 2.68465239 | 2.626759999 |
| 7800 | | Putative ring finger protein 10 | Q67UM6 | 250 | 9.80E-19 | 0.02707506201 | -1.384419799 | -1.975873378 | -1.4165422 | -0.761646 | -0.753895 | -1.006531959 |
| 7050 | | Zinc finger family protein | Q3ZDI4 | 247 | 1.70E-19 | 0.03357164100 | 2.603900793 | 2.645239551 | 2.55064734 | 3.15642289 | 3.18498219 | 2.623072857 |

| **Auxin biosynthesis/signal transduction** | | | | | | | | | | | | |
|---|---|---|---|---|---|---|---|---|---|---|---|---|
| 3469 | | Putative auxin-regulated protein | 022150 | 156 | 8.40E-10 | 0.00000000134 | -2.182780364 | -1.977513434 | -2.1746366 | 1.12525068 | 2.44488655 | 2.145523555 |
| 4860 | | Auxin Efflux Carrier | Q1RYA5 | 163 | 6.20E-10 | 0.00009116201 | 1.305049999 | 0.927006735 | 1.95717551 | 3.22632159 | 3.51680303 | 3.102785875 |
| 8921 | | Auxin-induced protein | P33081 | 316 | 8.00E-27 | 0.00462800590 | -2.098642863 | -1.247506552 | -0.5823168 | 1.09765912 | 1.93689822 | 1.976434015 |
| 7410 | | Auxin-binding protein ABP20 | 004011 | 354 | 7.50E-31 | 0.00970032205 | -0.467019884 | -0.521499575 | 0.14487617 | 1.2103527 | 1.17544823 | 0.921059594 |
| 8242 . | 8242 | Auxin influx carrier | Q257B2 | 945 | 1.80E-93 | 0.02278967842 | 0.019932496 | -0.167454704 | 1.25861033 | 1.9270846 | 1.7266444 | 1.017798075 |
| 180 | | Putative auxin-regulated protein | 022150 | 181 | 1.70E-12 | 0.04542922939 | 0.903341307 | 1.798136861 | 1.41570349 | 2.91797542 | 2.68897258 | 2.710678818 |

| ***Transporters*** | | | | | | | | | | | | |
|---|---|---|---|---|---|---|---|---|---|---|---|---|
| 10356 | | Intracellular chloride Q1RSI2 | | 534 | 6.80E-91 | 0.00000000013 | -1.612836023 | -1.094920759 | -0.5422099 | 3.52773165 | 4.16746114 | 4.503508301 |

| Probe number | Name | Annotation | UniRef90 | Score | E-value | p.val | Stage 1 | Stage 2 | Stage 3 | Stage 4 | Stage 5 | Stage 6 |
|---|---|---|---|---|---|---|---|---|---|---|---|---|
| | | channel | | | | | | | | | | |
| 3739 | | Sucrose transporter | Q7XA53 | 595 | 2.10E-56 | 0.00000000164 | -1.317258469 | -0.650783179 | -0.7451105 | 3.89365153 | 4.63117797 | 4.680705134 |
| 3838 | | Monosaccharide transporter 4 | Q6VEF2 | 429 | 7.90E-39 | 0.00000000674 | -4.529827508 | -3.323473545 | -3.9548701 | 1.31166486 | 2.02292197 | 2.22721995 |
| 1056 | | Multifunctional aquaporin | Q8W4T7 | 710 | 1.40E-68 | 0.00000009857 | 1.96059916 | 1.68558473 | 2.93065911 | 3.59759846 | 1.62926348 | 0.216902719 |
| 10025 | | Ras small GTPase, Rab type | Q1T281 | 382 | 7.80E-34 | 0.00000227378 | -0.991034158 | -1.222719401 | -0.9246263 | 1.10694827 | 1.95004767 | 1.67471184 |
| 271 | | Nonspecific lipid-transfer protein precursor | 023758 | 375 | 4.30E-33 | 0.00000485664 | -0.440130668 | -0.162165943 | -0.1511693 | 1.76051972 | 3.14566346 | 3.154719505 |
| 10355 | | Intracellular chloride channel | S1RSI2 | 806 | 9.20E-79 | 0.00000497384 | -3.040218075 | -3.133602942 | -3.0682114 | -0.4349603 | -1.218144 | -0.874784809 |
| 1822 | | Lipid transfer protein precursor | Q9M6B7 | 390 | 1.10E-34 | 0.00000510278 | -1.714198949 | -0.739838444 | 3.16937289 | 5.83924047 | 5.68719812 | 4.686504697 |
| 262 | | Nonspecific lipid-transfer protein precursor | 004004 | 128 | 7.10E-07 | 0.00000748778 | -0.258479901 | 1.750326558 | 4.4013145 | 5.95618208 | 6.37651491 | 5.796346189 |
| 6346 | | Plant lipid transfer protein | Q1S9L0 | 220 | 1.20E-16 | 0.00001520563 | 3.720461465 | 3.518693795 | 4.15118455 | 4.66975565 | 3.70962014 | 1.47169231 |
| 9582 | | Small GTP-binding protein | Q39345 | 714 | 5.40E-69 | 0.00002509089 | -0.630677235 | -1.131391532 | -0.4762646 | 0.45528868 | 0.08291592 | -0.433735944 |
| 10024 | | Ras small GTPase, Rab type | Q1T281 | 396 | 2.60E-35 | 0.00005379597 | -0.59779102 | -1.346348164 | -0.5358057 | 1.96326944 | 2.37074757 | 1.896897284 |
| 3599 | | Multidrug resistance-associated protein 14 | Q9LYS2 | 873 | 5.00E-85 | 0.00008952168 | -3.723918811 | -4.296188979 | -4.2999857 | -2.6875386 | -3.1651493 | -5.228318169 |
| 1176 | | RabGAP/TBC | Q1RZW2 | 137 | 9.00E-07 | 0.00017457583 | -0.930333972 | -0.136576228 | -0.9194294 | 1.57277812 | 2.20460052 | 2.133101664 |
| 6012 | | High-affinity nickel-transport protein-like | Q8H658 | 203 | 1.90E-14 | 0.00019365877 | 0.482118665 | -0.204974008 | 0.62193075 | 1.66475947 | 1.65368305 | 0.96286164 |
| 1941 | | Arf GTPase activating protein | Q1T0M4 | 201 | 1.20E-22 | 0.00022125234 | 0.73280586 | 0.560822632 | 0.58344579 | 1.68385426 | 2.00318378 | 1.945850459 |
| 2032 | | H+-transporting ATPase | P93265 | 774 | 3.40E-79 | 0.00024796966 | -0.664016692 | -0.847163172 | -0.2230745 | 0.90160941 | 0.73112925 | 0.697312209 |
| 6901 | | Potassium transporter | 022397 | 224 | 5.20E-16 | 0.00026946658 | 2.858300683 | 3.103054623 | 2.86194085 | 3.74911071 | 2.68207416 | 1.767881674 |
| 1759 | | Aquaporin-like transmembrane channel protein | 022339 | 137 | 1.70E-07 | 0.00054399557 | 2.552454165 | 2.098118712 | 3.14812044 | 2.33744041 | 0.78635091 | -2.196100887 |
| 2964 | | Lipid transfer protein LPT III | 022484 | 210 | 1.40E-15 | 0.00067024746 | 4.595262485 | 4.955356489 | 5.34400507 | 6.43156562 | 6.08898276 | 5.35734579 |
| 4170 | | SNARE 12 | Q5XQQ6 | 340 | 2.10E-29 | 0.00125449293 | -0.110641643 | -0.217805842 | -0.3682256 | 0.50936179 | -0.1574564 | -0.547896659 |
| 847 | | Nitrate transporter | Q9SZY4 | 640 | 3.80E-61 | 0.00140209316 | -0.447422778 | -1.199805084 | -0.0217874 | 0.75069838 | 0.32804523 | 0.262171896 |
| 3527 | | SNARE 13 | Q9LRP1 | 573 | 4.50E-54 | 0.00147075847 | 1.95299642 | 1.861072486 | 1.61859275 | 2.56812891 | 1.94838228 | 1.151722245 |
| 6582 | | Peptide/histidine transporter | 009014 | 129 | 6.40E-06 | 0.00220200204 | -1.171940785 | -0.804818505 | -1.2495838 | 0.42214355 | 0.02028958 | -0.051704703 |
| 1271 | | Putative vacuolar proton ATPase subunit E | Q9LKG0 | 672 | 1.50E-64 | 0.00600837890 | 1.77494299 | 1.433311107 | 1.87534008 | 2.34493756 | 1.54314044 | 0.694550236 |
| 6829 | | Transport protein particle 23 kDa subunit | Q03784 | 94 | 1.00E-06 | 0.01453403594 | 1.713427651 | 1.801744304 | 1.722107 | 2.19210314 | 2.06306485 | 1.276672912 |
| 3053 | | Potassium transporter 1 | 022397 | 213 | 8.90E-15 | 0.01654601057 | -4.102036434 | -4.109519076 | -3.6259648 | -1.7107772 | -2.9981216 | -3.857059312 |
| 9215 | | Plasma membrane proton ATPase | 022613 | 270 | 7.60E-21 | 0.01791813300 | 2.033181687 | 2.677627444 | 2.75176511 | 3.71709852 | 3.08857647 | 3.051038712 |
| 5426 | | ABC transporter | Q6X4V5 | 356 | 3.00E-30 | 0.02888245809 | 4.504270395 | 4.275240901 | 4.24144637 | 4.58697824 | 4.22097064 | 3.457253464 |
| 6125 | | Vacuolar ATP synthase subunit G 1 related | 082628 | 250 | 7.20E-20 | 0.03176623870 | 3.752158449 | 3.964663513 | 3.94649426 | 4.75190348 | 4.26285264 | 3.641681859 |
| 7039 | | NUDIX hydrolase; Glycosyl transferase, family 8 | Q1RTV6 | 211 | 1.30E-14 | 0.04286366626 | -1.390269556 | -1.397463511 | -1.6307063 | -0.7413481 | -1.3121466 | -2.045487907 |
| 1970 | | Nonspecific lipid-transfer protein precursor | 023758 | 380 | 1.30E-33 | 0.04581247828 | 1.393822453 | 1.367791712 | 2.14084201 | 2.82994856 | 3.85948883 | 3.559223224 |
| **Cell signalling** | | | | | | | | | | | | |
| 1485 | | Leucine-rich repeat | Q1SZL0 | 742 | 5.70E-72 | 0.00000000082 | -4.918715753 | -4.539316892 | -5.196866 | 0.15923632 | 1.34272658 | 0.432099694 |
| 9038 | | Protein kinase | Q1SAX1 | 588 | 1.20E-55 | 0.00000000128 | -5.002555419 | -4.886067288 | -4.66152 | -0.1963398 | 0.75491562 | 0.515568848 |
| 2957 | | Protein kinase | Q1S3M0 | 817 | 6.70E-80 | 0.00000000376 | -3.758145098 | -3.021380249 | -3.3772046 | 1.59441271 | 2.37965471 | 2.027269652 |
| 5889 | | Protein kinase | Q1S1R7 | 213 | 3.40E-15 | 0.00000037900 | 2.590889846 | 2.03253251 | 2.20606518 | 3.63541426 | 4.18901307 | 4.163033648 |
| 677 | | Leucine rich repeat protein precursor | Q708X5 | 144 | 6.80E-08 | 0.00000068466 | 2.724089042 | 3.00609648 | 4.15604946 | 5.40392202 | 4.94836557 | 3.792377312 |
| 6940 | | Protein kinase | Q8LES3 | 889 | 1.70E-87 | 0.00000382444 | -1.149825506 | -0.554467246 | -0.3455309 | 1.13519705 | 0.68276856 | 0.016769479 |
| 10024 | | Ras small GTPase, Rab type | Q1T281 | 396 | 2.60E-35 | 0.00005379597 | -0.59779102 | -1.346348164 | -0.5358057 | 1.96326944 | 2.37074757 | 1.896897284 |
| 9150 | | Protein kinase | Q1RZY7 | 104 | 5.80E-08 | 0.00008132054 | -0.546609423 | 0.184871167 | -0.6029804 | 1.5799121 | 2.28299273 | 2.188737558 |
| 4461 | | Aurora/ipl1 related kinase protein 2 | 001427 | 126 | 4.10E-06 | 0.00050291431 | -0.595225074 | -1.166782729 | -0.2889284 | 0.6528684 | 1.725422 | 1.252898236 |
| 1540 | | Mitogen-activated kinase kinase kinase | Q75PK5 | 890 | 1.30E-87 | 0.00051677871 | -4.058262428 | -5.263492082 | -4.3302126 | -2.7614137 | -1.4198642 | -1.724904668 |
| 7665 | | CBL-interacting protein kinase CIPK25 | Q8W1D5 | 240 | 4.10E-18 | 0.00061641020 | -0.000447233 | 0.622319785 | 0.53988826 | 1.76913819 | 2.22262239 | 2.193613747 |
| 6151 | | Leucine-rich repeat, plant specific 0 | Q1SXM4 | 1072 | 6.20E-107 | 0.00230557197 | 1.321229707 | 0.629648745 | 1.38392509 | 2.17377777 | 2.58053081 | 2.430626346 |
| 1548 | | CBL-interacting serine/threonine-protein kinase | 022932 | 138 | 4.30E-07 | 0.00343439532 | -3.044771782 | -1.674334339 | -2.5114731 | -1.1648593 | 1.1803253 | 0.523219308 |
| 5208 | | Leucine-rich repeat transmembrane protein kinase | Q8H811 | 667 | 5.40E-64 | 0.00370925536 | 2.462395879 | 1.826812718 | 2.66852368 | 2.93375309 | 1.99252533 | 0.418872438 |
| 6577 | | Mitogen-activated protein kinase | Q9XF36 | 388 | 4.70E-34 | 0.00611582320 | -0.729476752 | -0.754333298 | -0.9349012 | 0.23604523 | 0.86833241 | 0.848535648 |
| 9744 | | Leucine rich repeat protein | Q708X5 | 1020 | 1.90E-101 | 0.02065714051 | 2.394299664 | 2.148665382 | 2.79440861 | 2.92747957 | 2.25548424 | 0.156208244 |
| 7559 | | Receptor kinase-like protein | 049575 | 237 | 3.60E-17 | 0.02441442744 | -1.881342838 | -2.634214786 | -1.0978411 | -0.3195782 | 1.02261694 | 0.416100109 |
| 7857 | | Putative serine/threonine kinase | Q5SNF8 | 671 | 1.80E-64 | 0.04044238500 | 0.415503432 | 0.904732068 | 0.32780683 | 1.04013766 | 1.2718281 | 0.908040075 |
| 5978 | | Serine/threonine kinase SNFL1 | Q2RAX3 | 142 | 1.70E-07 | 0.04234063126 | -4.348061092 | -5.279456741 | -4.0864951 | -2.5605839 | -2.9240658 | -3.079336238 |

| **Metabolic enzymes** | | | | | | | | | | | | |
|---|---|---|---|---|---|---|---|---|---|---|---|---|
| 1086 | | Cytochrome P450 | 022162 | 164 | 8.3E-10 | 0.00000000000 | 3.032389932 | 3.010980712 | 3.72568672 | 5.07705759 | 3.34901625 | 0.069113337 |
| 8773 | | Xyloglucan endotransglucosylas e/hydrolase | Q5MB21 | 793 | 2.30E-77 | 0.00000000000 | -1.524337731 | -0.616604946 | -0.7629837 | 3.59252475 | 4.13362118 | 2.410904398 |
| 8785 | | Xyloglucan endotransglucosylas e/hydrolase protein | Q41638 | 1494 | 1.20E-151 | 0.00000000050 | -1.733967316 | -2.421812374 | -0.7958047 | 4.21693551 | 4.5050502 | 3.875360842 |
| 442 | | Cytochrome P450 | Q9FVS9 | 171 | 1.40E-10 | 0.00000000196 | -1.564049159 | -1.953762835 | -1.0228647 | 1.79499032 | 1.4441724 | 1.643467933 |
| 3866 | | Cytochrome P450 | 023066 | 204 | 4.30E-14 | 0.00000000239 | 2.809032052 | 2.318751193 | 3.4089673 | 4.40614768 | 3.02263006 | -0.17177749 |
| 3865 | | Cytochrome P450 | 023066 | 308 | 2.20E-25 | 0.00000000947 | 1.729701989 | 1.053334202 | 2.55114231 | 4.04673996 | 2.33898502 | -1.367957575 |
| 5268 | | Cytochrome P450 | 022189 | 432 | 4.40E-39 | 0.00000002657 | -0.171897256 | -0.895425496 | -0.9756393 | 1.48592865 | 2.94409851 | 3.016705623 |
| 2277 | | Sucrose synthase | P13708 | 1553 | 6.60E-158 | 0.00000027056 | -0.439437527 | -0.069079036 | 0.8503561 | 3.08989436 | 3.43768373 | 3.154857927 |
| 2764 | | Fructose-bisphosphate aldolase | Q9LF98 | 697 | 3.30E-67 | 0.00000127072 | -3.315564537 | -3.396709151 | -2.034645 | 0.2854297 | -0.0060413 | -1.893972014 |
| 7225 | | 4-coumarate--CoA ligase | 024145 | 135 | 1.30E-06 | 0.00001170707 | -3.392807621 | -2.57339549 | -2.8833741 | -0.1931861 | 1.58348073 | 1.056469759 |

| Probe number | Name | Annotation | UniRef90 | Score | E-value | p.val | Stage 1 | Stage 2 | Stage 3 | Stage 4 | Stage 5 | Stage 6 |
|---|---|---|---|---|---|---|---|---|---|---|---|---|
| 4449 | | Glycerol-3-phosphate acyltransferase 6 | 080437 | 310 | 7.70E-26 | 0.00001425513 | 2.249919466 | 2.469572224 | 2.19032326 | 3.58108203 | 3.48368982 | 3.088203474 |
| 3557 | | Cytochrome P450 | Q8W228 | 247 | 8.70E-19 | 0.00003884093 | -5.002555419 | -5.279456741 | -5.196866 | -3.0211567 | -0.1919588 | -1.790765445 |
| 3607 | | NAD-dependent epimerase/dehydrata se | Q1 S9W4 | 709 | 1.80E-68 | 0.00011426118 | 0.661266219 | 0.023141031 | 1.20854474 | 2.43590222 | 2.40155015 | 1.724341354 |
| 315 | | Glycerol-3-phosphate acyltransferase | 080437 | 799 | 5.20E-78 | 0.00013567488 | 2.695211624 | 1.986341153 | 2.82178878 | 3.74825362 | 3.81957336 | 3.694984355 |
| 8293 | | Cytochrome P450 | Q2LAK8 | 685 | 6.40E-66 | 0.00017785569 | -0.480274815 | -1.159496037 | 0.41613278 | 1.32346335 | 0.04458868 | -3.130314459 |
| 9630 | | Cytochrome P450 | P24465 | 442 | 3.60E-40 | 0.00033344908 | -5.002555419 | -5.279456741 | -5.196866 | -2.1967108 | -0.4141071 | 0.336193208 |
| 8906 | | Arginine decarboxylase | Q43075 | 716 | 3.40E-69 | 0.00051500050 | 1.019505613 | 0.765260943 | 1.34196687 | 2.08934466 | 2.50717259 | 2.105115071 |
| 8011 | | Anthranilate synthase | Q42565 | 583 | 4.20E-55 | 0.00065694455 | -0.193649947 | -0.452886551 | -0.1278975 | 1.34583088 | 2.92075743 | 2.65634125 |
| 4297 | | UDP-N-acetylglucosamine pyrophosphorylase | 074933 | 260 | 2.80E-20 | 0.00079958809 | 0.503440233 | -0.178374591 | 0.3246836 | 1.03722059 | 1.22365096 | 0.319679224 |
| 3297 | | Sterol 24-C-methyltransferase (EC 2.1.1.41) | 014321 | 438 | 9.90E-40 | 0.00084196480 | 2.769429323 | 2.441382945 | 2.77251583 | 3.41873174 | 3.18208003 | 2.644645047 |
| 9491 | | Sucrose synthase | 024301 | 137 | 1.30E-06 | 0.00113919317 | -1.217596254 | -0.830100863 | -1.5572254 | 0.92994132 | 1.42975146 | 0.612576299 |
| 8599 | | Glycerol-3-phosphate acyltransferase | 080437 | 170 | 2.00E-10 | 0.00258638457 | -3.027410375 | -4.5486423 | -4.5633539 | -3.4820752 | -3.4779842 | -3.932050425 |
| 8188 | | GDP-mannose 4,6 dehydratase | Q9SNY3 | 752 | 4.70E-73 | 0.00639818172 | 2.550950715 | 1.697564241 | 2.33211714 | 2.67583385 | 2.00184201 | 1.094084539 |
| 2789 | | Dihydroorotate dehydrogenase | 027281 | 195 | 6.30E-14 | 0.00736459498 | -1.800621337 | -2.291634612 | -1.6564933 | -0.5080053 | -0.8784721 | -0.753250448 |
| 7831 | | UDP-glucose 6-dehydrogenase | 002373 | 187 | 2.50E-12 | 0.01011821623 | 1.899318041 | 1.497757307 | 2.28617602 | 2.8570059 | 1.84726514 | 1.331998693 |
| 6825 | | Caffeoyl-CoA O-methyltransferase related cluster evalue= score= | 004854 | 387 | 2.30E-34 | 0.01267445737 | -1.252462091 | -1.323023707 | -1.6345192 | -1.0042278 | -0.4719877 | -1.166979093 |
| 1694 | | GDP-mannose pyrophosphorylase | Q9C5B8 | 613 | 2.70E-58 | 0.01348169710 | -1.24934575 | -1.393697699 | -0.699937 | -0.1622729 | -0.5484718 | -1.141943185 |
| 10512 | | Thioredoxin reductase | Q39242 | 618 | 7.10E-59 | 0.02252231919 | 1.334730579 | 0.762940036 | 0.98806079 | 1.41232523 | 1.62216657 | 1.408585365 |
| 5993 | | Fatty acid | Q1SBJ5 | 641 | 3.00E-61 | 0.02531588900 | -0.467611935 | -0.754191298 | -0.4362309 | 0.51933886 | 0.87353021 | 1.031540515 |
| | | desaturase | | | | | | | | | | |
| 3378 | | Glutamate dehydrogenase | Q5F2M8 | 777 | 1.10E-75 | 0.03474465899 | -1.334105071 | -1.480148306 | -0.8723308 | -0.1603482 | -0.5571784 | -0.806093582 |
| 1511 | | UDP-glucose 6-dehydrogenase | Q96558 | 882 | 8.60E-87 | 0.03706085573 | -4.370424953 | -5.039749969 | -4.687599 | -3.652005 | -2.5296887 | -3.365775054 |
| 6160 | | Cytochrome P450 | 048786 | 102 | 5.00E-06 | 0.03879776470 | 0.167895497 | -0.489998008 | 0.31509902 | 0.8897543 | 1.86859301 | 1.426445395 |
| 10354 | | Cinnamyl alcohol dehydrogenase | 004079 | 182 | 3.00E-12 | 0.04097855995 | 1.553486831 | 1.070663683 | 2.18261759 | 2.76160348 | 3.4288268 | 2.844747578 |
| 5660 | | Cellulose synthase | Q2IB40 | 858 | 3.10E-84 | 0.04257005140 | 0.967276576 | 0.397490386 | -0.3441734 | 1.08109772 | 0.0351023 | -0.133560091 |
| 8034 | | Glutathione S-transferase | P32110 | 289 | 5.60E-24 | 0.04826837621 | -4.477751826 | -4.680896575 | -4.7276831 | -2.6555421 | -3,4032561 | -2.876803019 |

**Table 3**

| **Probe number** | **Name** | **UniRef90** | **Annotation** | **fold increase** | **E-value** | **Score** | **ptab** |
|---|---|---|---|---|---|---|---|
| **Flavonoid pathway enzymes** | | | | | | | |
| 4090 | *TrUFGT4* | Q1RXH1 | UDP-glucuronosyl/UDP-glucosyltransferase # | 8.28 | 6.30E-72 | 491 | 0.00 |
| 4093 | *TrGT12* | 004114 | Flavonoid 3-O-glucosyltransferase | 7.59 | 3.20E-18 | 257 | 0.05 |
| 5084 | *TrIF3*'*H* | Q6WNQ9 | Isoflavone 3'-hydroxylase | 3.97 | 5.90E-59 | 618 | 0.00 |
| 9326 | | Q40316 | Vestitone reductase | 2.51 | 1.60E-161 | 1587 | 0.01 |
| 10572 | *TrCHS9* | P51090 | Chalcone synthase # | 2.21 | 1.30E-72 | 748 | 0.02 |
| 476 | *TrDFRL5* | Q653W0 | Dihydroflavonol-4-reductase | 2.20 | 1.50E-43 | 474 | 0.05 |
| 7860 | *TrCHS11* | Q1S1C0 | Chalcone synthases # | 2.14 | 8.70E-07 | 135 | 0.03 |
| 10073 | *TrCHR1* | Q41399 | Chalcone reductase | 2.08 | 4.20E-80 | 821 | 0.01 |
| 8037 | *TrGST5* | 004874 | Glutathione S-transferase | 1.97 | 3.00E-21 | 263 | 0.02 |
| 10571 | *TrCHS5* | P17957 | Chalcone synthase 2 * | 1.93 | 3.60E-88 | 895 | 0.03 |
| 3588 | *TrDFRL2* | Q6TQT0 | Dihydroflavonal-4-reductase 2 * | 1.83 | 4.60E-145 | 1432 | 0.00 |
| 3311 | *TrCytB5-1* | Q9M5B0 | Cytochrome b5 DIF-F * | 1.81 | 1.50E-37 | 417 | 0.01 |
| 4096 | *TrGT11* | 022183 | Glucosyltransferase | 1.79 | 3.30E-09 | 136 | 0.04 |
| 10563 | *TrCHS2* | P17957 | Chalcone synthase * | 1.75 | 1.60E-174 | 1710 | 0.01 |
| 2320 | *TrANSL1* | Q2TUV8 | Anthocyanidin synthase 3 * | 1.73 | 1.20E-135 | 1343 | 0.00 |
| 10644 | *TrART1* | Q8S342 | Anthocyanidin rhamnosyl-transferase * | 1.73 | 1.70E-65 | 681 | 0.01 |
| 10564 | *TrCHS6* | P30081 | Chalcone synthase * | 1.67 | 4.40E-170 | 1668 | 0.02 |
| 9976 | *TrIFOMT2* | 022308 | Isoflavone-7-O-methytransferase 6 # | 1.66 | 1.90E-34 | 388 | 0.04 |
| 6625 | *TrGT10* | Q8S9A6 | Glucosyltransferase-3 | 1.57 | 2.60E-46 | 500 | 0.01 |
| 10569 | *TrCHS10* | Q2HZ40 | Chalcone synthase # | 1.53 | 5.10E-85 | 865 | 0.03 |
| 7303 | *TrDFRL3* | P73212 | Dihydroflavonol-4-reductase # | 1.52 | 3.80E-12 | 152 | 0.02 |
| 5323 | *TrCytB5-3* | Q1SHH9 | Cytochrome b5 | 1.51 | 9.50E-45 | 485 | 0.03 |
| 9456 | *TrOMT5* | Q1SBL8 | Methyltransferase # | 1.51 | 2.70E-49 | 530 | 0.01 |
| 6634 | *TrF3H2* | Q9M547 | Flavanoid 3-hydroxylase # | 1.49 | 5.70E-63 | 659 | 0.01 |
| 9065 | *TrGST6* | 004437 | Glutathione S-transferase | 1.44 | 9.10E-19 | 134 | 0.02 |
| 818 | *TrOMT6* | 027940 | Methyltransferase | 1.41 | 1.20E-23 | 286 | 0.01 |
| 1186 | *TrCHI-2* | 022604 | Chalcone isomerase * | 1.35 | 7.80E-13 | 184 | 0.02 |
| 627 | *TrGT13* | 004253 | Glucosyl transferase | 1.31 | 1.80E-67 | 699 | 0.04 |
| 683 | *TrANAT4* | 004201 | Anthocyanin 5-aromatic acyltransferase | 1.27 | 1.10E-10 | 172 | 0.04 |
| 10411 | *TrAAT1* | Q1SBS4 | Anthocyanin acyltransferase | 1.24 | 1.50E-12 | 189 | 0.01 |

| **Transcriptional factors** | | | | | | | |
|---|---|---|---|---|---|---|---|
| 10214 | *TrWDR5* | 014053 | WD-repeat protein | 5.00 | 4.70E-14 | 154 | 0.00 |
| 2153 | *TrMYB2* | Q4JL84 | Transcription factor MYB59 r * | 4.57 | 2.40E-07 | 134 | 0.00 |
| 9046 | *TrMYB10* | P92973 | CCA1 (MYB-related transcription factor) | 2.98 | 9.20E-18 | 148 | 0.00 |
| 4672 | | Q1RSB1 | Nucleic acid-binding, OB-fold | 2.44 | 4.90E-06 | 130 | 0.03 |
| 8791 | *TrMYB11* | Q56TL1 | Late elongated hypocotyl (MYB-related transcription factor) | 2.41 | 8.20E-35 | 398 | 0.00 |
| 133 | | Q1S281 | AP2 domain | 2.19 | 6.20E-55 | 583 | 0.03 |
| 1843 | | 013381 | CCAAT-binding transcription factor subunit AAB-1 | 1.99 | 4.30E-10 | 160 | 0.00 |
| 6820 | | P93015 | Squamosa promoter-binding-like protein 3 * | 1.79 | 2.10E-08 | 141 | 0.00 |
| 5756 | *TrMYB6* | Q9LX82 | Transcription factor MYB48 # | 1.71 | 1.70E-46 | 351 | 0.01 |
| 5256 | | Q1S049 | Zinc finger, LRP1-type | 1.66 | 3.20E-11 | 168 | 0.00 |
| 350 | *TrMYC2* | Q71SQ1 | MYC1 # | 1.50 | 6.90E-06 | 125 | 0.02 |
| 8463 | | P93356 | LIM-domain SF3 protein | 1.48 | 5.00E-67 | 697 | 0.02 |
| 1068 | *TrbHLH3* | 049687 | BHLH protein-like | 1.44 | 1.60E-23 | 293 | 0.03 |
| 8253 | | 022800 | Zinc finger protein CONSTANS-LIKE 14 # | 1.39 | 5.00E-07 | 139 | 0.04 |
| 4631 | *TrMYB3* | Q70RD0 | MYB10 protein # | 1.39 | 5.00E-46 | 499 | 0.05 |
| 4182 | *TrMYB9* | Q69LP9 | Myb-related transcription factor-like | 1.37 | 3.00E-06 | 104 | 0.02 |
| 2965 | | Q5MAR7 | Transcription factor DREBIII-1 | 1.37 | 1.10E-32 | 369 | 0.01 |
| 3248 | | P32583 | Suppressor protein SRP40 | 1.27 | 7.00E-06 | 135 | 0.05 |
| 1591 | *TrWDR6* | 022467 | WD-40 repeat protein MSI1 | 1.18 | 5.90E-19 | 246 | 0.04 |

| **Protein-protein interaction/Protein stability** | | | | | | | |
|---|---|---|---|---|---|---|---|
| 10475 | | Q1S6E0 | Ubiquitin system component Cue; UBA-like | 2.58 | 2.90E-22 | 275 | 0.01 |
| 1927 | | Q5N7R4 | RING-H2 finger protein RHG1a-like * | 1.99 | 2.20E-09 | 159 | 0.01 |
| 1500 | | Q1RYP6 | Zinc finger, SWIM-type | 1.98 | 5.20E-06 | 137 | 0.01 |
| 6955 | | Q2HRJ4 | Zinc finger, RING-type * | 1.92 | 9.90E-51 | 542 | 0.04 |
| 7815 | | Q1PCS0 | GRAS1 * | 1.82 | 1.30E-06 | 137 | 0.04 |
| 5002 | | 004544 | F20P5.26 protein | 1.82 | 2.30E-34 | 389 | 0.02 |
| 119 | | Q9MOW3 | Polyubiquitin related | 1.76 | 2.80E-153 | 1468 | 0.00 |
| 8801 | | Q1SN88 | Zinc finger, RING-type | 1.72 | 3.60E-07 | 135 | 0.01 |
| 3632 | | Q5JNB8 | Zinc finger protein-like | 1.71 | 5.10E-08 | 140 | 0.02 |
| 87 | | 023759 | Ubiquitin-like protein | 1.68 | 2.80E-43 | 432 | 0.00 |
| 10019 | | 004177 | Zinc-finger protein BcZFP1 | 1.58 | 5.10E-12 | 178 | 0.02 |
| 5332 | | Q2QQX2 | DHHC zinc finger domain, putative | 1.54 | 1.30E-24 | 297 | 0.01 |
| 4025 | | Q9M0W3 | Polyubiquitin | 1.53 | 1.70E-145 | 1438 | 0.00 |
| 9998 | | Q6K6A4 | Putative NADH dehydrogenase | 1.53 | 5.20E-45 | 490 | 0.02 |
| 7774 | | Q8W2X7 | Ubiquitin-conjugating enzyme, putative | 1.51 | 3.20E-78 | 803 | 0.01 |
| 484 | | Q3MIH3 | Ubiquitin and ribosomal protein L40 | 1.50 | 4.70E-54 | 574 | 0.02 |
| 3411 | | 075380 | NADH-ubiquinone oxidoreductase 13 kDa-A subunit | 1.49 | 5.40E-07 | 131 | 0.05 |
| 871 | | P35133 | Ubiquitin-conjugating enzyme E2-17 kDa | 1.48 | 2.60E-73 | 756 | 0.00 |
| 6835 | | Q1SGE4 | Zinc finger, AN1-type; Zinc finger, A20-type | 1.48 | 5.80E-13 | 186 | 0.03 |
| 1753 | | Q4KTB1 | S30-ubiquitin-like | 1.44 | 3.90E-12 | 177 | 0.04 |
| 600 | | 094650 | Ubiquitin-like modifier hub1 | 1.42 | 1.10E-22 | 279 | 0.03 |
| 6677 | | Q1SRY4 | Zinc finger, RING-type | 1.42 | 7.20E-85 | 824 | 0.03 |
| 5055 | | Q42541 | Ubiquitin-conjugating enzyme E2 13 | 1.41 | 4.80E-70 | 726 | 0.03 |
| 8775 | | 076080 | Zinc finger A20 domain-containing protein | 1.39 | 6.80E-08 | 96 | 0.04 |
| 2453 | | 023759 | Ubiquitin-like protein | 1.31 | 4.40E-43 | 436 | 0.02 |
| 1346 | | P90789 | Probable NADH dehydrogenase [ubiquinone] 1 | 1.30 | 2.60E-06 | 124 | 0.01 |
| 1947 | | P93028 | Ubiquitin activating enzyme 1 | 1.28 | 1.90E-78 | 805 | 0.02 |
| 1792 | | 082353 | RING-H2 finger protein ATL2M | 1.27 | 3.90E-12 | 179 | 0.01 |
| 6090 | | P34670 | Putative zinc finger protein | 1.26 | 7.90E-15 | 210 | 0.01 |
| 259 | | Q4N6V0 | Ubiquitin/ribosomal fusion protein, putative | 1.24 | 1.80E-52 | 558 | 0.01 |

| **Auxin biosynthesis/Signal transduction** | | | | | | | |
|---|---|---|---|---|---|---|---|
| 4800 | | UQ69LK3 | Auxin-induced protein-like | 3.94 | 2.20E-13 | 126 | 0.01 |
| 3469 | | 022150 | Putative auxin-regulated protein # | 3.53 | 8.40E-10 | 158 | 0.00 |
| 589 | | 022150 | Putative auxin-regulated protein | 1.49 | 4.40E-10 | 160 | 0.01 |

| **Transporters** | | | | | | | |
|---|---|---|---|---|---|---|---|
| 1559 | | 022110 | Lipid transfer protein * | 4.10 | 9.80E-06 | 119 | 0.01 |
| 5497 | | 022110 | Lipid transfer protein | 2.14 | 1.40E-07 | 135 | 0.04 |
| 73 | | Q506K0 | Putative aquaporin | 1.82 | 2.00E-97 | 984 | 0.00 |
| 1822 | | Q9M6B7 | Lipid transfer protein precursor # | 1.68 | 1.10E-34 | 392 | 0.04 |
| 6864 | | P36095 | Vacuolar protein sorting protein 24 | 1.67 | 1.90E-18 | 237 | 0.04 |
| 6565 | | 022925 | Vacuolar sorting receptor 2 precursor | 1.62 | 1.20E-37 | 422 | 0.02 |
| 8128 | | 023429 | Vesicle-associated membrane protein 724 | 1.60 | 1.20E-10 | 165 | 0.01 |
| 528 | | P47111 | Vacuolar protein sorting protein 55 | 1.56 | 8.40E-09 | 148 | 0.00 |
| 10854 | | 000476 | Sodium-dependent phosphate transport protein 4 | 1.46 | 1.70E-16 | 180 | 0.00 |
| 4018 | | 049838 | Sucrose transporter | 1.41 | 5.70E-24 | 297 | 0.00 |
| 2733 | | Q4VWF0 | Histidine-containing phosphotransfer protein | 1.37 | 1.50E-18 | 240 | 0.00 |
| 5266 | | Q1SU64 | Plant lipid transfer protein/Par allergen | 1.35 | 1.30E-47 | 514 | 0.04 |
| 5676 | | P98204 | Phospholipid-transporting ATPase 1 | 1.26 | 8.40E-48 | 509 | 0.03 |

| **Transcription/Translation** | | | | | | | |
|---|---|---|---|---|---|---|---|
| 2815 | | 059835 | DNA polymerase delta subunit 4 | 1.87 | 1.90E-07 | 135 | 0.00 |
| 2117 | | Q1S1M5 | Histone deacetylase 2a | 1.72 | 2.80E-11 | 174 | 0.00 |
| 5305 | | Q1STG3 | RNA-binding region RNP-1 | 1.66 | 1.10E-47 | 472 | 0.00 |
| 6069 | | 065759 | Histone H2A | 1.64 | 9.00E-42 | 459 | 0.00 |
| 4428 | | Q7G8Y3 | Putative chromatin remodelling complex | 1.44 | 2.00E-15 | 221 | 0.01 |
| 7423 | | Q06835 | Pre-mRNA-splicing factor RDS3 | 1.43 | 3.80E-29 | 340 | 0.01 |
| 9861 | | Q25BM1 | RNA recognition motif containing protein | 1.33 | 2.40E-43 | 474 | 0.04 |
| 4818 | | Q1S0C5 | RNA recognition motif putative | 1.31 | 2.30E-15 | 222 | 0.05 |
| 10559 | | 024591 | Histone deacetylase 2a | 1.28 | 3.80E-20 | 190 | 0.00 |

| **Cell signalling** | | | | | | | |
|---|---|---|---|---|---|---|---|
| 8503 | | 004388 | A-type cyclin | 3.19 | 3.20E-17 | 230 | 0.00 |
| 1910 | | 022100 | ATMRK1 * | 2.32 | 6.90E-30 | 347 | 0.03 |
| 6989 | Q6UY58 | Q6UY58 | Lectin-like receptor kinase | 2.22 | 4.30E-85 | 868 | 0.01 |
| 2936 | | Q8LKU7 | Putative serine/ threonine kinase | 1.85 | 4.10E-10 | 150 | 0.03 |
| 10037 | | Q1T3X4 | Prefoldin; t-snare; Protein kinase PKN/PRK1 | 1.67 | 1.00E-66 | 708 | 0.04 |
| 4113 | | Q1RY61 | Protein kinase | 1.67 | 2.30E-20 | 263 | 0.02 |
| 7399 | | 082458 | Rac GTPase activating protein | 1.56 | 3.60E-24 | 297 | 0.05 |
| 5209 | | 022932 | CBL-interacting serine/threonine-protein kinase 11 | 1.55 | 2.50E-33 | 379 | 0.02 |
| 9770 | | 082458 | Rac GTPase activating protein 1 | 1.52 | 2.10E-13 | 199 | 0.04 |
| 2943 | | 004098 | Receptor-kinase isolog, 5' partial * | 1.51 | 4.30E-06 | 133 | 0.03 |
| 2349 | | 023249 | Cks1 protein | 1.44 | 2.80E-35 | 398 | 0.00 |
| 6940 | | Q8LES3 | Protein kinase | 1.42 | 1.70E-877 | 890 | 0.01 |
| 6663 | | 022178 | Putative receptor-like protein kinase | 1.43 | 9.80E-63 | 657 | 0.03 |
| 3913 | | Q5NBP9 | Protein kinase C substrate 80K-H isoform 2-like | 1.41 | 1.40E-64 | 673 | 0.03 |
| 979 | | Q70AH8 | Receptor-like kinase with LRR repeats | 1.39 | 1.90E-09 | 153 | 0.02 |
| 7805 | | Q69SP5 | Putative receptor protein kinase | 1.33 | 4.30E-10 | 173 | 0.01 |
| 7269 | | P43289 | Shaggy-related protein kinase gamma | 1.32 | 5.40E-167 | 1641 | 0.04 |
| 1323 | | P92958 | SNF1-related protein kinase | 1.32 | 5.00E-28 | 332 | 0.02 |
| 9834 | | Q6YW44 | Putative MAP3K delta-1 protein kinase | 1.30 | 3.20E-73 | 755 | 0.02 |
| 2514 | | Q1SH98 | Protein kinase | 1.26 | 3.00E-19 | 253 | 0.03 |
| 908 | | Q09749 | ADIPOR-like receptor | 1.22 | 7.80E-14 | 197 | 0.02 |
| 9016 | | P43293 | Probable serine/threonine-protein kinase NAK | 1.15 | 4.70E-07 | 137 | 0.02 |

| **Metabolic enzymes** | | | | | | | |
|---|---|---|---|---|---|---|---|
| 7720 | | Q8W228 | Cytochrome P450 r | 7.11 | 1.20E-43 | 477 | 0.01 |
| 2755 | | P46257 | Fructose-bisphosphate aldolase | 3.22 | 4.60E-88 | 896 | 0.01 |
| 9410 | | Q93XM0 | Xyloglucan endo-transglycosylase | 3.05 | 2.80E-54 | 577 | 0.00 |
| 6211 | | P38419 | Lipoxygenase | 2.66 | 1.70E-07 | 148 | 0.01 |
| 1112 | | Q1SNH9 | 2OG-Fe(II) oxygenase # | 2.24 | 2.30E-176 | 1729 | 0.00 |
| 3494 | | Q1T4K0 | Aldehyde dehydrogenase | 2.09 | 5.00E-68 | 707 | 0.04 |
| 2375 | | 023920 | 4-hydroxyphenylpyruvate dioxygenase | 1.89 | 3.80E-24 | 294 | 0.00 |
| 8530 | | 016924 | Asparaginyl trna synthetase protein 2 | 1.77 | 5.00E-19 | 252 | 0.00 |
| 2174 | | 022340 | Limonene synthase | 1.74 | 5.00E-08 | 151 | 0.00 |
| 781 | | 002773 | Mannosyl-oligosaccharide 1,2-alpha-mannosidase IA | 1.72 | 3.60E-15 | 218 | 0.00 |
| 10663 | | 023255 | Adenosylhomocysteinase 1 | 1.67 | 4.50E-73 | 754 | 0.00 |
| 160 | | 068975 | Exopolygalacturonase | 1.55 | 5.40E-07 | 141 | 0.01 |
| 5806 | | Q2IJL2 | Peptidylprolyl isomerase, FKBP-type | 1.53 | 7.70E-06 | 81 | 0.00 |
| 10736 | | P80969 | Tyramine N-feruloyltransferase 10/30 | 1.51 | 6.60E-17 | 224 | 0.01 |
| 10159 | | Q1S3A6 | Aldo/keto reductase | 1.48 | 2.60E-108 | 1087 | 0.00 |
| 10125 | | 022769 | NADH-ubiquinone oxidoreductase 24 kDa subuni | 1.47 | 2.80E-36 | 407 | 0.03 |
| 7901 | | 022162 | Putative cytochrome P450 | 1.45 | 4.70E-09 | 159 | 0.01 |
| 9068 | | 022631 | ADP-glucose pyrophosphorylase large subunit | 1.45 | 3.30E-14 | 207 | 0.00 |
| 8049 | | Q1RYZ6 | Glycoside hydrolase | 1.41 | 8.00E-45 | 488 | 0.00 |
| 676 | | Q6ZDX2 | Putative pectinesterase | 1.36 | 6.60E-122 | 957 | 0.00 |
| 6078 | | Q9SQI7 | Dihydrolipoamide S-acetyltransferase | 1.33 | 1.30E-74 | 769 | 0.01 |
| 1911 | | 048661 | Spermidine synthase 2 | 1.32 | 4.30E-51 | 547 | 0.02 |
| 7814 | | 023051 | Ent-kaurenoic acid oxidase 1 | 1.29 | 1.30E-12 | 192 | 0.04 |
| 197 | | P37216 | Phospho-2-dehydro-3-deoxyheptonate aldolase 2 | 1.28 | 5.50E-89 | 581 | 0.01 |
| 7076 | | Q944G3 | Acetyl Co-A acetyltransferase | 1.37 | 3.00E-22 | 206 | 0.02 |

**Table 4**

| **Probe number** | **Name** | **UniRef90** | **Annotation** | **Fold reduction** | **E-value** | **Score** | **ptab** |
|---|---|---|---|---|---|---|---|
| **Flavonoid pathway enzymes** | | | | | | | |
| 2895 | *TrlFR1* | O48601 | NADPH:isoflavone reductase # | 24.15 | 3.60E-50 | 536 | 4.08E-05 |
| 2585 | *TrANR* | Q84XT1 | Anthocyanidin reductase* | 3.79 | 1.80E-114 | 1143 | 5.65E-05 |
| 3886 | *Tr*/*FOMT1* | O22308 | Isoflavone-7-O-methytransferase 6 # | 3.67 | 1.50E-27 | 323 | 1.08E-03 |
| 8985 | *TrANAT3* | O04201 | Putative anthocyanin 5-aromatic acyltransferase | 3.34 | 7.90E-10 | 164 | 1.10E-05 |
| 4495 | *TrUFGT6* | O24341 | UDP-glucose glucosyltransferase | 2.96 | 4.50E-09 | 157 | 3.70E-03 |
| 3922 | *TrF3'H1* | Q2PEY1 | Putative flavonoid 3'-hydroxylase # | 2.47 | 5.00E-84 | 835 | 2.88E-02 |
| 1511 | | Q96558 | UDP-glucose 6-dehydrogenase # | 2.34 | 8.60E-87 | 882 | 6.10E-03 |
| 10081 | *TrGT7* | O04114 | Flavonoid 3-O-glucosyltransferase # | 1.88 | 6.80E-18 | 237 | 6.05E-03 |
| 7304 | *TrDFR4* | Q9C6L6 | 4-reductase # | 1.73 | 5.90E-58 | 609 | 3.61E-02 |
| 4847 | *TrOMT1* | Q1S0J0 | Generic methyltransferase * | 1.51 | 1.50E-06 | 134 | 8.63E-03 |

| **Transcriptional factors** | | | | | | | |
|---|---|---|---|---|---|---|---|
| 2998 | *TrWDR7* | Q3MV14 | WD repeat protein | 3.58 | 4.30E-20 | 253 | 4.39E-02 |
| 6567 | | Q1SSY4 | Transcription factor E2F/dimerisation partner | 2.11 | 3.80E-82 | 838 | 5.81E-03 |
| 4051 | | O48885 | CONSTANS homolog | 2.05 | 5.70E-10 | 162 | 8.18E-05 |
| 1403 | | Q1SLX9 | Zinc finger, Dof-type related | 1.98 | 3.00E-07 | 140 | 2.60E-02 |
| 6269 | | O13282 | Transcription initiation factor TFIID | 1.79 | 6.60E-11 | 176 | 4.22E-02 |
| 754 | | O24456 | Guanine nucleotide-binding protein beta subunit-like protein | 1.76 | 1.50E-06 | 131 | 7.18E-03 |
| 572 | | O82199 | Putative CCCH-type zinc finger protein | 1.70 | 2.00E-23 | 283 | 3.43E-02 |
| 6766 | | Q1SH85 | Helix-loop-helix DNA-binding related | 1.51 | 3.00E-07 | 99 | 3.79E-02 |
| 9359 | | Q1SGH7 | Zinc finger, RING-type; Thioredoxin-related related | 1.50 | 1.50E-11 | 177 | 1.01E-02 |
| 2942 | | Q1RL87 | Zinc finger protein related | 1.46 | 4.30E-07 | 133 | 1.26E-02 |
| 1828 | | Q2QMN5 | WRKY transcription factor 1 | 1.46 | 1.20E-08 | 150 | 2.39E-02 |
| 3318 | | Q2VY12 | CONSTANS interacting protein 6 | 1.45 | 5.50E-22 | 275 | 1.02E-02 |
| 10276 | | Q9M1G6 | BZIP transcription factor-like protein | 1.45 | 2.80E-06 | 90 | 6.93E-03 |
| 7306 | | Q1SJS5 | Zinc finger, RING-type; RINGv related | 1.41 | 6.00E-28 | 252 | 2.13E-02 |
| 10084 | | Q15296 | Zinc finger, C3HC4 type (RING finger) | 1.40 | 2.80E-39 | 242 | 2.55E-02 |
| 5662 | *TrMYB12* | Q2HTW0 | Myb, DNA-binding | 1.37 | 1.70E-49 | 530 | 4.24E-02 |
| 7508 | | Q9ATD1 | GHMYB9 related | 1.21 | 5.70E-43 | 467 | 4.74E-02 |
| 6971 | | Q2V987 | Transcription factor APFI-like related | 1.20 | 3.40E-69 | 715 | 4.99E-02 |

| **Protein-protein interaction/Protein stability** | | | | | | | |
|---|---|---|---|---|---|---|---|
| 10837 | | O23759 | Ubiquitin-like protein | 2.89 | 7.20E-14 | 194 | 1.37E-02 |
| 5528 | | Q1S2R3 | Gigantea protein | 2.72 | 4.70E-86 | 875 | 1.72E-02 |
| 6057 | | O64438 | ARG10 related | 2.51 | 3.80E-96 | 970 | 1.69E-02 |
| 10045 | | O76080 | Zinc finger A20 domain-containing protein 2 | 2.45 | 5.20E-06 | 121 | 3.21E-02 |
| 7800 | | Q67UM6 | Putative ring finger protein 10 # | 2.16 | 9.80E-19 | 250 | 3.98E-02 |
| 6379 | | Q208P4 | RAMOSUS4 | 2.03 | 1.10E-65 | 683 | 4.09E-02 |
| 7795 | | Q6ASX1 | FYVE zinc finger containing protein | 2.01 | 8.40E-14 | 206 | 4.20E-02 |
| 9043 | | O64762 | Putative RING-H2 finger protein ATL2F | 1.96 | 8.60E-10 | 159 | 2.04E-03 |
| 82 | | O64438 | ARG10 | 1.91 | 2.00E-28 | 331 | 3.45E-02 |
| 10319 | | Q8L7L0 | GTP-binding protein | 1.87 | 3.70E-78 | 800 | 2.55E-02 |
| 7038 | | Q9FLH0 | Putative nuclear matrix constituent protein 1-like | 1.84 | 2.50E-32 | 379 | 3.74E-03 |
| 8824 | | Q1SGE4 | Zinc finger, AN1-type | 1.82 | 1.50E-12 | 180 | 2.40E-02 |
| 8548 | | Q2PF41 | BEL1-like homeodomain transcription factor | 1.79 | 5.30E-08 | 149 | 3.32E-02 |
| 2124 | | O64425 | Putative E3 ubiquitin ligase, RMA1 | 1.71 | 3.40E-20 | 253 | 4.41E-02 |
| 8925 | | Q8W419 | Twin LOV protein 1 | 1.44 | 4.30E-13 | 192 | 3.65E-02 |
| 10241 | | P31252 | Ubiquitin-activating enzyme E1 3 | 1.22 | 4.50E-07 | 105 | 4.03E-02 |
| 2441 | | O22967 | Dof zinc finger protein DOF2.3 | 1.21 | 3.60E-11 | 168 | 2.08E-02 |
| 5930 | | Q8S4W7 | DELLA protein GAI1 | 1.13 | 2.00E-159 | 1334 | 4.36E-02 |

| **Auxin biosynthesis/signal transduction** | | | | | | | |
|---|---|---|---|---|---|---|---|
| 2862 | | O23661 | Auxin response factor 3 | 2.92 | 9.30E-22 | 276 | 4.15E-02 |
| 4224 | | O48629 | Putative auxin-repressed protein | 1.59 | 8.30E-12 | 102 | 3.82E-02 |
| 4860 | | Q1RYA5 | Auxin Efflux Carrier # | 1.51 | 6.20E-10 | 163 | 2.96E-03 |

| **Transporters** | | | | | | | |
|---|---|---|---|---|---|---|---|
| 149 | | Q4PLT5 | Non-specific lipid transfer protein | 3.89 | 1.40E-28 | 333 | 3.50E-03 |
| 2279 | | Q9M390 | Peptide transport-like protein | 2.83 | 5.20E-57 | 601 | 2.37E-02 |
| 3804 | | Q9ZUY2 | Putative membrane transporter related cluster evalue= | 2.50 | 2.30E-11 | 181 | 4.85E-02 |
| 2146 | | O023429 | Vesicle-associated membrane protein 724 | 2.00 | 2.80E-82 | 839 | 1.66E-02 |
| 8632 | | O22397 | Potassium transporter 1 | 1.79 | 1.50E-71 | 738 | 2.74E-02 |
| 7543 | | Q4L224 | Putative plasma membrane Na+/H+ antiporter related | 1.70 | 4.30E-24 | 302 | 1.01E-02 |
| 7875 | | O23213 | Sugar transporter like protein | 1.63 | 5.10E-07 | 138 | 3.51E-02 |
| 2030 | | Q6SL79 | Na+/H+ antiporter | 1.60 | 4.20E-22 | 278 | 3.56E-02 |
| 714 | | O27682 | ABC transporter related | 1.59 | 2.00E-08 | 151 | 1.87E-02 |
| 2790 | | Q9LEG2 | Putative sugar transporter | 1.54 | 7.90E-45 | 485 | 2.21E-02 |
| 2902 | | Q1RUP2 | Mg2+ transporter protein, CorA-like | 1.53 | 2.50E-76 | 757 | 2.25E-02 |
| 6376 | | O22305 | Peptide transporter | 1.48 | 3.30E-20 | 261 | 1.35E-02 |
| 9818 | | Q1T4P5 | Sugar transporter related | 1.46 | 2.20E-20 | 261 | 1.37E-02 |
| 6544 | | Q9XHL6 | Sucrose transport protein SUT1 | 1.43 | 6.10E-68 | 510 | 3.89E-03 |
| 4749 | | Q1RYG7 | Plant lipid transfer protein/Par allergen | 1.42 | 1.80E-13 | 189 | 2.30E-02 |
| 10103 | | O01258 | Vacuolar protein sorting 26 | 1.39 | 4.80E-22 | 271 | 1.23E-02 |
| 271 | | O23758 | Nonspecific lipid-transfer protein precursor | 1.29 | 4.30E-33 | 375 | 6.66E-03 |
| 1060 | | O14787 | Transportin-2 | 1.21 | 1.80E-13 | 201 | 3.18E-02 |

| **Transcription /Translation** | | | | | | | |
|---|---|---|---|---|---|---|---|
| 1973 | | O23467 | Splicing factor SF3a like protein related | 4.28 | 3.00E-16 | 215 | 3.82E-02 |
| 2911 | | O27801 | Ribosomal RNA large subunit methyltransferase J | 1.89 | 1.10E-13 | 192 | 2.12E-02 |
| 1593 | | P23116 | Eukaryotic translation initiation factor 3 subunit 10 | 1.80 | 4.90E-10 | 172 | 4.91E-02 |
| 10682 | | O26361 | Translation initiation factor 2 gamma subunit | 1.76 | 3.20E-08 | 148 | 3.00E-02 |
| 6853 | | O49659 | Translation factor EF-1 alpha - like protein | 1.44 | 8.80E-33 | 371 | 1.74E-02 |

| **Cell signalling** | | | | | | | |
|---|---|---|---|---|---|---|---|
| 1904 | | Q2HV99 | Protein kinase; Type I EGF | 5.38 | 8.90E-48 | 516 | 2.61 E-02 |
| 997 | | Q9LXA4 | Pto kinase interactor-like protein r | 4.91 | | 706 | 5.04E-03 |
| 9038 | | Q1SAX1 | Protein kinase # | 3.00 | 1.20E-55 | 588 | 1.88E-02 |
| 976 | | Q1SW67 | Tyrosine protein kinase, active site | 2.83 | 1.10E-68 | 722 | 5.52E-03 |
| 10346 | | Q1SAI9 | Protein kinase | 2.12 | 1.40E-65 | 684 | 2.06E-02 |
| 3650 | | P46573 | Protein kinase APK1B, chloroplast precursor | 2.02 | 3.20E-50 | 536 | 1.16E-02 |
| 4461 | | 001427 | Aurora/ipl1 related kinase protein # | 1.99 | 4.10E-06 | 126 | 2.66E-03 |
| 1742 | | Q6TKQ5 | Protein kinase-like protein | 1.96 | 8.30E-45 | 486 | 3.09E-02 |
| 4574 | | 014011 | Cell cycle control protein cwf8 related | 1.95 | 9.20E-24 | 291 | 1.31E-02 |
| 10338 | | 023190 | MAP3K-like protein kinase | 1.90 | 7.90E-09 | 158 | 6.00E-03 |
| 2588 | | Q1SNH1 | Protein kinase | 1.89 | 3.00E-50 | 537 | 5.68E-03 |
| 2304 | | Q9LT86 | MAP3K protein kinase-like protein | 1.68 | 5.60E-42 | 458 | 1.52E-02 |
| 2957 | | Q1S3M0 | Protein kinase # | 1.68 | 6.70E-80 | 817 | 4.33E-02 |
| 2211 | | 004375 | Serine/threonine protein phosphatase 2A | 1.67 | 3.90E-06 | 130 | 2.75E-02 |
| 10222 | | 022178 | Putative receptor-like protein kinase | 1.67 | 1.70E-11 | 183 | 3.50E-02 |
| 8321 | | Q1SRR9 | Protein kinase | 1.64 | 4.80E-55 | 582 | 1.66E-02 |
| 9791 | | Q4L0F8 | Protein phosphatase 2c | 1.60 | 2.00E-76 | 742 | 2.66E-02 |
| 8706 | | Q9FHY4 | MAP kinase | 1.59 | 5.40E-06 | 112 | 9.58E-03 |
| 6577 | | Q9XF36 | Mitogen-activated protein kinase # | 1.53 | 4.70E-34 | 388 | 2.67E-03 |
| 543 | | Q2LJM0 | Putative receptor kinase | 1.51 | 1.80E-33 | 385 | 7.15E-04 |
| 8917 | | Q9SZ53 | Protein phosphatase 2C-like protein | 1.51 | 8.10E-36 | 401 | 3.30E-02 |
| 4530 | | Q9M6R8 | MAP kinase PsMAPK2 | 1.45 | 9.40E-74 | 759 | 5.53E-03 |
| 1888 | | Q56YN3 | NAD(H) kinase 1 | 1.45 | 2.50E-66 | 615 | 4.52E-02 |
| 3612 | | Q6V5I0 | Protein kinase related | 1.45 | 1.50E-10 | 167 | 5.78E-03 |
| 8644 | | Q56WD6 | Serine/threonine kinase | 1.43 | 4.70E-54 | 573 | 1.76E-02 |
| 9497 | | Q1SZH0 | Protein kinase | 1.41 | 1.80E-06 | 133 | 1.50E-02 |
| 7606 | | Q6K4T4 | Putative SERK2 protein | 1.40 | 8.00E-75 | 769 | 2.38E-02 |
| 8881 | | Q5ZE73 | Putative calcium-dependent protein kinase | 1.38 | 1.80E-45 | 492 | 9.73E-03 |
| 3884 | | Q1SQ13 | Protein kinase | 1.26 | 5.40E-80 | 818 | 2.07E-02 |

| **Metabolic enzymes** | | | | | | | |
|---|---|---|---|---|---|---|---|
| 5967 | | Q2MJ11 | Cytochrome P450 monooxygenase CYP93A | 5.33 | 1.00E-90 | 919 | 1.46E-03 |
| 1766 | | Q1T6B0 | NAD-dependent epimerase/dehydratase | 4.91 | 7.90E-50 | 532 | 4.31E-04 |
| 3328 | | Q1SDS4 | E-class P450, group I | 2.85 | 1.20E-71 | 739 | 4.91E-02 |
| 3023 | | Q1S0N0 | Nicotianamine synthase | 2.73 | 2.20E-67 | 699 | 6.79E-03 |
| 4772 | | O64896 | Trehalose-6-phosphate phosphatase | 2.49 | 1.50E-06 | 132 | 2.71E-02 |
| 5538 | | O27188 | L-tyrosine decarboxylase | 2.36 | 3.60E-11 | 173 | 2.32E-02 |
| 8979 | | Q655Q3 | Pectin methylesterase PME1-like protein | 2.00 | 1.50E-07 | 139 | 9.28E-03 |
| 9988 | | Q2MIZ8 | Cytochrome P450 monooxygenase CYP711A* | 1.88 | 2.30E-47 | 510 | 1.64E-02 |
| 5993 | | Q1SBJ5 | Fatty acid desaturase # | 1.86 | 3.00E-61 | 641 | 6.20E-03 |
| 4064 | | Q2MIZ2 | Cytochrome P450 monooxygenase CYP93B | 1.74 | 8.80E-55 | 580 | 8.76E-04 |
| *5810* | | Q05047 | Cytochrome P450 | 1.73 | 2.00E-10 | 170 | 1.80E-02 |
| 6510 | | Q33DY0 | Cytochrome P450 | 1.72 | 1.30E-49 | 531 | 2.05E-02 |
| 637 | | O13283 | NAD(P)H-dependent D-xylose reductase | 1.69 | 4.20E-22 | 271 | 2.87E-02 |
| 1874 | | Q56Y11 | Dehydrodolichyl diphosphate synthase 2 | 1.60 | 4.80E-27 | 318 | 2.03E-02 |
| 1398 | | Q4U3T8 | Diacylglycerol acyltransferase DGAT2 | 1.49 | 1.40E-07 | 142 | 3.66E-02 |
| 10380 | | O24081 | Peroxidase1A precursor | 1.43 | 5.50E-83 | 846 | 4.89E-02 |
| 2871 | | Q8LFD1 | Putative lipid phosphate phosphatase 3 | 1.43 | 2.30E-36 | 406 | 2.59E-02 |
| 8987 | | Q9ZRQ2 | 2-oxoglutarate dehydrogenase, E1 subunit | 1.43 | 7.70E-84 | 853 | 8.74E-03 |
| 2457 | | O04807 | Omega-3 fatty acid desaturase | 1.29 | 1.80E-37 | 416 | 5.94E-03 |
| 8592 | | O24145 | 4-coumarate-CoA ligase 1 | 1.28 | 2.30E-12 | 187 | 1.11E-02 |

**Table 5**

| **Gene** | **RT-PCR primers (5'-3')** | |
|---|---|---|
| *TrANR* | Forward | TTGCTACACCTGTGAACTTTGCTT |
| | Reverse | GCAATTGCTTTCAACACATTCAAC |
| *TrLAR* | Forward | ATTGTCATCCATCACAGCTTCCT |
| | Reverse | TGACATCGCCATGACCATAAA |
| *TrCHS10* | Forward | TGTTGAAGTACCAAAGCTAGGTAAAGAA |
| | Reverse | TTTTGGTTGTCCCCATTCACTTA |
| *TrCHS2* | Forward | GGCAAGCATTGTTTGGAGATG |
| | Reverse | ACTTCTGGCAAAGGGTCTGAAC |
| *TrCHS6* | Forward | GGCTGAAAATGGACTTAAAACCA |
| | Reverse | GGCCCAAATCCAAACAACAC |
| *TrCHS7* | Forward | GCGGAAGAAATCGGCTCAA |
| | Reverse | AACACACCCCAATCAAGTCCTT |
| *TrANS1* | Forward | CATGGTGCCAGGTTTGCA |
| | Reverse | CCAGGTACACATTTTGCTGTGA |
| *TrANS3* | Forward | AAGCTAGAGAAACTTGGCGTGAA |
| | Reverse | TTGTTGGAGAATTTGCATATTGTTG |
| *TrANAT3* | Forward | GGTGCGCCGAGGAGTAGAG |
| | Reverse | CCATCAAAATTCCCAAGTGGAA |
| *TrUFGT4* | Forward | CTTTCGGCTGAAGGATTTGC |
| | Reverse | TTCCAACGTGGAATCATTTGAA |
| *TrMYB1* | Forward | TGAATCTTTGGAACCACTAATGGA |
| | Reverse | AAGCAACAACTTGAAGCAAAATCA |
| *TrMYB8* | Forward | TGGCTTCTGATGATCCAGCTT |
| | Reverse | CCGACGCTAGCAGAACGTTT |
| *TrMYB5* | Forward | GCAAAGCATCTTCCAGGAAGA |
| | Reverse | GCTTTTGTATCCTTGTCCTCCAA |
| *TrEFa* | Forward | TCGAGAAGGAAGCTGCTGAAA |
| | Reverse | CCCAGGCATACTTGAATGACCT |
| *TrGAPDH* | Forward | GTTTGGTTGCTAGAGTTGCTTTGA |
| | Reverse | CGGTAGTGATGAAAGGATCGTTAA |
| *TrHIST* | Forward | CAGGAAGCTGCTGAGGCCTAT |
| | Reverse | TAGCATGAATTGCGCACAAGT |
| *TrSAMS* | Forward | GGGTCACATGTTTGGCTATGC |
| | Reverse | GTTGCAAGGACATGGCTCAA |
| *TrUBIQ* | Forward | CGGACCAGCAGCGTCTG |
| | Reverse | GAGGGTGGACTCCTTTTGAATG |

## Claims

1. A method of identifying a gene encoding a polypeptide or polypeptide isoform which is substantially more active in either a proanthocyanidin (PA) or anthocyanin (ANT) pathway of a plant, said method including
providing
material from said plant at two or more developmental stages; and
an oligonucleotide probe capable of hybridizing with RNA from a gene encoding a polypeptide which is active in a flavonoid biosynthetic pathway;
extracting RNA from said plant material;
hybridizing the oligonucleotide probe with the RNA using a microarray to generate an expression profile;
measuring PA and ANT levels in said plant material quantitatively using liquid chromatography mass spectroscopy to generate a metabolic profile;
comparing said expression profile with said metabolite profile using a computer to identify said gene encoding a polypeptide or polypeptide isoform which is substantially more active in either a PA or ANT pathway.

2. A method according to claim 1, wherein the polypeptide or polypeptide isoform is an enzyme which catalyses one of the final reactions in the synthesis of anthocyanins, after the leucoanthocyanidin branch point in the ANT pathway.

3. A method according to claim 2, wherein said polypeptide or polypeptide isoform is selected from the group consisting of GST, G3T, UFGT, OMT, ART, ANAT and AAT.

4. A method according to claim 1, wherein the polypeptide or polypeptide isoform is a transcription factor, preferably wherein said polypeptide or polypeptide isoform is selected from the group consisting of MYB, bHLH, MYC and WDR.

5. A method of manipulating the flavonoid biosynthetic pathway in a plant, said method including identifying a gene encoding a polypeptide or polypeptide isoform which is substantially more active in either a PA or ANT pathway of said plant using a method according to any one of claims 1 to 4, and up- or down-regulating expression of said gene to increase or decrease the level of PA or ANT in said plant.

6. A method according to claim 5, wherein said method includes down-regulating expression of a gene encoding a polypeptide or polypeptide isoform which is an enzyme which catalyses one of the final reactions in the synthesis of anthocyanins, after the leucoanthocyanidin branch point in the ANT pathway.

7. A method according to claim 5, wherein said method includes up- and/or down-regulating expression of one or more genes encoding a transcription factor.

8. A method of enhancing bloat safety of a plant, said method including
identifying a gene encoding a polypeptide or polypeptide isoform which is substantially more active in a PA pathway using a method according to any one of claims 1 to 4, and up-regulating or down-regulating expression of said gene to increase the level of PA in said plant; or
identifying a gene encoding a polypeptide or polypeptide isoform which is substantially more active in an ANT pathway using a method according to any one of claims 1 to 4, and up-regulating or down-regulating expression of said gene to increase the level of PA in said plant.

9. A method according to claim 8, wherein said method includes down-regulating expression of a gene encoding a polypeptide or polypeptide isoform which is an enzyme which catalyses one of the final reactions in the synthesis of anthocyanins, after the leucoanthocyanidin branch point in the ANT pathway.

10. A method according to claim 8, wherein said method includes up- and/or down-regulating expression of one or more genes encoding a transcription factor.

## Patentansprüche

1. Ein Verfahren zum Identifizieren eines Gens, das ein Polypeptid oder eine Polypeptidisoform kodiert, das/die in entweder einem Proanthocyanidin- (PA-) oder Anthocyanin (ANT-) Stoffwechselweg einer Pflanze wesentlich aktiver ist, jenes Verfahren umfassend:
Bereitstellen
von Material aus jener Pflanze an zwei oder mehr Entwicklungsstadien; und
einer Oligonukleotidsonde, die mit RNA aus einem Gen hybridisieren kann, das ein Polypeptid kodiert, das in einem Flavonoid-Biosyntheseweg aktiv ist;
Extrahieren von RNA aus jenem Pflanzenmaterial;
Hybridisieren der Oligonukleotidsonde mit der RNA unter Verwendung eines Microarrays, um ein Expressionsprofil zu erzeugen;
qualitatives Messen der PA- und ANT-Spiegel in jenem Pflanzenmaterial unter Verwendung von Flüssigchromatographie-Massenspektroskopie, um ein Stoffwechselprofil zu erzeugen;
Vergleichen jenes Expressionsprofils mit jenem Stoffwechselprofil unter Verwendung eines Computers, um jenes Gen zu identifizieren, das ein Polypeptid oder eine Polypeptidisoform kodiert, das/die in entweder einem Pa- oder ANT-Stoffwechselweg wesentlich aktiver ist.

2. Ein Verfahren gemäß Anspruch 1, wobei das Polypeptid oder die Polypeptidisoform ein Enzym ist, das eine der letzten Reaktionen in der Synthese von Anthocyaninen katalysiert, nach dem Leukoanthocyanidin-Verzweigungspunkt in dem ANT-Stoffwechselweg.

3. Ein Verfahren gemäß Anspruch 1, wobei jenes Polypeptid oder jene Polypeptidisoform ausgewählt ist aus der Gruppe bestehend aus GST, G3T, UFGT, OMT, ART, ANAT und AAT.

4. Ein Verfahren gemäß Anspruch 1, wobei das Polypeptid oder die Polypeptidisoform ein Transkriptionsfaktor ist, vorzugsweise wobei jenes Polypeptid oder jene Polypeptidisoform ausgewählt ist aus der Gruppe bestehend aus MYB, bHLH, MYC und WDR.

5. Ein Verfahren zum Manipulieren des Flavonoid-Biosynthesewegs in einer Pflanze, jenes Verfahren umfassend Identifizieren eines Gens, das ein Polypeptid oder eine Polypeptidisoform kodiert, das/die in entweder einem PA- oder ANT-Stoffwechselweg jener Pflanze wesentlich aktiver ist, unter Verwendung eines Verfahrens gemäß irgendeinem der Ansprüche 1 bis 4, und Hoch- oder Herunterregulieren der Expression jenes Gens, um den Spiegel von PA oder ANT in jener Pflanze zu erhöhen oder zu verringern.

6. Ein Verfahren gemäß Anspruch 5, wobei jenes Verfahren das Herunterregulieren der Expression eines Gens umfasst, das ein Polypeptid oder eine Polypeptidisoform kodiert, das/die ein Enzym ist, das eine der letzten Reaktionen in der Synthese von Anthocyaninen katalysiert, nach dem Leukoanthocyanidin-Verzweigungspunkt in dem ANT-Stoffwechselweg.

7. Ein Verfahren gemäß Anspruch 5, wobei jenes Verfahren das Hoch- und/oder Herunterregulieren der Expression von einem oder mehr Genen umfasst, die einen Transkriptionsfaktor kodieren.

8. Ein Verfahren zur Verbesserung der Blähsicherheit einer Pflanze, jenes Verfahren umfassend:
Identifizieren eines Gens, das ein Polypeptid oder eine Polypeptidisoform kodiert, das/die in einem PA-Stoffwechselweg wesentlich aktiver ist, unter Verwendung eines Verfahrens gemäß irgendeinem der Ansprüche 1 bis 4, und Hochregulieren oder Herunterregulieren der Expression jenes Gens, um den Spiegel von PA in jener Pflanze zu erhöhen; oder
Identifizieren eines Gens, das ein Polypeptid oder eine Polypeptidisoform kodiert, das/die in einem ANT-Stoffwechselweg wesentlich aktiver ist, unter Verwendung eines Verfahrens gemäß irgendeinem der Ansprüche 1 bis 4, und Hochregulieren oder Herunterregulieren der Expression jenes Gens, um den Spiegel von PA in jener Pflanze zu erhöhen.

9. Ein Verfahren gemäß Anspruch 8, wobei jenes Verfahren das Herunterregulieren der Expression eines Gens umfasst, das ein Polypeptid oder eine Polypeptidisoform kodiert, das/die ein Enzym ist, das eine der letzten Reaktionen in der Synthese von Anthocyaninen katalysiert, nach dem Leukoanthocyanidin-Verzweigungspunkt in dem ANT-Stoffwechselweg.

10. Ein Verfahren gemäß Anspruch 8, wobei jenes Verfahren das Hoch- und/oder Herunterregulieren der Expression von einem oder mehr Genen umfasst, die einen Transkriptionsfaktor kodieren.

## Revendications

1. Procédé d'identification d'un gène codant pour un polypeptide ou une isoforme de polypeptide qui est sensiblement plus actif dans une voie de la proanthocyanidine (PA) ou de l'anthocyanine (ANAT) d'une plante, ledit procédé comprenant
la fourniture
d'un matériel provenant de ladite plante à deux ou plusieurs stades de développement ; et
d'une sonde oligonucléotidique capable de s'hybrider à un ARN provenant d'un gène codant pour un polypeptide qui est actif dans une voie de biosynthèse des flavonoïdes ;
l'extraction de l'ARN à partir dudit matériel végétal ;
l'hybridation de la sonde oligonucléotidique à l'ARN en utilisant un microarray pour générer un profil d'expression ;
la mesure des taux de PA et d'ANT dans ledit matériel végétal de manière quantitative en utilisant la chromatographie en phase liquide-spectrométrie de masse pour générer un profil métabolique ;
la comparaison dudit profil d'expression audit profil métabolique en utilisant un ordinateur pour identifier ledit gène codant pour un polypeptide ou une isoforme de polypeptide qui est sensiblement plus actif dans une voie de la PA ou de l'ANT.

2. Procédé selon la revendication 1, dans lequel le polypeptide ou l'isoforme de polypeptide est une enzyme qui catalyse une des réactions finales dans la synthèse d'anthocyanines, après le point de bifurcation des leucoanthocyanidines dans la voie de l'ANT.

3. Procédé selon la revendication 2, dans lequel ledit polypeptide ou ladite isoforme de polypeptide est choisi dans le groupe constitué par GST, G3T, UFGT, OMT, ART, ANAT et AAT.

4. Procédé selon la revendication 1, dans lequel le polypeptide ou l'isoform de polypeptide est un facteur de transcription, de préférence dans lequel ledit polypeptide ou ladite isoforme de polypeptide est choisi dans le groupe constitué par MYB, bHLH, MYC et WDR.

5. Procédé de manipulation de la voie de biosynthèse des flavonoïdes chez une plante, ledit procédé comprenant l'identification d'un gène codant pour un polypeptide ou une isoforme de polypeptide qui est sensiblement plus actif dans une voie de la PA ou de l'ANT de ladite plante en utilisant un procédé selon l'une quelconque des revendications 1 à 4, et la régulation à la hausse ou à la baisse de l'expression dudit gène pour augmenter ou diminuer le taux de PA ou d'ANT dans ladite plante.

6. Procédé selon la revendication 5, dans lequel ledit procédé comprend la régulation à la baisse de l'expression d'un gène codant pour un polypeptide ou une isoforme de polypeptide qui est une enzyme qui catalyse une des réactions finales dans la synthèse des anthocyanines, après le point de bifurcation des leucoanthocyanidines dans la voie de l'ANT.

7. Procédé selon la revendication 5, dans lequel ledit procédé comprend la régulation à la hausse et/ou à la baisse de l'expression d'un ou plusieurs gènes codant pour un facteur de transcription.

8. Procédé de renforcement de la sécurité d'une plante par rapport aux ballonnements, ledit procédé comprenant
l'identification d'un gène codant pour un polypeptide ou une isoforme de polypeptide qui est sensiblement plus actif dans une voie de la PA en utilisant un procédé selon l'une quelconque des revendications 1 à 4, et la régulation à la hausse ou la régulation à la baisse de l'expression dudit gène pour augmenter le taux de PA dans ladite plante ; ou
l'identification d'un gène codant pour un polypeptide ou une isoforme de polypeptide qui est sensiblement plus actif dans une voie de l'ANT en utilisant un procédé selon l'une quelconque des revendications 1 à 4, et la régulation à la hausse ou la régulation à la baisse de l'expression dudit gène pour augmenter le taux de PA dans ladite plante.

9. Procédé selon la revendication 8, dans lequel ledit procédé comprend la régulation à la baisse de l'expression d'un gène codant pour un polypeptide ou une isoforme de polypeptide qui est une enzyme qui catalyse une des réactions finales dans la synthèse des anthocyanines, après le point de bifurcation des leucoanthocyanidines dans la voie de l'ANT.

10. Procédé selon la revendication 8, dans lequel ledit procédé comprend la régulation à la hausse et/ou à la baisse de l'expression d'un ou plusieurs gènes codant pour un facteur de transcription.
